# EUROPEAN PATENT APPLICATION

(11) **EP 4 756 036 A2**
(43) Date of publication of application: **10.06.2026**
(21) Application number: 26173892.6
(22) Date of filing: 15.12.2023
(51) Int. Cl.: C12Q 1/6806

(54) **FLOW CELLS AND METHODS**

(30) Priority: 16.12.2022 US 202263387874 P; 21.12.2022 US 202263476585 P; 29.09.2023 US 202363586716 P; 21.11.2023 US 202363601655 P
(62) Divisional of application: 23847821.8
(71) Applicant: Illumina, Inc., San Diego, CA 92122 (US)
(72) Inventor: ARTIOLI, Gianluca Andrea, San Diego, CA 92122 (US); BASUKI, Johan Sebastian, San Diego, CA 92122 (US); CIESLA, Craig, San Diego, CA 92122 (US); DILL, Tyler J., San Diego, CA 92122 (US); FRASER, Louise Jane, San Diego, CA 92122 (US); GORMLEY, Niall Anthony, San Diego, CA 92122 (US); HO, Yuan Heng Trevor, San Diego, CA 92122 (US); KARUNAKARAN, Aathavan, San Diego, CA 92122 (US); MARTINS VITORIANO, Maria Ines, San Diego, CA 92122 (US); MOORE, Jessica, San Diego, CA 92122 (US); RICOULT, Sebastien Georg Gabriel, San Diego, CA 92122 (US); THOMSON, Vicki S., San Diego, CA 92122 (US); VON HATTEN, Xavier, San Diego, CA 92122 (US); WEIR, Jacqueline C., San Diego, CA 92122 (US)
(74) Representative: Forresters IP LLP

(57) **Abstract**

An example of a flow cell includes a substrate having depressions separated by interstitial regions; a polymeric hydrogel positioned within each of the depressions; and a plurality of transposome complexes immobilized within each of the depressions by a biotin-containing linker. In this example, each of the plurality of the transposome complexes is of a single type including a transposon end with a portion of a transferred strand hybridized to a portion of a non-transferred strand, wherein the transferred strand includes a first amplification domain and is free of an index sequence.

## Description

### CROSS-REFERENCE TO RELATED APPLICATIONS

This application claims the benefit of U.S. Provisional Patent Application 63/601,655, filed November 21, 2023, and U.S. Provisional Patent Application Serial No. 63/586,716, filed September 29, 2023, and U.S. Provisional Application Serial Number 63/476,585, filed December 21, 2022, and U.S. Provisional Patent Application Serial No. 63/387,874, filed December 16, 2022, the contents of each of which are incorporated herein by reference in its entirety.

### REFERENCE TO SEQUENCE LISTING

The Sequence Listing submitted herewith is hereby incorporated by reference in its entirety. The name of the file ILI256BPCT_IP-2542-PCT_Sequence_Listing.xml, the size of the file is 14,888 bytes, and the date of creation of the file is December 14, 2023.

### BACKGROUND

Double-stranded DNA (dsDNA) target molecules can be fragmented and tagged to generate a library of smaller, single-stranded DNA molecules (ssDNA). These smaller, single-stranded DNA molecules may be used as templates in DNA sequencing reactions. The templates may enable short read lengths to be obtained, and then during data analysis, overlapping short sequence reads can be aligned to reconstruct the longer nucleic acid sequences. Some methods for fragmentation and tagging of double-stranded DNA generate excessive waste, involve expensive instruments for fragmentation, and are time-consuming.

### SUMMARY

Some examples of the flow cells disclosed herein include surface chemistry that enables repurposing of the flow cell functionality. For example, in a single workflow, the flow cell may be used for tagmentation and then for amplification and sequencing. For another example, the flow cell may be reused for multiple cycles of transposome complex binding and tagmentation. These example flow cells can improve efficiency, enable indexing of tagmented fragments, and/or enable enrichment.

Other examples of the flow cells disclosed herein include surface chemistry that is light activated. These example flow cells enable spatial positioning control during surface preparation and/or methods taking place on the flow cell surface. Thus, these example flow cells can improve efficiency and/or enable flow cell (or spatial) indexing (as opposed to indexing of individual tagmented DNA fragments).

Other examples of the flow cells disclosed herein utilize heat, light, or a pH change to activate transposome complexes in a predetermined position/area of the flow cell. Through selective and sequential transposome complex activation, multiple DNA samples can be sequentially introduced into the flow cell and exposed to tagmentation at respective predetermined positions/areas. Thus, cluster generation for a particular sample takes place at a particular predetermined area. This allows the samples to be indexed spatially, as each area includes fragments or clusters of a different DNA sample.

### BRIEF DESCRIPTION OF THE DRAWINGS

Features of examples of the present disclosure will become apparent by reference to the following detailed description and drawings, in which like reference numerals correspond to similar, though perhaps not identical, components. For the sake of brevity, reference numerals or features having a previously described function may or may not be described in connection with other drawings in which they appear.
Fig. 1 is a top view of a flow cell;
Fig. 2A is a cross-sectional view of one flow channel architecture of the flow cell of Fig. 1, which includes depressions separated by interstitial regions;
Fig. 2B is a cross-sectional view of another flow channel architecture of the flow cell of Fig. 1, which includes a single lane without depressions separated by interstitial regions;
Fig. 3A through Fig. 3H are chemical flow diagrams or chemical structures illustrating visible light member pairs;
Fig. 4 is a schematic flow diagram depicting an example of a method involving repurposing a flow cell surface for multiple applications, including tagmentation and amplification;
Fig. 5 is a schematic flow diagram depicting another example of a method involving repurposing a flow cell surface for multiple applications, including tagmentation and amplification;
Fig. 6 is a schematic flow diagram depicting still another example of a method involving repurposing a flow cell surface for multiple applications, including tagmentation, enrichment, and amplification;
Fig. 7 is a schematic flow diagram depicting still another example of a method involving repurposing a flow cell surface for multiple applications, including tagmentation, enrichment, and amplification
Fig. 8 is a schematic flow diagram depicting still another example of a method involving repurposing a flow cell surface for multiple applications, including enriching a methylated bacterial DNA;

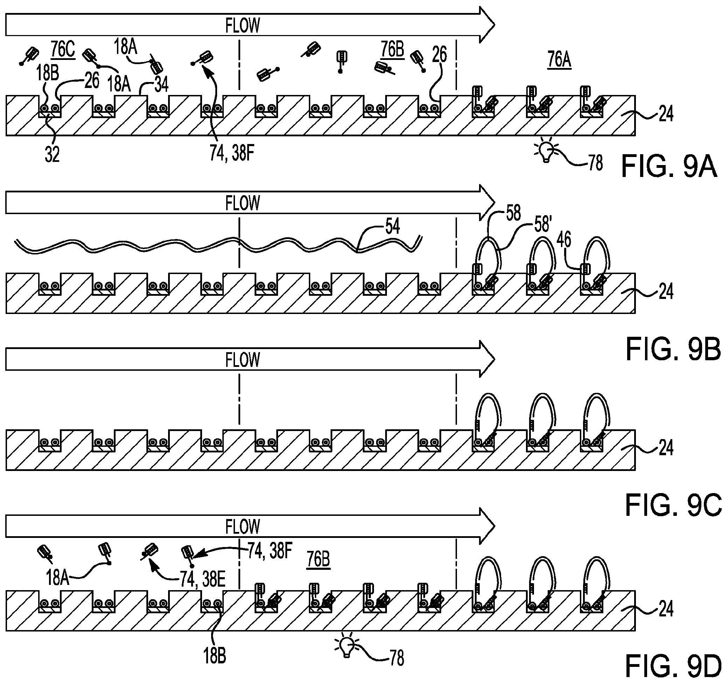

Fig. 9A through Fig. 9D are schematic illustrations that together depict a method utilizing light activated surface chemistry to selectively attach a tagmentation entity (e.g., transposome complex) to a predetermined area of a flow cell, where Fig. 9A depicts the selective attachment of transposome complexes at a first predetermined area upon exposure to light, Fig. 9B depicts the introduction of a first DNA sample and the tagmentation of the first DNA sample at the first predetermined area, Fig. 9C depicts the removal of transposase enzymes from the transposome complexes at the first predetermined area, and Fig. 9D depicts the selective attachment of transposome complexes at a second predetermined area upon exposure to light;
Fig. 10A depicts a flow cell with different areas for performing different applications, at least one of which is light activated;
Fig. 10B depicts another flow cell with different areas for performing different applications, at least one of which is light activated;
Fig. 10C depicts still another flow cell with different areas for performing different applications, at least one of which is light activated;

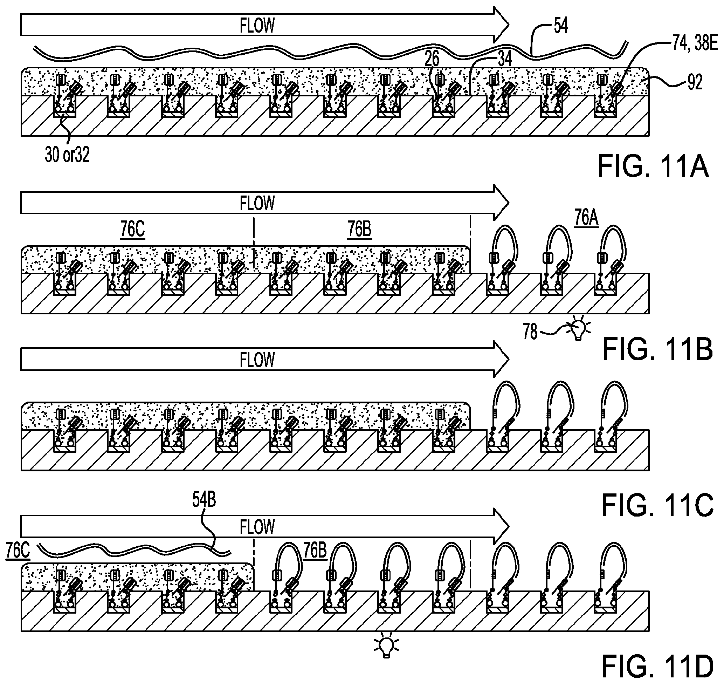

Fig. 11A through Fig. 11D are schematic illustrations that together depict a method utilizing a removable coating to selectively access a tagmentation entity (e.g., transposome complex) at a predetermined area of a flow cell, where Fig. 11A depicts the introduction of a first DNA sample into a flow cell having a removable coating overlying the transposome complexes, Fig. 11B depicts the selective removal of the removable coating from a first predetermined area and the tagmentation of the first DNA sample at the first predetermined area, Fig. 11C depicts the removal of transposase enzymes from the transposome complexes at the first predetermined area, and Fig. 11D depicts the introduction of a second DNA sample into the flow cell, the selective removal of the removable coating from a second predetermined area, and the tagmentation of the second DNA sample at the second predetermined area;

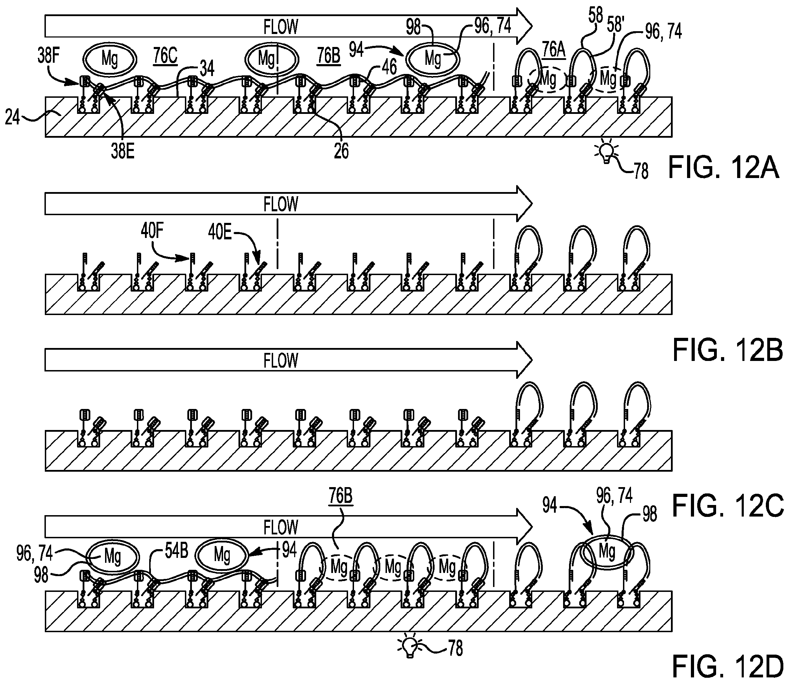

Fig. 12A through Fig. 12D are schematic illustrations that together depict a method utilizing an encapsulated complex to selectively release a tagmentation entity (e.g., a catalyst metal) at a predetermined area of a flow cell, where Fig. 12A depicts the introduction of a first DNA sample and an encapsulated complex into a flow cell, the selective release of the tagmentation entity from the encapsulated complex in the first predetermined area, and tagmentation of the first DNA sample at the first predetermined area, Fig. 12B depicts the removal of the untagmented portion of the first DNA sample and of the transposase enzymes from the transposome complexes across the flow cell, Fig. 12C depicts the introduction of fresh transposase enzymes to reform transposome complexes, and Fig. 12D depicts the introduction of a second DNA sample and an encapsulated complex into the flow cell, the selective release of the tagmentation entity from the encapsulated complex in a second predetermined area, and tagmentation of the second DNA sample at the second predetermined area;

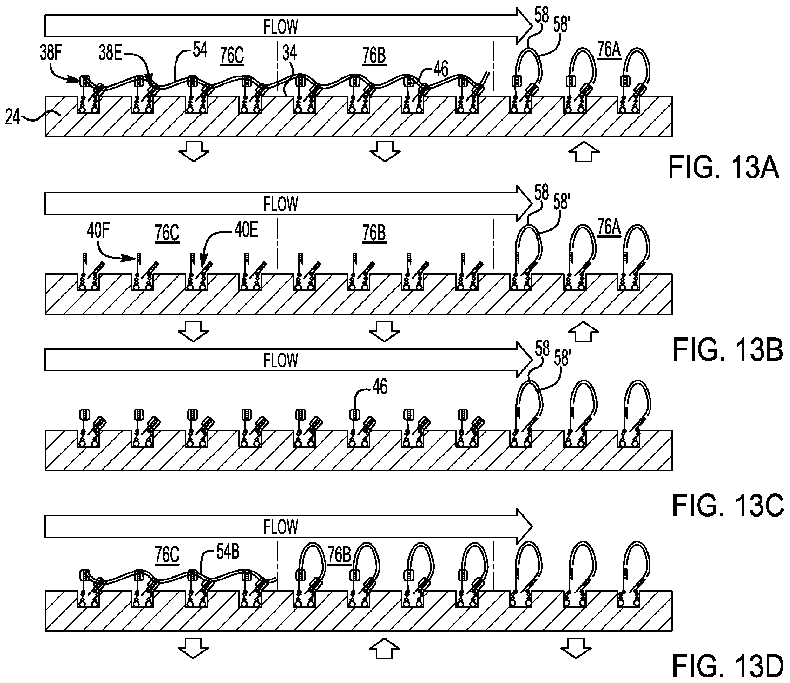

Fig. 13A through Fig. 13D are schematic illustrations that together depict a method utilizing heat to selectively activate a tagmentation entity (e.g., transposome complex) at a predetermined area of a flow cell, where Fig. 13A depicts the introduction of a first DNA sample into a flow cell and the tagmentation of the first DNA sample at the first predetermined area upon exposure to heat; Fig. 13B depicts the removal of the transposase enzymes from the transposome complexes across the flow cell, Fig. 13C depicts the introduction of fresh transposase enzymes to reform transposome complexes, and Fig. 13D depicts the introduction of a second DNA sample into the flow cell and the tagmentation of the second DNA sample at a second predetermined area upon exposure to heat;
Fig. 14A through Fig. 14D depict different examples of the transposome complexes that can be used in different examples of the method disclosed herein;
Fig. 15A is a schematic top view of a lane of a flow cell with different target primers patterned at different regions along the lane;
Fig. 15B is an enlarged, cross-sectional and perspective view of a portion of any one of the regions of the flow cell lane of Fig. 15A, where target primers are attached within depressions of the flow cell lane, and where transposome complexes containing complementary spatial tags are depicted in one of the depressions;
Fig. 16A depicts a bound complex, including a DNA sample bound to transposome complexes containing spatial tags, passivated by a hydrophobic polymer; and
Fig. 16B depicts a bound complex passivated by an anti-fouling agent.

### DETAILED DESCRIPTION

Some examples of the flow cells disclosed herein include surface chemistry for immobilizing transposome complexes and primers to the surface of the flow cell. By incorporating this surface chemistry, the flow cell can be repurposed for a particular application. On flow cell tagmentation generates DNA sample fragments (i.e., library fragments of the larger DNA sample) on the same surface where amplification and sequencing of the fragments takes place. This eliminates the need for off flow cell DNA sample preparation to generate the library fragments, and thus provides a more stream-lined and efficient process. Some of the methods using these example flow cells also enable library fragment indexing and/or enrichment.

Other examples of the flow cells disclosed herein include light activated surface chemistry. This enables spatial location/position control over surface chemistry activation, which in turn enables selective surface preparation and/or reaction initiation.

Still other examples of the flow cells disclosed herein include utilize heat, light, or a pH change to activate transposome complexes in a predetermined area of the flow cell. This enables control over where a sample is seeded and amplified, and thus enables multiple samples to be used and identified by spatial indexing.

### Definitions

Terms used herein will be understood to take on their ordinary meaning in the relevant art unless specified otherwise. Several terms used herein and their meanings are set forth below.

As used herein, the singular forms "a," "an," and "the" refer to both the singular as well as plural, unless the context clearly indicates otherwise. The term "comprising" as used herein is synonymous with "including," "containing," or "characterized by," and is inclusive or open-ended and does not exclude additional, unrecited elements or method steps.

Reference throughout the specification to "one example," "another example," "an example," and so forth, means that a particular element (e.g., feature, structure, composition, configuration, and/or characteristic) described in connection with the example is included in at least one example described herein, and may or may not be present in other examples. In addition, it is to be understood that the described elements for any example may be combined in any suitable manner in the various examples unless the context clearly dictates otherwise.

The terms "substantially" and "about" used throughout this disclosure, including the claims, are used to describe and account for small fluctuations, such as those due to variations in processing. For example, these terms can refer to less than or equal to ±5% from a stated value, such as less than or equal to ±2% from a stated value, such as less than or equal to ±1% from a stated value, such as less than or equal to ±0.5% from a stated value, such as less than or equal to ±0.2% from a stated value, such as less than or equal to ±0.1% from a stated value, such as less than or equal to ±0.05% from a stated value.

*Adapter.* An oligonucleotide sequence that can be fused to a nucleic acid molecule, for example, by ligation or tagmentation. Suitable adapter lengths may range from about 10 nucleotides to about 100 nucleotides, or from about 12 nucleotides to about 60 nucleotides, or from about 15 nucleotides to about 50 nucleotides. The adapter may include any combination of nucleotides and/or nucleic acids. In some examples, the adapter can include an amplification domain, e.g., having a universal nucleotide sequence, such as a P5 or P7 sequence, that can serve as a starting point for template amplification and cluster generation. In other examples, the adapter can include a sequence that is complementary to at least a portion of a flow cell surface bound primer (which includes the universal nucleotide sequence). In the latter example, the adapter sequence can hybridize to the complementary flow cell surface bound primer during amplification and cluster generation. In some examples, the adapter can also include a sequencing primer sequence (i.e., sequencing binding site) or a sequencing sample index (i.e., a barcode sequence). Combinations of different adapters may be incorporated into the nucleic acid molecule, such as the DNA fragments generated via tagmentation.

*Amplification Domain:* A portion of an adapter having a universal nucleotide sequence, such as a P5 or P7 sequence or a complement thereof, that can serve as a starting point for template amplification and cluster generation.

*Corresponds with:* When one primer "corresponds with" an amplification domain, the primer and amplification domain may have the same sequence, so that a copy of the amplification domain generates a sequence complementary to the primer; or they may have complementary sequences when the amplification domain is introduced as part of an adapter.

*Depositing:* Any suitable application technique, which may be manual or automated, and, in some instances, results in modification of the surface properties. Generally, depositing may be performed using vapor deposition techniques, coating techniques, grafting techniques, or the like. Some specific examples include chemical vapor deposition (CVD), spray coating (e.g., ultrasonic spray coating), spin coating, dunk or dip coating, doctor blade coating, puddle dispensing, flow through coating, aerosol printing, screen printing, microcontact printing, inkjet printing, or the like.

*Depression:* A discrete concave feature in a substrate or a layer of a substrate (e.g., a patterned resin) having a surface opening that is at least partially surrounded by interstitial region(s) of the substrate or the layer. Depressions can have any of a variety of shapes at their opening in a surface including, as examples, round, elliptical, square, polygonal, star shaped (with any number of vertices), etc. The cross-section of a depression taken orthogonally with the surface can be curved, square, polygonal, hyperbolic, conical, angular, etc. The depression may also have more complex architectures, such as ridges, step features, etc.

*DNA Sample:* A polymeric form of nucleotides of any length that includes deoxyribonucleotides, deoxyribonucleotide analogs, or complementary deoxyribonucleotides derived from an RNA (ribonucleic acid) sample. The DNA sample is double stranded. The DNA sample may include naturally occurring DNA, which includes a nitrogen containing heterocyclic base (a nucleobase such as adenine, thymine, cytosine and/or guanine), a sugar (specifically deoxyribose, i.e., a sugar lacking a hydroxyl group that is present at the 2' position in ribose), and a backbone containing phosphodiester bonds. An analog structure can have an alternate backbone linkage including any of a variety known in the art.

The DNA sample may be genomic DNA (gDNA) that can be isolated from one or more cells, bodily fluids (e.g., whole blood, blood spots, saliva) or tissues. gDNA can be prepared by lysing a cell that contains the DNA. The cell may be lysed under conditions that substantially preserve the integrity of the cell's gDNA. In one particular example, thermal lysis may be used to lyse a cell. In another particular example, exposure of a cell to alkaline pH can be used to lyse a cell while causing relatively little damage to gDNA. Any of a variety of basic compounds can be used for lysis including, for example, potassium hydroxide, sodium hydroxide, and the like. Additionally, relatively undamaged gDNA can be obtained from a cell lysed by an enzyme that degrades the cell wall. Cells lacking a cell wall either naturally or due to enzymatic removal can also be lysed by exposure to osmotic stress. Other conditions that can be used to lyse a cell include exposure to detergents, mechanical disruption, sonication heat, pressure differential such as in a French press device, or Dounce homogenization. Agents that stabilize gDNA can be included in a cell lysate or isolated gDNA sample including, for example, nuclease inhibitors, chelating agents, salts, buffers and the like. A crude cell lysate containing gDNA may be used without further isolation of the gDNA. In one example, a whole blood sample may be lysed using an inorganic salt free lysis buffer, and the crude lysate may be exposed to specific processing steps to generate a complexed crude lysate. This complexed crude lysate can also be used as the DNA sample without further isolation or purification.

*Each:* When used in reference to a collection of items, each identifies an individual item in the collection, but does not necessarily refer to every item in the collection. Exceptions can occur if explicit disclosure or context clearly dictates otherwise.

*Flow Cell*: A vessel having an enclosed flow channel where a reaction can be carried out, or a vessel having a channel that is open to a surrounding environment and in which a reaction can be carried out. The vessel with an open flow channel may be referred to herein as an open wafer flow cell. Any example of the flow cell may include an inlet for delivering reagent(s) to the channel, and an outlet for removing reagent(s) from the channel. In some examples, the flow cell enables the detection of the reaction that occurs therein. For example, the flow cell can include one or more transparent surfaces allowing for the optical detection of arrays, optically labeled molecules, or the like.

*Flow channel:* An area that is defined between two bonded or otherwise attached components or that is defined within a lane so that it is open to the surrounding environment. The flow channel can selectively receive a liquid sample. In some examples, the flow channel may be defined between two patterned sequencing surfaces or a patterned sequencing surface and a lid, and thus may be in fluid communication with one or more components of the sequencing surface(s).

*Fragment:* A portion or piece of the DNA sample. A "partially adapted fragment" is a portion or piece of the DNA sample that has been tagmented, and thus includes an adapter ligated to the 5' end of the DNA fragment. A "fully adapted fragment" is a portion or piece of the DNA sample that has adapters incorporated at both the 3' and 5' ends of the DNA fragment.

*Primer*: A single stranded nucleic acid molecule that can hybridize to a target sequence, such as an adapter attached to a fragment. As one example, a flow cell surface bound primer can serve as a starting point for fragment amplification and cluster generation. As another example, a flow cell surface bound primer can serve as a hybridization point for a spatial tag, and thus for targeting attachment of particular transposome complexes and DNA samples. As still another example, a primer (e.g., a sequencing primer) may be introduced that can hybridize to fragments or fragment amplicons in order to prime synthesis of a new strand that is complementary to the fragments or fragment amplicons. Any primer can include any combination of nucleotides or analogs thereof. In some examples, the primer is a single-stranded oligonucleotide or polynucleotide. The primer length can be any number of bases long. In an example, each of the flow cell surface bound primer and the sequencing primer is a short strand, ranging from 10 to 60 bases, or from 20 to 40 bases.

*Tagmentation Entity*: Any component involved in the process of tagmentation. The term may refer to a transposome complex, a transposase enzyme, a reagent, or the like.

*Tagmentation*: A process in which the DNA sample is cleaved/fragmented and tagged (e.g., with the adapters) for analysis. Tagmentation is an *in vitro* transposition reaction.

*Transferred and Non-Transferred Strands*: The term "transferred strand" refers to a sequence that includes a transferred portion of a transposon end. Similarly, the term "non-transferred strand" refers to a sequence that includes the non-transferred portion of a transposon end. The 3'-end of a transferred strand is joined or transferred to a double stranded fragment during tagmentation. The non-transferred strand is not joined or transferred to the double stranded fragment during tagmentation. In an example, the transferred and non-transferred strands include at least partially complementary portions that are covalently bound together.

*Transposase or Transposase Enzyme:* An enzyme that is capable of forming a functional complex with a transposon end-containing composition (e.g., transposons, transposon ends, transposon end compositions) and catalyzing insertion or transposition of the transposon end-containing composition into the double-stranded DNA sample with which it is incubated, for example, in the *in vitro* transposition reaction (i.e., tagmentation). A transposase as presented herein can also include integrases from retrotransposons and retroviruses. Although many examples described herein refer to Tn5 transposase and/or hyperactive Tn5 transposase, it will be appreciated that any transposase that is capable of inserting a transposon end with sufficient efficiency to 5'-tag and fragment the DNA sample for its intended purpose can be used.

*Transposome Complex:* An entity formed between a transposase and a double stranded nucleic acid including a transposase integration recognition site. For example, the transposome complex can be a transposase enzyme pre-incubated with double-stranded transposon DNA under conditions that support non-covalent complex formation. Double-stranded transposon DNA can include, for example, Tn5 DNA, a portion of Tn5 DNA, a transposon end composition, a mixture of transposon end compositions or other double-stranded DNAs capable of interacting with a transposase, such as the hyperactive Tn5 transposase.

*Transposon End*: A double-stranded nucleic acid strand that exhibits only the nucleotide sequences (the "transposon end sequences") that are necessary to form the complex with the transposase that is functional in tagmentation. The double-stranded nucleic acid strand of the transposon end can include any nucleic acid or nucleic acid analogue suitable for forming the functional complex with the transposase. For example, the transposon end can include natural DNA or DNA analogs (with modified bases and/or backbones), and can include nicks in one or both strands.

### Flow Cells

Some examples of the flow cells disclosed herein include a biotin-containing linker that enables the flow cell to be repurposed for different applications. Other examples of the flow cells disclosed herein include a visible light responsive member that enables the flow cell to be selectively activated.

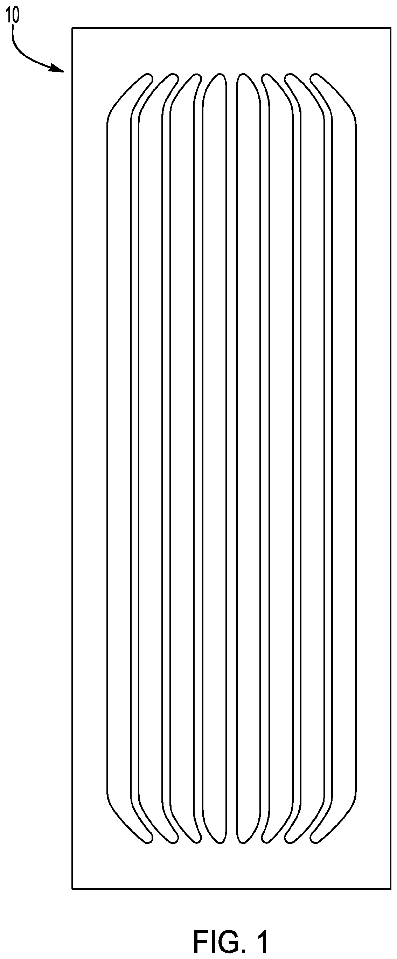

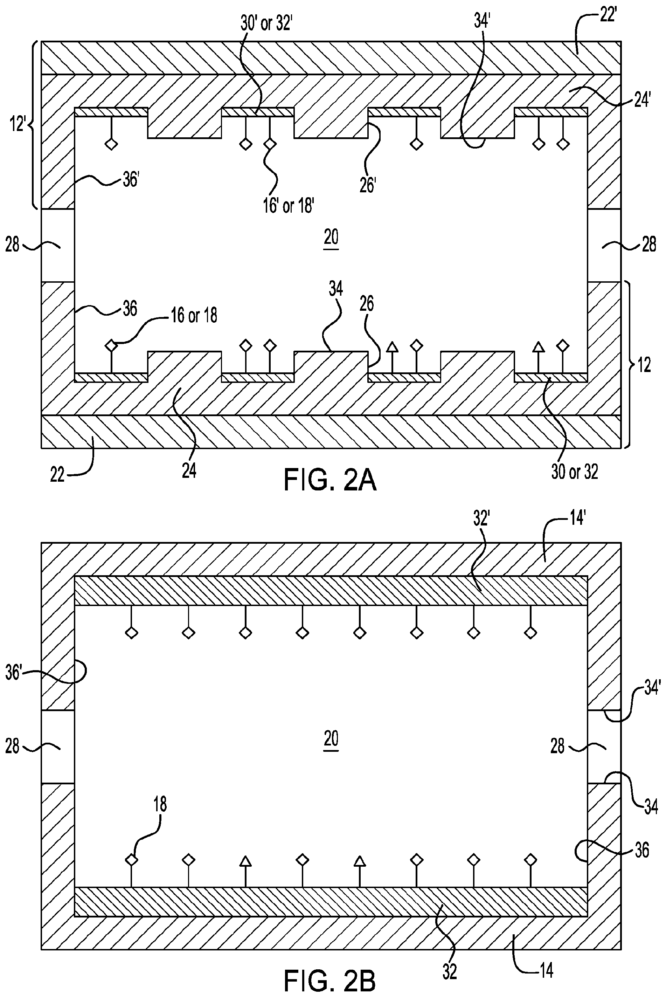

An example of the flow cell 10 is depicted from the top view in Fig. 1, and different examples of the flow cell architecture are shown in Fig. 2A and Fig. 2B.

A top view of an example of the flow cell 10 is shown in Fig. 1. As will be discussed in reference to Fig. 2A and Fig. 2B, some examples of the flow cell 10 include two opposed substrates 12 and 12' or 14 and 14', each of which may be configured with a biotin-containing linker 16, 16' or a visible light responsive member 18, 18'. Other example flow cells 10 include one substrate 12 or 14, which may be configured with the biotin-containing linker 16 or the visible light responsive member 18, and a lid that is bonded to a portion of the substrate 12 or 14. Still other example flow cells 10 include one substrate 12 or 14, which may be configured with the biotin-containing linker 16 or the visible light responsive member 18, and is not bonded to another component, but rather is open to the surrounding environment.

In the examples shown in Fig. 2A and Fig. 2B, a flow channel 20 is defined between the two opposed substrates 12 and 12' or 14 and 14'. In other examples, the flow cell 10 includes one substrate 12 or 12' or 14 or 14' and the lid (not shown) attached to the substrate 12 or 12' or 14 or 14'. In these examples, the flow channel 20 is defined between the substrate 12 or 12' or 14 or 14' and the lid. In the open wafer version, the flow channel 20 may be defined by the lane 36 (see Fig. 2B).

Different substrates 12 or 12' or 14 or 14' are shown in Fig. 2A and Fig. 2B.

In the example shown in Fig. 2B, the substrates 14, 14' are single layered structures. Examples of suitable single layered structures for the substrate 14, 14' include epoxy siloxane, glass, modified or functionalized glass, polymeric materials (including acrylics, polystyrene and copolymers of styrene and other materials, polypropylene, polyethylene, polybutylene, polyurethanes, polytetrafluoroethylene (such as TEFLON^{®} from Chemours), cyclic olefins/cyclo-olefin polymers (COP) (such as ZEONOR^{®} from Zeon), polyimides, nylon (polyamides), etc.), ceramics/ceramic oxides, silica, fused silica, or silica-based materials, aluminum silicate, silicon and modified silicon (e.g., boron doped p+ silicon), silicon nitride (Si₃N₄), silicon oxide (SiO₂), tantalum pentoxide (Ta₂O₅) or other tantalum oxide(s) (TaOₓ), hafnium oxide (HfO₂), carbon, metals, or the like. In any of the examples disclosed herein, the substrates 14, 14' may be selected to be transparent to visible light.

In the examples shown in Fig. 2A, the substrates 12 or 12' are multi-layered structures. The multi-layered structures of the substrates 12, 12' include a base support 22, 22' and a patterned material 24, 24' on the base support 22, 22'. In any of the examples disclosed herein, the components of the substrates 12, 12' may be selected to be transparent to visible light.

The base support 22, 22' may be any of the examples set forth herein for the single layered structure of the substrate 14, 14'. The patterned material 24, 24' may be any material that is capable of being patterned with depressions 26, 26'.

In an example, the patterned material 24, 24' may be an inorganic oxide that is selectively applied to the base support 22, 22', e.g., via vapor deposition, aerosol printing, or inkjet printing, in the desired pattern. Examples of suitable inorganic oxides include tantalum oxide (e.g., Ta₂O₅), aluminum oxide (e.g., Al₂O₃), silicon oxide (e.g., SiO₂), hafnium oxide (e.g., HfO₂), etc. In another example, the patterned material 24, 24' may be a resin matrix material that is applied to the base support 22, 22' and then patterned. Suitable deposition techniques include chemical vapor deposition, dip coating, dunk coating, spin coating, spray coating, puddle dispensing, ultrasonic spray coating, doctor blade coating, aerosol printing, screen printing, microcontact printing, etc. Suitable patterning techniques include photolithography, nanoimprint lithography (NIL), stamping techniques, embossing techniques, molding techniques, microetching techniques, printing techniques, etc. Some examples of suitable resins include a polyhedral oligomeric silsesquioxane-based resin, a non-polyhedral oligomeric silsesquioxane epoxy resin, a poly(ethylene glycol) resin, a polyether resin (e.g., ring opened epoxies), an acrylic resin, an acrylate resin, a methacrylate resin, an amorphous fluoropolymer resin (e.g., CYTOP^{®} from Bellex), and combinations thereof.

In an example, the substrates 12, 12' or 14, 14' may be round and have a diameter ranging from about 2 mm to about 300 mm, or may be a rectangular sheet or panel having its largest dimension up to about 10 feet (~ 3 meters). In an example, the substrate 12, 12' or 14, 14' is a wafer having a diameter ranging from about 200 mm to about 300 mm. Wafers may subsequently be diced to form an individual flow cell substrate. In another example, the substrate 12, 12' or 14, 14' is a die having a width ranging from about 0.1 mm to about 10 mm. While example dimensions have been provided, it is to be understood that a substrate 12, 12' or 14, 14' with any suitable dimensions may be used. For another example, a panel may be used that is a rectangular support, which has a greater surface area than a 300 mm round wafer. Panels may subsequently be diced to form individual flow cells.

The flow cell 10 also includes the flow channel 20. While several flow channels 20 are shown in Fig. 1, it is to be understood that any number of flow channels 20 may be included in the flow cell 10 (e.g., a single channel 20, four channels 20, etc.). Each flow channel 20 may be isolated from each other flow channel 20 in a flow cell 10 so that fluid introduced into any particular flow channel 20 does not flow into any adjacent flow channel 20.

At least a portion of the flow channel 20 may be defined in the substrate 12, 12' or 14, 14' using any suitable technique that depends, in part, upon the material(s) of the substrate 12, 12' or 14, 14'. With the open wafer flow cell, the entire flow channel 20 may be defined by the lane 36 that is defined in the substrate 12 or 14. In one example, at least a portion of the flow channel 20 is etched into a glass substrate, such as substrate 14, 14'. In another example, at least a portion of the flow channel 20 may be patterned into a resin matrix material of a multi-layered structure using photolithography, nanoimprint lithography, etc. In enclosed versions of the flow cell 10, a separate material (e.g., material 28) may be applied to the substrate 12, 12' or 14, 14' so that the separate material 28 defines at least a portion of the walls of the flow channel 20.

In an example, the flow channel 20 has a substantially rectangular configuration with rounded ends. The length and width of the flow channel 20 may be smaller, respectively, than the length and width of the substrate 12, 12' or 14, 14' so that a portion of the substrate surface surrounding the flow channel 20 is available for attachment to another substrate 12, 12' or 14, 14' or to a lid or to define the perimeter of the open flow channel 20. In some instances, the width of each flow channel 20 can be at least about 1 mm, at least about 2.5 mm, at least about 5 mm, at least about 7 mm, at least about 10 mm, or more. In some instances, the length of each flow channel 54 can be at least about 10 mm, at least about 25 mm, at least about 50 mm, at least about 100 mm, or more. The width and/or length of each flow channel 20 can be greater than, less than or between the values specified above. In another example, the flow channel 20 is square (e.g., 10 mm x 10 mm).

The depth/height of each flow channel 20 can be as small as a few monolayers thick, for example, when microcontact, aerosol, or inkjet printing is used to deposit the separate material 28 that partially defines the flow channel walls. In other examples, the depth/height of each flow channel 20 can be about 1 µm, about 10 µm, about 50 µm, about 100 µm, or more. In an example, the depth/height may range from about 10 µm to about 100 µm. In another example, the depth/height is about 5 µm or less. It is to be understood that the depth/height of each flow channel 20 can also be greater than, less than or between the values specified above. The depth/height of the flow channel 20 may also vary along the length and width of the flow cell 10, e.g., when depressions 26, 26' are used.

The example flow cell architecture of Fig. 2A includes the depressions 26, 26' separated by interstitial regions 34, 34'. Many different layouts of the depressions 26, 26' may be envisaged, including regular, repeating, and non-regular patterns. In an example, the depressions 26, 26' are disposed in a hexagonal grid for close packing and improved density. Other layouts may include, for example, rectangular layouts, triangular layouts, and so forth. In some examples, the layout or pattern can be an x-y format in rows and columns. In some other examples, the layout or pattern can be a repeating arrangement of the depressions 26, 26' and the interstitial regions 34, 34'. In still other examples, the layout or pattern can be a random arrangement of the depressions 26, 26' and the interstitial regions 34, 34'.

The layout or pattern may be characterized with respect to the density (number) of the depressions 26, 26' in a defined area. For example, the depressions 26, 26' may be present at a density of approximately 2 million per mm². The density may be tuned to different densities including, for example, a density of about 100 per mm², about 1,000 per mm², about 0.1 million per mm², about 1 million per mm², about 2 million per mm², about 5 million per mm², about 10 million per mm², about 50 million per mm², or more, or less. It is to be further understood that the density can be between one of the lower values and one of the upper values selected from the ranges above, or that other densities (outside of the given ranges) may be used. As examples, a high density array may be characterized as having depressions 26, 26' separated by less than about 100 nm, a medium density array may be characterized as having the depressions 26, 26' separated by about 400 nm to about 1 µm, and a low density array may be characterized as having the depressions 26, 26' separated by greater than about 1 µm.

The layout or pattern of the depressions 26, 26' may also or alternatively be characterized in terms of the average pitch, or the spacing from the center of one depression 26, 26' to the center of an adjacent depression 26, 26' (center-to-center spacing) or from the right edge of one depression 26, 26' to the left edge of an adjacent depression 26, 26' (edge-to-edge spacing). The pattern can be regular, such that the coefficient of variation around the average pitch is small, or the pattern can be non-regular in which case the coefficient of variation can be relatively large. In either case, the average pitch can be, for example, about 50 nm, about 0.1 µm, about 0.5 µm, about 1 µm, about 5 µm, about 10 µm, about 100 µm, or more or less. The average pitch for a particular pattern can be between one of the lower values and one of the upper values selected from the ranges above. In an example, the depressions 26, 26' have a pitch (center-to-center spacing) of about 1.5 µm. While example average pitch values have been provided, it is to be understood that other average pitch values may be used.

The size of each depression 26, 26' may be characterized by its volume, opening area, depth, and/or diameter. For example, the volume can range from about 1×10⁻³ µm³ to about 100 µm³, e.g., about 1×10⁻² µm³, about 0.1 µm³, about 1 µm³, about 10 µm³, or more, or less. For another example, the opening area can range from about 1×10⁻³ µm² to about 100 µm², e.g., about 1×10⁻² µm², about 0.1 µm², about 1 µm², at least about 10 µm², or more, or less. For still another example, the depth can range from about 0.1 µm to about 100 µm, e.g., about 0.5 µm, about 1 µm, about 10 µm, or more, or less. For yet another example, the diameter or length and width can range from about 0.1 µm to about 100 µm, e.g., about 0.5 µm, about 1 µm, about 10 µm, or more, or less.

The example flow cell architecture of Fig. 2A includes the depressions 26, 26' defined in respective lanes 36, 36'. This architecture may be desirable for the substrate 12 when is used to form an open wafer flow cell 10. While not shown, it is to be further understood that the depressions 26, 26' may be defined across a substantially flat substrate surface (i.e., not in a lane 36, 36'), and the material 28 may completely define the side walls of the enclosed flow cells 10.

The example flow cell architecture of Fig. 2B also includes the lane 36, 36', without depressions 26, 26'. In this example, the lane 36, 36' extends just short of the full length and the full width of the substrate 14, 14' so that interstitial regions 34, 34' are formed at a perimeter of the lane 36, 36'. The flow cell architecture of Fig. 2B may be desirable for the methods disclosed herein that enable selective attachment of a tagmentation entity (see Fig. 9A through Fig. 9D) or those that enable selective activation of a transposome complex (see the figure 11, 12 and 13 series).

In one example, the flow cell architecture shown in Fig. 2A or in Fig. 2B includes a polymeric hydrogel 32, 32' with the visible light responsive member 18, 18' attached thereto.

The polymeric hydrogel 32, 32' may be poly(N-(5-azidoacetamidylpentyl)acrylamide-co-acrylamide (PAZAM) or another of the acrylamide copolymers disclosed herein (e.g., poly(N-N'-dimethylacrylamide), polyethylene glycol (PEG)-acrylate, PEG-diacrylate, PEG-amine, PEG-carboxylate, PEG-dithiol, PEG-epoxide, PEG-isocyanate, PEG-maleimide, crosslinked poly(methyl methacrylate) (PMMA), polyvinylpyrrolidone (PVPON), polyvinyl alcohol (PVA), polyethylene oxide-polypropylene oxide block copolymers (PEO-PPO), poly(hydroxyethyl methacrylate) (PHEMA), poly(N-isopropylacrylamide) (PNIPAAm), poly(lactic acid)-poly(ethylene glycol) block copolymers, poly(ethylene glycol)-poly(lactic-co-glycolic acid) block copolymers, poly(acrylic-co-vinylsulfonic acid), poly(acrylamide-co-vinylsulfonic acid), poly(L-aspartic acid), poly(aspartamide), adipic dihydrazide modified or aldehyde modified poly(L-glutamic acid), bisacrylamide, or hydrogels based on one or more of polylysine, starch, agar, agarose, heparin, alginate, alginate sulfate, dextran sulfate, hyaluronan, pectin, carrageenan, gelatin, chitosan, cellulose, and collagen, or combinations or mixtures thereof.

In one example, the polymeric hydrogel 32, 32' includes an acrylamide copolymer. In this example, the acrylamide copolymer has a structure (I): 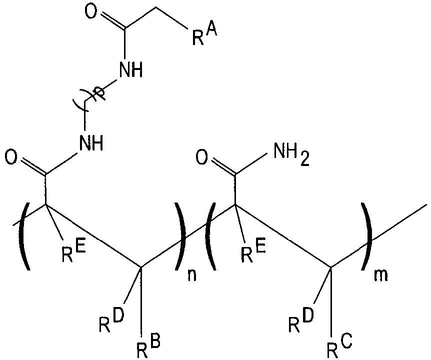 wherein:
R^{A} is an azide or a tetrazine or any other functional group that can attach to an alkyne, an amino, an alkenyl, an alkyne, a halogen, a hydraarea, a hydrazine, a carboxyl, a hydroxy, a tetrazole, nitrone, sulfate, or thiol;
R^{B} is H or optionally substituted alkyl;
R^{C}, R^{D}, and R^{E} are each independently selected from the group consisting of H and optionally substituted alkyl;
each of the -(CH₂)ₚ- can be optionally substituted;
p is an integer in the range of 1 to 50;
n is an integer in the range of 1 to 50,000; and
m is an integer in the range of 1 to 100,000.

One specific example of the acrylamide copolymer represented by structure (I) is poly(N-(5-azidoacetamidylpentyl)acrylamide-co-acrylamide, PAZAM.

One of ordinary skill in the art will recognize that the arrangement of the recurring "n" and "m" features in structure (I) are representative, and the monomeric subunits may be present in any order in the polymer structure (e.g., random, block, patterned, or a combination thereof).

The molecular weight of the acrylamide copolymer may range from about 5 kDa to about 1500 kDa or from about 10 kDa to about 10 MDa, or may be, in a specific example, about 312 kDa.

In some examples, the acrylamide copolymer is a linear polymer. In some other examples, the acrylamide copolymer is a lightly cross-linked polymer.

In some examples, the gel material may be a variation of structure (I). In one example, the acrylamide unit may be replaced with N,N-dimethylacrylamide ( 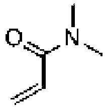 ). In another example, the acrylamide unit in structure (I) may be replaced with, 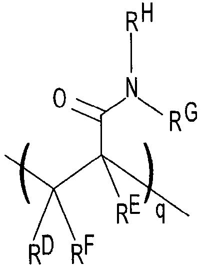 where R^{D}, R^{E}, and R^{F} are each H or a C1-C6 alkyl, and R^{G} and R^{H} are each a C1-C6 alkyl (instead of H as is the case with the acrylamide). In this example, q may be an integer in the range of 1 to 100,000. In another example, the N,N-dimethylacrylamide may be used in addition to the acrylamide unit. In this example, structure (I) may include 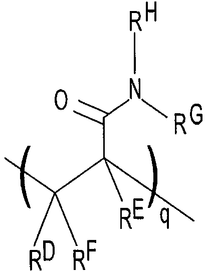 in addition to the recurring "n" and "m" features, where R^{D}, R^{E}, and R^{F} are each H or a C1-C6 alkyl, and R^{G} and R^{H} are each a C1-C6 alkyl. In this example, q may be an integer in the range of 1 to 100,000.

As another example of the polymeric hydrogel 32, 32', the recurring "n" feature in structure (I) may be replaced with a monomer including a heterocyclic azido group having structure (II): 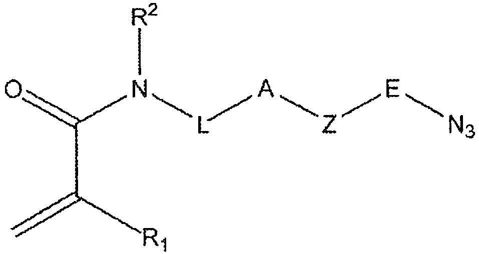 wherein R¹ is H or a C1-C6 alkyl; R₂ is H or a C1-C6 alkyl; L is a linker including a linear chain with 2 to 20 atoms selected from the group consisting of carbon, oxygen, and nitrogen and 10 optional substituents on the carbon and any nitrogen atoms in the chain; E is a linear chain including 1 to 4 atoms selected from the group consisting of carbon, oxygen and nitrogen, and optional substituents on the carbon and any nitrogen atoms in the chain; A is an N substituted amide with an H or a C1-C4 alkyl attached to the N; and Z is a nitrogen containing heterocycle. Examples of Z include 5 to 10 carbon-containing ring members present as a single cyclic structure or a fused structure. Some specific examples of Z include pyrrolidinyl, pyridinyl, or pyrimidinyl.

As still another example, the gel material may include a recurring unit of each of structure (III) and (IV): 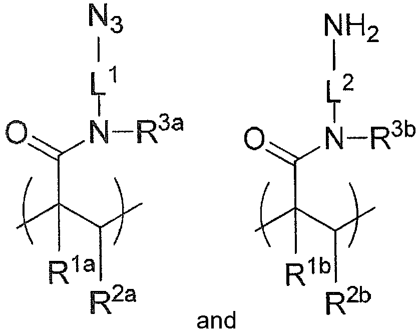 wherein each of R^{1a}, R^{2a}, R^{1b} and R^{2b} is independently selected from hydrogen, an optionally substituted alkyl or optionally substituted phenyl; each of R^{3a} and R^{3b} is independently selected from hydrogen, an optionally substituted alkyl, an optionally substituted phenyl, or an optionally substituted C7-C14 aralkyl; and each L¹ and L² is independently selected from an optionally substituted alkylene linker or an optionally substituted heteroalkylene linker.

As mentioned, in some examples, the polymeric hydrogel 32, 32' has one member 18, 18' of a visible light responsive pair attached thereto. A "visible light responsive pair" refers to two or three reagents that undergo a coupling reaction when exposed to visible light. When the pair includes two reagents, one is attached to the polymeric hydrogel 32, 32' and the other is attached to a tagmentation entity (e.g., transposome complexes), a primer, or another entity that is to be introduced to the flow cell surface. When the pair includes three reagents, one is attached to the polymeric hydrogel 32, 32', another is attached to the entity that is to be introduced to the flow cell surface, and third is present in the formulation that is introduced when the coupling reagent is to be performed.

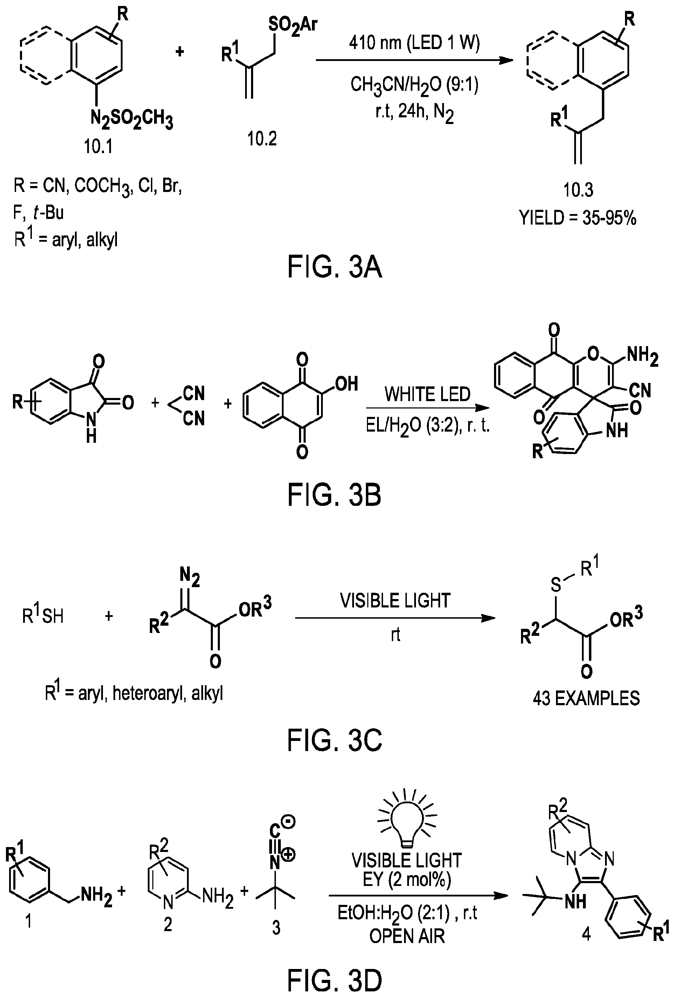

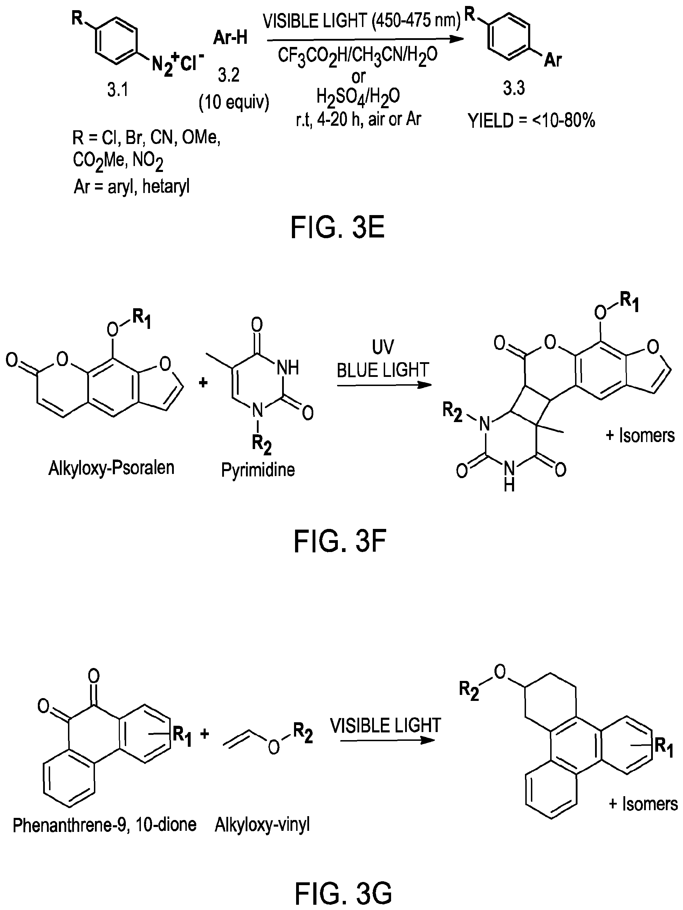

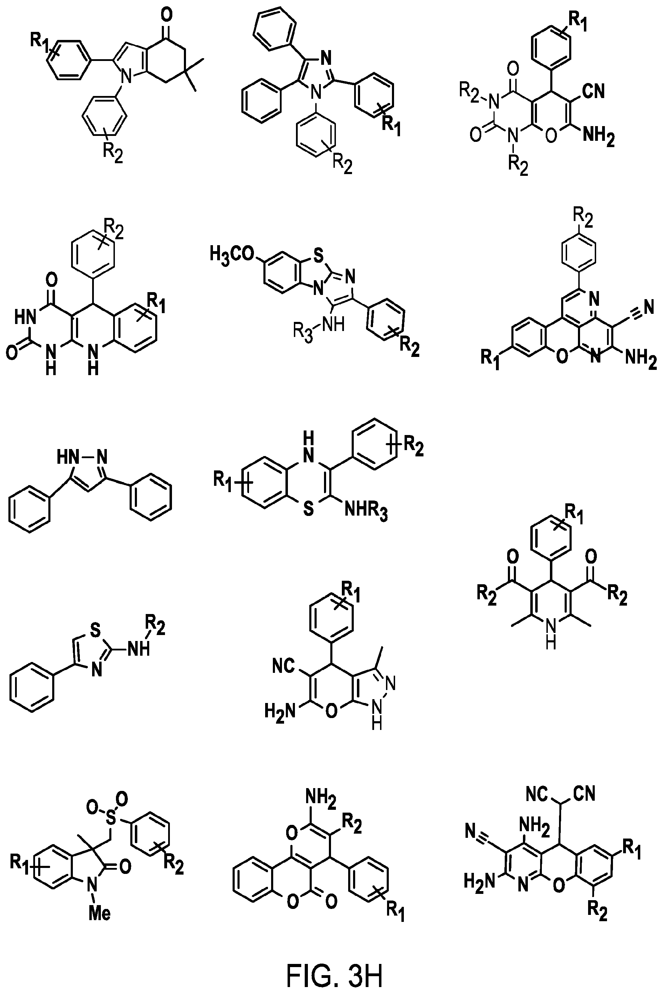

Examples of visible light responsive pairs are shown in Fig. 3A through Fig. 3H. In each of these figures, R and R1 or R1 and R2 or bold font represent(s) moieties that connect upon light activation. Either of these groups could be attached to the polymeric hydrogel 32, 32' or the entity that is to be introduced to the flow cell surface. When a third entity is used in the coupling reaction, it may be introduced with the entity that is to be introduced to the flow cell surface.

The first visible light responsive member 18, 18' may be attached to the polymeric hydrogel 32, 32' via covalent bonding or non-covalent bonding. As one example, first visible light responsive member 18, 18' may be attached to some of the R^{A} groups. Non-covalent bonding (e.g., biotin-streptavidin) may be used as long as neither member of the non-covalent binding pair can bind to the other member of the visible light responsive pair.

In another example, the flow cell architecture shown in Fig. 2A includes a biotinylated polymeric hydrogel 30, 30', which includes the biotin-containing linker 16, 16'. In these examples, biotin is attached to the surface of the polymeric hydrogel (e.g., 32, 32') through some of the R^{A} groups (e.g., the azide, tetrazine, or other functional group that can attach to an alkyne). The biotin is attached to a linker, such as bicyclo[6.1.0]nonyne (BCN), which can covalently attach to some of the R^{A} groups. The combination of the biotin and the linker is one example of the biotin-containing linker 16, 16'. In another example, streptavidin is attached to the biotin, which is attached to the linker that can covalently attach to some of the R^{A} groups. In this example, the biotin-containing linker 16, 16' includes the linker, the biotin, and the streptavidin. The biotin-containing linker 16, 16' may be added to the polymeric hydrogel (e.g., 32, 32') before or after the polymeric hydrogel (e.g., 32, 32') is applied to the depressions 26, 26' in order to form the biotinylated polymeric hydrogel 30, 30'. The streptavidin may be part of the linker 16, 16', or added to the flow cell 10 before or simultaneously with a biotinylated transposome complex or a biotinylated primer that is to be linked to the linker 16, 16'.

In still other examples, the flow cell 10 does not include either the visible light responsive member 18, 18' or the biotin-containing linker 16, 16'.

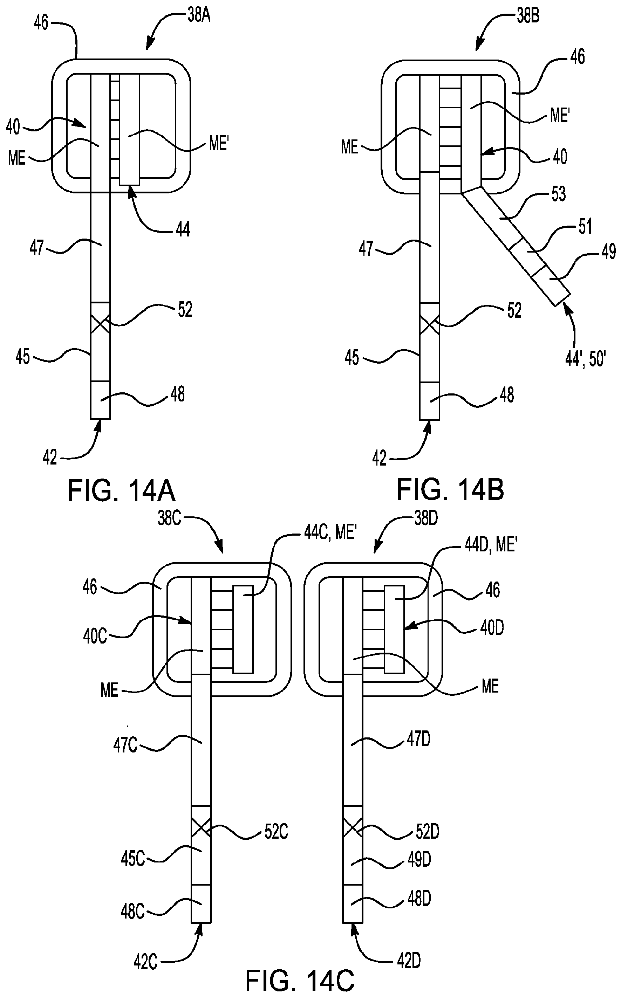

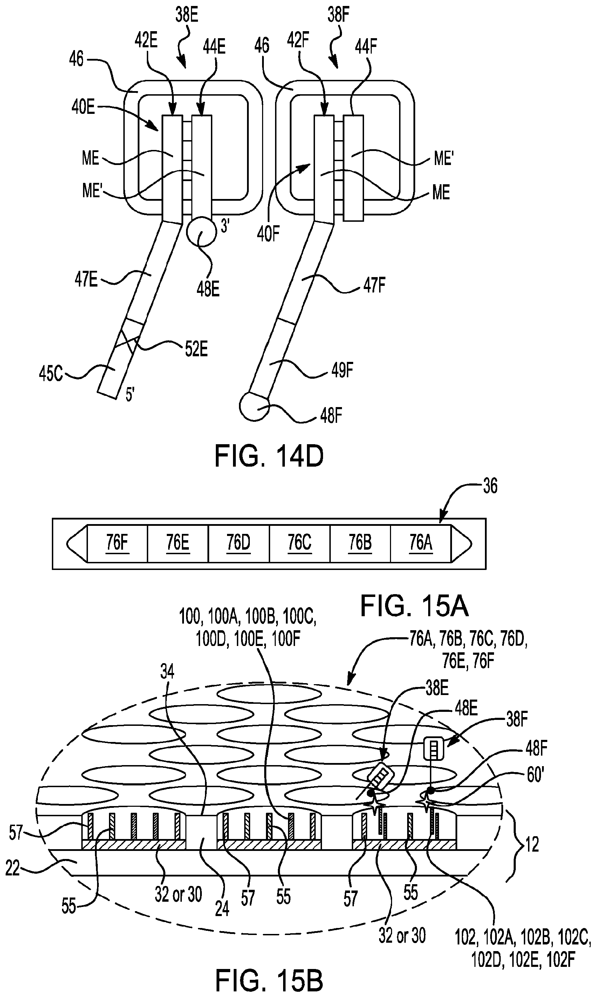

In some of these examples, the transposome complexes, such as those shown in Fig. 14C or Fig. 14D, are capable of directly binding to the R^{A} groups of the polymeric hydrogel 32, 32'. These example flow cells may include the transposome complexes bound thereto or may have them introduced as part of a method.

In other of these examples, the flow cell 10 includes target primers that are capable of hybridizing to a portion of the transposome complex. The target primers are further described in reference to Fig. 15A and Fig. 15B.

### Repurposing Flow Cell Surfaces

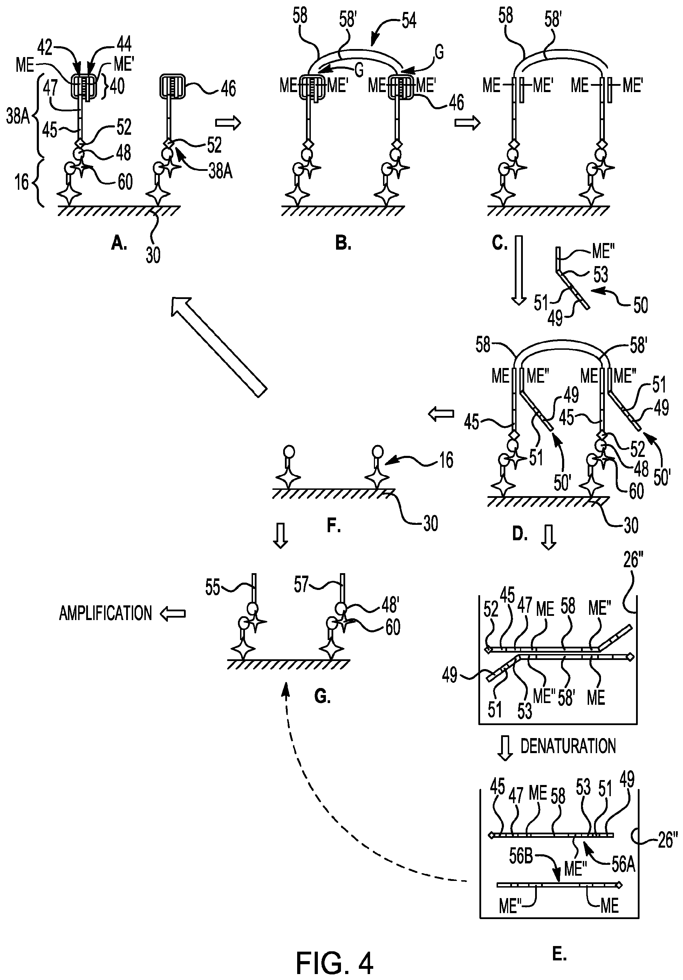

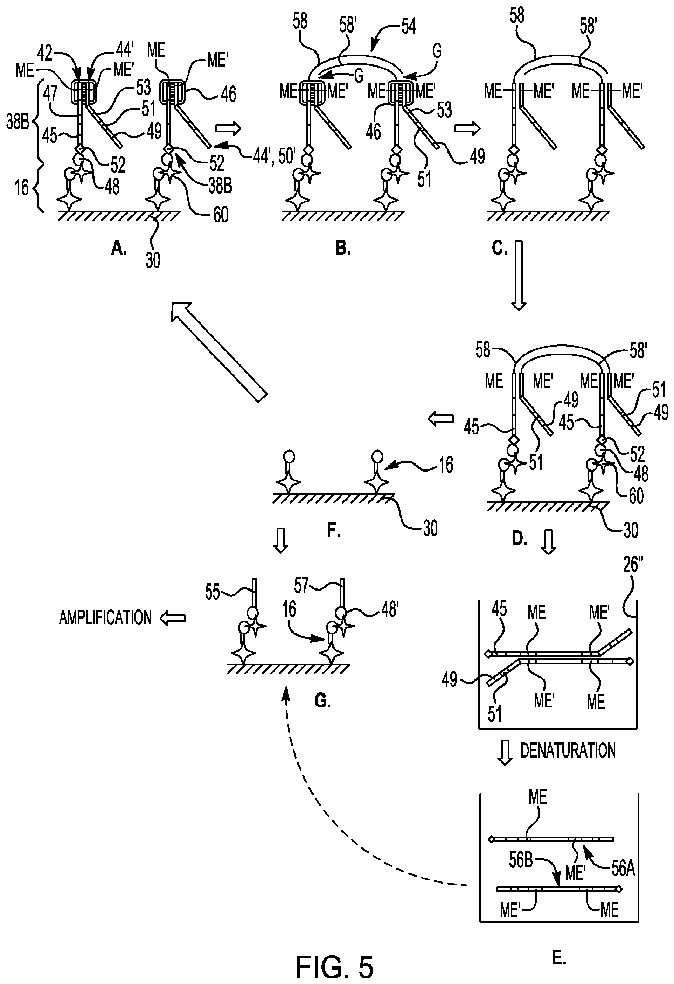

Some of the flow cells 10 may be repurposed for various applications. Example methods (shown and described in reference to Fig. 4 and Fig. 5) include generating at least partially adapted DNA sample fragments on a flow cell 10 using a plurality of transposome complexes 38A (Fig. 4 and Fig. 14A) or 38B (Fig. 5 or Fig. 14B) attached to a surface of the flow cell 10 within a flow channel 20 by a biotin-containing linker 16; cleaving the at least partially adapted DNA sample fragments such that the biotin-containing linkers 16 remain attached to the surface; and one of: using the flow cell 10 for a subsequent cycle of generating at least partially adapted DNA sample fragments; or using the flow cell 10 to amplify at least some of the previously cleaved and at least partially adapted DNA sample fragments.

These example methods are performed with the flow cells 10 that include the architecture shown in Fig. 2A. More specifically, these flow cells 10 include the substrate 12 having depressions 26 separated by interstitial regions 34; a polymeric hydrogel (specifically the biotinylated polymeric hydrogel 30, 30') positioned within each of the depressions 26; and a plurality of transposome complexes 38A or 38B immobilized within each of the depressions 26 by the biotin-containing linker 16. In one example, each of the plurality of the transposome complexes 38A, 38B is of a single type including a transposon end 40 with a portion of a transferred strand 42 hybridized to a portion of a non-transferred strand 44 or 44', wherein the transferred strand 42 includes a first amplification domain 45 and is free of an index sequence. In another example, each of the plurality of the transposome complexes 38A, 38B is of a single type including a transposon end 40 with a portion of a transferred strand 42 hybridized to a portion of a non-transferred strand 44 or 44', wherein the transferred strand 42 includes a first amplification domain 45 and also includes an index sequence (not shown in Fig. 4 or Fig. 5). During repurposing, the newly added transposome complexes 38A or 38B will include a different index sequence so that each sample is uniquely indexed.

In the examples shown in Fig. 4 and Fig. 5, the biotin-containing linker 16 includes a graftable group (e.g., BCN or any of the R^{A} groups) and biotin. As noted, streptavidin 60 may be part of the linker 16, or may be added to the flow cell 10 before or simultaneously with the transposome complex 38A, 38B.

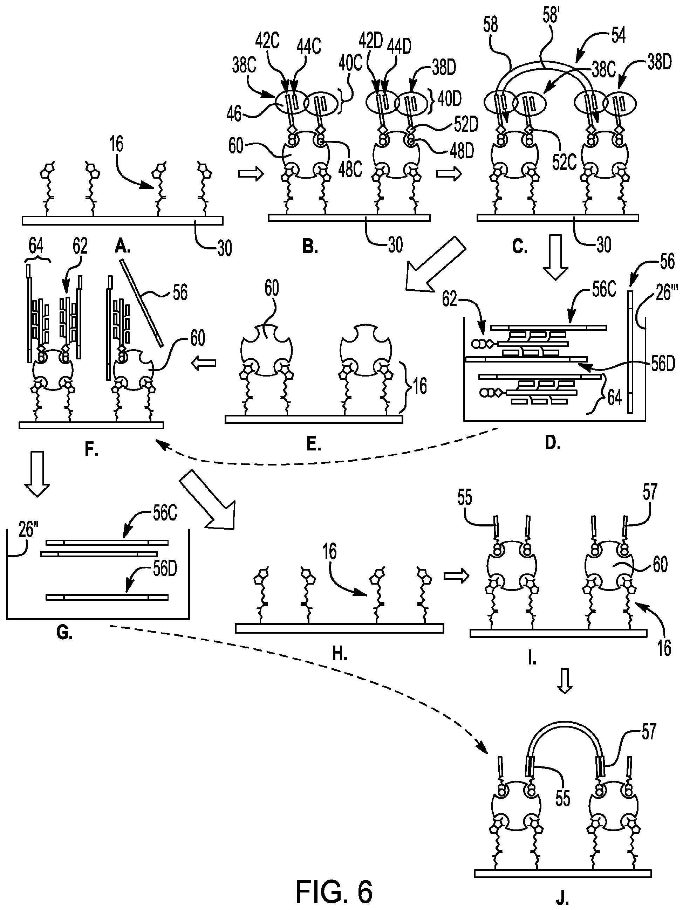

In each of Fig. 4, Fig. 14A, Fig. 5, and Fig. 14B, single transposome complexes 38A, 38B are shown. However, it is to be understood that transposome complexes 38A, 38B will form dimers in solution and when attached to the flow cell surface. It is to be understood that for simplicity, single transposome complexes are shown in Fig. 4 and Fig. 5. The dimer form of some of the transposome complexes 38A, 38B are shown, e.g., in Fig. 6.

In the example of Fig. 4 and Fig. 14A, each of the transposome complexes 38A includes a transposase enzyme 46 non-covalently bound to the transposon end 40. Each transposon end 40 is a double-stranded nucleic acid strand, one strand ME of which is part of a transferred strand 42 and the other strand ME' of which is the non-transferred strand 44. In other words, the transposon end 40 includes a portion of the transferred strand 42 that is hybridized to the non-transferred strand 44.

The transferred strand 42 includes a 5' end functional group 48. In this example, the 5' end functional group 48 is biotin. The transferred strand 42 also includes a first amplification domain 45, and a sequencing primer sequence 47 that is attached to one strand ME of the transposon end 40. The strand ME of the transposon end 40 is positioned at the 3' end of the transferred strand 42.

In this example, the first amplification domain 45 has a different sequence than a second amplification domain 49 (e.g., P5') of a subsequently introduced adapter 50 (which forms a forked adapter 50' when hybridized to the transferred strand 42 as shown at D in Fig. 4), but has the same sequence as one primer 55 (e.g., P7) that is to be introduced to the flow cell 10 (e.g., at G in Fig. 4). The first amplification domain 45 and the primer 55, together with the second amplification domain 49 and another primer 57 (e.g., P5 or P15) that is to be introduced to the flow cell surface, enable the amplification of the DNA sample fragments generated during tagmentation.

The P5 primer sequence is one of:
P5 #1: 5' → 3'
   AATGATACGGCGACCACCGAGAUCTACAC (SEQ. ID. NO. 1);
P5 #2: 5' → 3'
   AATGATACGGCGACCACCGAGAnCTACAC (SEQ. ID. NO. 2)
where "n" is inosine in SEQ. ID. NO. 2; or
P5 #3: 5' → 3'
   AATGATACGGCGACCACCGAGAnCTACAC (SEQ. ID. NO. 3)
where "n" is alkene-thymidine (i.e., alkene-dT) in SEQ. ID. NO. 3.

The P5' sequence is the complement of any of the P5 examples.

The P7 primer sequence may be any of the following:
P7 #1: 5' → 3'
   CAAGCAGAAGACGGCATACGAnAT (SEQ. ID. NO. 4)
P7 #2: 5' → 3'
   CAAGCAGAAGACGGCATACnAGAT (SEQ. ID. NO. 5)
P7 #3: 5' → 3'
   CAAGCAGAAGACGGCATACnAnAT (SEQ. ID. NO. 6)
where "n" is 8-oxoguanine in each of SEQ. ID. NOS. 4-6.

It is to be understood that other sequences may be used for the amplification domains 45, 49 (e.g., P7, P5') and for the primers 55, 57 (e.g., P7, P5), as long as the combination enables the desired amplification. As such, the designations P5, P5', and P7 are provided as examples, and the corresponding domains 45, 49 and/or primers 55, 57 are not limited to the specific sequences set forth herein. As examples, a P15, PA, PB, PC, or PD primer may be used.
The P15 primer sequence is:
P15: 5' → 3'
   AATGATACGGCGACCACCGAGAnCTACAC (SEQ. ID. NO. 7)
where "n" is allyl-T (i.e., a thymine nucleotide analog having an allyl functionality).

The other primer sequences (PA-PD) mentioned above include:
PA 5' → 3'
   GCTGGCACGTCCGAACGCTTCGTTAATCCGTTGAG (SEQ. ID. NO. 8)
PB 5' → 3'
   CGTCGTCTGCCATGGCGCTTCGGTGGATATGAACT (SEQ. ID. NO. 9)
PC 5' → 3'
   ACGGCCGCTAATATCAACGCGTCGAATCCGCAACT (SEQ. ID. NO. 10)
PD 5' → 3'
   GCCGCGTTACGTTAGCCGGACTATTCGATGCAGC (SEQ. ID. NO. 11)

While not shown in the example sequences for PA-PD, it is to be understood that any of these sequences may include a cleavage site 52, such as uracil, 8-oxoguanine, allyl-T, diols, etc. at any point in the strand. The sequences for the first amplification domain 45/primer 55 and for the second amplification domain 49/primer 57 may be selected to have orthogonal cleavage sites (i.e., one cleavage site is not susceptible to the cleaving agent used for the other cleavage site), so that after amplification, forward or reverse strands can be cleaved, leaving the other of the reverse or forward strands for sequencing.

The primers 55, 57 may also include a polyT sequence at the 5' end of the primer sequence. In some examples, the polyT region includes from 2 T bases to 20 T bases. As specific examples, the polyT region may include 3, 4, 5, 6, 7, or 10 T bases.

The sequencing primer sequences 47, 53 (the latter of which is part of the adapter 50) have different sequences from each other that respectively bind to sequencing primers introduced into the flow cell 10 after tagmentation and amplification. As examples, the sequencing primer sequence 47 may bind a sequencing primer that primes synthesis of a new strand that is complementary to forward strand fragments/fragment amplicons and the sequencing primer sequence 53 may bind a sequencing primer that primes synthesis of a new strand that is complementary to reverse strand fragments/fragment amplicons.

The transposon end 40 of each transposome complex 38A includes the strands ME respectively hybridized to the strands ME'. As such, the strands ME, ME' are complementary. The double stranded transposon end 40 is capable of complexing with the transposase 46. As examples, the strands ME, ME' of the transposon end 40 may be the related but non-identical 19-base pair (bp) outer end (e.g., strand ME) and inner end (e.g., strand ME') sequences that serve as the substrate for the activity of the Tn5 transposase, or the mosaic ends recognized by a wild-type or mutant Tn5 transposase, or the R1 end (e.g., strand ME) and the R2 end (strand ME') recognized by the MuA transposase.

At the outset of the method shown in Fig. 4 (at A), the transposome complexes 38A are attached to the biotinylated polymeric hydrogel 30 in the depression 26. Avidin or streptavidin 60 may be used to attach the 5' end functional group 48 (which in this example is biotin) with the biotinylated polymeric hydrogel 30. As examples, the avidin or streptavidin 60 may be pre-attached to the 5' end functional group 48 before the complexes 38A are introduced into the flow cell 10 and allowed to incubate; or the avidin or streptavidin 60 may be introduced into the flow cell 10 with the 5' biotinylated transposome complexes 38A and allowed to incubate; or the avidin or streptavidin 60 may be introduced into the flow cell 10 and allowed to incubate with the biotinylated polymeric hydrogel 30, and then the 5' biotinylated transposome complexes 38A may be added and allowed to incubate; or the avidin or streptavidin 60 and biotin are pre-attached to one another and then introduced into the flow cell 10 where the biotin attaches to the surface of the polymeric hydrogel through some of the R^{A} groups (directly or through a linker), and then the 5' biotinylated transposome complexes 38A may be introduced into the flow cell 10 containing the streptavidin-biotin bound linker 16 as part of the biotinylated polymeric hydrogel 30.

In this example method, fully adapted DNA fragments are generated by introducing a DNA sample 54 and a tagmentation buffer to the flow cell 10 (see B); performing tagmentation of the DNA sample 54 using the plurality of transposome complexes 38A (see B); removing the transposase enzyme 46 of each of the plurality of transposome complexes 38A (see C); replacing the non-transferred strand 44 of each of the plurality of transposome complexes 38A with an adapter 50 including a sequence, e.g., ME", complementary to the ME portion of the transferred strand 42; a sequencing primer sequence 53; an index sequence 51; and a second amplification domain 49 that is complementary to the primer 47 that is or is to be immobilized within each of the depressions 26 of the flow cell 10, thereby dehybridizing the non-transferred strand 44 and hybridizing the adapter 50 to the portion ME of the transferred strand 42 of each of the plurality of transposome complexes 38A (thus forming the forked adapter 50', see D); and performing gap fill ligation to attach the DNA sample fragments to respective forked adapters 50' (see D).

As shown at B in Fig. 4, the DNA sample (shown fragmented at reference numeral 54) is introduced into the flow cell 10 for tagmentation. The DNA sample 54 may be introduced with a tagmentation buffer, which may include water, an optional co-solvent (e.g., dimethylformamide), a metal co-factor for the transposase (e.g., magnesium acetate), and a buffer salt (e.g., Tris(hydroxymethyl) aminomethane (Tris or TRIS) acetate salt, pH 7.6). In an example, the optional co-solvent may be present in an amount up to about 11%, the metal co-factor may be present in a concentration ranging from about 3 mM to about 10 mM, and the buffer salt may be present in a concentration ranging from about 7 mM to about 12 mM.

When the DNA sample 54 is introduced into the flow cell 10 including the transposome complexes 38A, the DNA sample 54 is fragmented and the 5' ends of both strands of the duplex fragments 58, 58' are ligated to respective 3' ends of the transferred strands 42 of the transposome complexes 38A. Fragmentation and ligation may take place at a temperature at or above 30°C. In one example, the temperature may range from 30°C to about 55°C. In another example, the temperature may range from 35°C to about 45°C. The 3' ends of the duplex fragments 58, 58' are not ligated to the 5' ends of the non-transferred strands 44. As such, a gap G (shown in Fig. 4 at B) exists between the 3' end of each DNA fragment strand 58, 58' and the 5' end of the corresponding non-transferred strand 44. In one example, each gap G is nine (9) base pairs long.

As shown in Fig. 4 at C, the transposases 46 are then removed from the complexes 38A, which are now respectively attached to the fragments 58, 58' via the transferred strands 42. Transposase 46 removal may be accomplished, for example, using sodium dodecyl sulfate (SDS) or proteinase, or by heating the flow cell to about 60°C. When heat is used, some example methods involve introducing a washing solution into the flow cell 10; and heating the flow cell 10, containing the washing solution, to about 60°C. An example washing solution is an aqueous solution including a buffer agent (e.g., Tris), a salt (e.g., sodium chloride, sodium citrate, etc.), a surfactant (e.g., TWEEN polysorbates), and/or a chelating agent (e.g., EDTA). In one example, the washing solution includes water, the salt at a concentration ranging from about 25 mM to about 50 mM, the surfactant in an amount ranging from about 0.01 wt% to about 0.1 wt%, and optionally the chelating agent. The washing solution may have a relatively high pH, e.g., ranging from about 7 to about 10.

The non-transferred strand 44 of each of the plurality of transposome complexes 38A is replaced with the adapter 50, which creates the forked adapter 50' shown in Fig. 4 at D. As shown between C and D, the adapter 50 includes the sequence ME" that is complementary to the ME portion of the transferred strand 42, the sequencing primer sequence 53, the index sequence 51, and the second amplification domain 49, e.g., P5', that is complementary to the primer 47 (e.g., P5) that is or is to be immobilized within each of the depressions 26 of the flow cell 10. The index sequence 51 is a unique barcode sequence that can be used for DNA sample fragment identification and indexing. The non-transferred strand 44 is dehybridized and the adapter 50 is hybridized to the portion ME of the transferred strand 42 to create the forked adapter 50'.

Gap fill ligation is then performed to attach the DNA sample fragments 58, 58' to the sequence ME" of respective forked adapters 50' (see Fig. 4 at D). Gap fill ligation may be performed with any suitable gap fill ligation enzyme (e.g., tTaq608 polymerase, T7 exo minus polymerase, etc.) and any suitable ligase (e.g., E. coli DNA ligase, T4 DNA ligase, etc.), in combination with a solution of nucleotides. Gap fill ligation may take place at a temperature ranging from about 37°C to about 50°C for about 5 minutes.

As a result of gap fill ligation, fully adapted DNA fragments are attached to the flow cell 10. The fully adapted fragments are shown at D and include the first amplification domain 45 (e.g., P7) at one end and the second amplification domain 49 (e.g., P5') at the other end, with the respective DNA fragments 58, 58' therebetween.

The fully adapted DNA fragments are then cleaved by introducing a suitable cleaving agent for the cleavage site 52. As examples, uracil can be cleaved by Uracil-DNA glycosylase (UDG), inosine can be cleaved by Endo IV, 8-oxoguanine can be cleaved by 8-oxoguanine DNA glycosylase, and vicinal diol linkages can be cleaved by oxidation, such as treatment with a periodate reagent.

By cleaving the fully adapted DNA sample fragments at the cleavage site 52, the biotin-containing linker 16 remains attached to the surface, as shown at F in Fig. 4.

The cleaved fully adapted DNA fragments are transported into a holding receptacle 26". This is shown at E in Fig. 4. In one example, the holding receptacle 26" is well or chamber that is patterned into the substrate 12 or 14. In this example, the holding receptacle 26" is in fluid communication with the flow channel 20, but is separate from the flow channel 20. The holding receptacle 26" does not include the polymeric hydrogel 30. Alternatively, the holding receptacle may be off of the flow cell, such as in a cartridge on the instrument that receives the flow cell. Within the holding receptacle 26", the cleaved fully adapted DNA fragments are denatured to form single stranded, fully adapted DNA fragments 56A, 56B. In an example, denaturing takes place in NaOH or Tris HCl and heat (e.g., at about 90°C).

After cleaving the fully adapted DNA sample fragments such that the biotin-containing linker 16 remains attached to the flow cell surface, and prior to using the flow cell 10, the method further comprises removing streptavidin 60 from the biotin-containing linker 16. Streptavidin (or avidin) 60 removal is depicted at F in Fig. 4.

To remove streptavidin 60, a biotin streptavidin cleavage composition may be introduced into the flow cell 10. Examples of the biotin streptavidin cleavage composition include about 95% formamide and about 10mM ethylenediaminetetraacetic acid (EDTA), or from about 10% by volume to about 50% by volume of a formamide reagent and a balance of a salt buffer. At suitable reaction temperatures, these cleavage compositions disrupt the biotin-streptavidin interactions, thus releasing whatever is attached (e.g., streptavidin, the 5' end functional group 48 (biotin)) to the biotin of the biotin-containing linker 16. Alternatively, a hot wash with biotin and/or desthiobiotin causes the newly added biotin and/or desthiobiotin to compete with the already bound biotin. By disrupting the biotin-streptavidin interactions, the streptavidin 60 and the 5' end functional group 48 (biotin) are removed, leaving the biotin - BCN (i.e., one example of the biotin-containing linker 16) attached to the polymeric hydrogel 30.

The flow cell 10 may then be used for a subsequent cycle of generating new fully adapted DNA sample fragments (e.g., going from F back to A in Fig. 4), or to amplify at least some of the previously cleaved and fully adapted DNA sample fragments (e.g., the single stranded fully adapted sample fragments 56A, 56B).

When the flow cell 10 is used for the subsequent cycle of generating new fully adapted DNA sample fragments, the subsequent cycle of generating fully adapted DNA sample fragments includes: introducing a new plurality of the transposome complexes 38A into the flow channel 20, whereby at least some of the new plurality of the transposome complexes 38A attach to at least some of the biotin-containing linkers 16 within the flow channel 20; introducing a new DNA sample 54 and a new tagmentation buffer to the flow channel 20; and repeating the tagmentation, transposase enzyme 46 removal, replacement of the non-transferred strand 44 with the adapter 50, and gap fill ligation with the new DNA sample. The repeated steps may be performed in the same manner as described herein. For example, streptavidin 60 may be added with the transposome complexes 38A (which include biotin as the 5' end group 48) to attach them to the biotin-containing linkers 16. The flow cell 10 may be used again and again to prepare single stranded fully adapted fragments 56A, 56B from several different DNA samples.

When the flow cell 10 is used for amplification of at least some of the previously cleaved and fully adapted DNA sample fragments, the method involves attaching first and second primers 55, 57 (e.g., P7, P5) to at least some of the biotin-containing linkers 16 within the flow channel 20 (see G in Fig. 4); and introducing single stranded forms of the previously cleaved and fully adapted DNA sample fragments 56A, 56B into the flow cell 10.

While P5 and P7 primers are referenced, it is to be understood that other sequences may be used for the primers 55, 57, as long as the combination enables the desired amplification of the single stranded fully adapted DNA sample fragments 56A, 56B. The primers 55, 57 include a 5' end group 48' (in this example biotin) so that they can attach to the biotin-containing linkers 16 through streptavidin 60. Any of the avidin/streptavidin techniques described herein for attaching the transposome complexes 38A to the biotinylated polymeric hydrogel 30 may be used to attach the primers 55, 57.

Once the primers 55, 57 are attached, the single stranded fully adapted sample fragments 56A, 56B may be transported back to the flow channel 20, where each will seed in a depression 26 and undergo amplification. The second amplification domain 49 (e.g., P5') of the single stranded fully adapted sample fragments 56A, 56B hybridizes to the primer 57 in a depression 26. The sample fragments 56A, 56B are copied from the hybridized primers 57 (e.g., P5) by 3' extension using a high-fidelity DNA polymerase. The original sample fragments 56A, 56B are denatured, leaving the copies immobilized all around the depression 26. Isothermal bridge amplification or some other form of amplification may be used to amplify the immobilized copies. For example, the copied templates loop over to hybridize to an adjacent, complementary primer 55 (e.g., P7), and a polymerase copies the copied templates to form double stranded bridges, which are denatured to form two single stranded strands. These two strands loop over and hybridize to adjacent, complementary primers and are extended again to form two new double stranded loops. The process is repeated on each template copy by cycles of isothermal denaturation and amplification to create dense clonal clusters of amplicons within the depressions 26. Each cluster of double stranded bridges is denatured. In an example, the reverse strands are removed by specific base cleavage, leaving forward template strands. This example of clustering is referred to as bridge amplification, and is one example of the amplification that may be performed. It is to be understood that other amplification techniques may be used.

Sequencing may then be performed. In one example, sequencing by synthesis is performed by introducing a sequencing primer followed by an incorporation mix including labeled nucleotides. Optical imaging may be used to detect each instance of nucleotide incorporation.

After sequencing, the biotin streptavidin cleavage composition may again be introduced to remove the primers 55, 57, and amplicons and nascent strands attached thereto. This leaves the biotin-containing linker 16 within the depressions 26 ready for another cycle (similar to Fig. 4 at F).

In the example of Fig. 5 and Fig. 14B, each of the transposome complexes 38B includes a transposase enzyme 46 non-covalently bound to the transposon end 40. Each transposon end 40 is a double-stranded nucleic acid strand, one strand ME of which is part of a transferred strand 42 and the other strand ME' of which is a portion of the non-transferred strand 44'. In other words, the transposon end 40 includes a portion of the transferred strand 42 that is hybridized to a portion of the non-transferred strand 44'. As such, the strands ME, ME' are complementary. Any of the strands ME, ME' described herein may be used.

The transferred strand 42 includes a 5' end functional group 48. In this example, the 5' end functional group 48 is biotin. The transferred strand 42 also includes the sequencing primer sequence 47 that is attached to one strand ME of the transposon end 40, and the first amplification domain 45. While not shown, the transferred stand 42 may also include the index sequence between the sequencing primer sequence 47 and the first amplification domain 45. The strand ME of the transposon end 40 is positioned at the 3' end of the transferred strand 42.

In this example, the non-transferred strand 44' is an adapter that further includes a sequencing primer sequence 53 (attached to the strand ME'), an index sequence 51, and a second amplification domain 49 (e.g., P5') that is complementary to a primer 57 (e.g., P5) that is to be immobilized within each of the depressions 26 of the flow cell 10 (see G in Fig. 5).

The first amplification domain 45 has a different sequence than the second amplification domain 49, but has the same sequence as one primer 55 that is to be introduced to the flow cell 10 (e.g., at G in Fig. 5). The second amplification domain 49 is a complement of the other primer 57 that is to be attached to the flow cell 10. The first amplification domain 45 and the primer 55, together with the second amplification domain 49 and the primer 57, enable the amplification of the DNA sample fragments 54 generated during tagmentation. Any of the amplification domains 45, 49 may be used in this example as long as they generate single stranded, fully adapted DNA fragments 56A, 56B, and any of the primers 55, 57 may be used in this example as long as they are able to amplify the single stranded, fully adapted DNA fragments 56A, 56B that are generated.

The sequencing primer sequences 47, 53 have different sequences from each other that respectively bind to sequencing primers introduced into the flow cell 10 after tagmentation and amplification.

The index sequence 51 is a unique barcode sequence that can be used for DNA sample fragment identification and indexing. In another example, the index sequence is a unique molecular index (UMI).

At the outset of the method shown in Fig. 5 (at A), the transposome complexes 38B are attached to the biotinylated polymeric hydrogel 30 in the depression 26. Avidin or streptavidin 60 may be used to attach the 5' end functional group 48 (which in this example is biotin) with the biotinylated polymeric hydrogel 30. As examples, the avidin or streptavidin 60 may be pre-attached to the 5' end functional group 48 before the complexes 38B are introduced into the flow cell 10 and allowed to incubate; or the avidin or streptavidin 60 may be introduced into the flow cell 10 with the 5' biotinylated transposome complexes 38B and allowed to incubate; or the avidin or streptavidin 60 may be introduced into the flow cell 10 and allowed to incubate with the biotinylated polymeric hydrogel 30, and then the 5' biotinylated transposome complexes 38B may be added and allowed to incubate; or the avidin or streptavidin 60 and biotin are pre-attached to one another and then introduced into the flow cell 10 where the biotin attaches to the surface of the polymeric hydrogel through some of the R^{A} groups (directly or through a linker), and then the 5' biotinylated transposome complexes 38B may be introduced into the flow cell 10 containing the streptavidin-biotin bound linker 16 as part of the biotinylated polymeric hydrogel 30.

In this example method, fully adapted DNA fragments are generated by introducing a DNA sample 54 and a tagmentation buffer to the flow channel 10 (see B); performing tagmentation of the DNA sample 54 using the plurality of transposome complexes 38B (see B); removing the transposase enzyme 46 of each of the plurality of transposome complexes 38B (see C); and performing gap fill ligation to attach the DNA sample fragments to respective non-transferred strands 44' (e.g., the forked adapters 50', see D).

As shown at B in Fig. 5, the DNA sample 54 (shown as fragments 58, 58') is introduced into the flow cell 10 for tagmentation. The DNA sample 54 may be introduced with the tagmentation buffer as described in reference to Fig. 4.

When the DNA sample 54 is introduced into the flow cell 10 including the transposome complexes 38B, the DNA sample 54 is fragmented and the 5' ends of both strands of the duplex fragments 58, 58' are ligated to respective 3' ends of the transferred strands 42 of the transposome complexes 38B. Fragmentation and ligation may take place as described in reference to Fig. 4. The 3' ends of the fragments 58, 58' are not attached to the non-transferred strands 44', and thus respective gaps G are formed.

As shown in Fig. 5 at C, the transposases 46 are then removed from the complexes 38B, which are now respectively attached to the fragments 58, 58' via the transferred strands 42. Transposase 46 removal may be accomplished as described in Fig. 4.

Gap fill ligation is then performed to attach the DNA sample fragments 58, 58' to the non-transferred strands 44' (see Fig. 5 at D). As a result of gap fill ligation, fully adapted DNA fragments (with the first amplification domain 45 at one end and the second amplification domain 49 at the other end) are attached to the flow cell 10.

The fully adapted DNA fragments are then cleaved by introducing a suitable cleaving agent for the cleavage site 52. Any of the example cleaving agents disclosed herein may be used. By cleaving the fully adapted DNA sample fragments at the cleavage site 52, the biotin-containing linker 16 remains attached to the surface, as shown at F in Fig. 5.

The cleaved fully adapted DNA fragments are transported into a holding receptacle 26". This is shown at E in Fig. 5. Within the holding receptacle 26", the cleaved fully adapted DNA fragments are denatured to form single stranded, fully adapted DNA fragments 56A, 56B. In an example, denaturing takes place in NaOH or Tris HCl and heat.

After cleaving the fully adapted DNA sample fragments such that the biotin-containing linker 16 remains attached to the flow cell surface, and prior to using the flow cell 10, the method further comprises removing streptavidin 60 from the biotin-containing linker 16. Streptavidin 60 removal is depicted at F in Fig. 5. Streptavidin 60 and the 5' end functional group 48 (biotin) of the transferred strand 42 may be removed using the biotin streptavidin cleavage composition as described in reference to Fig. 4.

The flow cell 10 may then be used for a subsequent cycle of generating new fully adapted DNA sample fragments (e.g., going from F back to A in Fig. 5), or to amplify at least some of the previously cleaved and fully adapted DNA sample fragments (e.g., the single stranded fully adapted sample fragments 56A, 56B).

When the flow cell 10 is used for the subsequent cycle of generating new fully adapted DNA sample fragments, the subsequent cycle of generating fully adapted DNA sample fragments includes: introducing a new plurality of the transposome complexes 38B into the flow channel 20, whereby at least some of the new plurality of the transposome complexes 38B attach to at least some of the biotin-containing linkers 16 within the flow channel 20; introducing a new DNA sample 54 and a new tagmentation buffer to the flow channel 20; and repeating the tagmentation, transposase enzyme 46 removal, and gap fill ligation with the new DNA sample 54. The repeated steps may be performed in the same manner as described herein. For example, streptavidin 60 may be added with the transposome complexes 38B to attach them to the biotin-containing linkers 16. The flow cell 10 may be used again and again to prepare fully adapted fragments 56A, 56B from several different DNA samples. In these examples, the fully adapted fragments 56A, 56B from several different DNA samples may be stored in the holding receptacle 26" and then introduced when it is desirable to perform amplification of all of the fragments.

When the flow cell 10 is used for amplification of at least some of the previously cleaved and fully adapted DNA sample fragments, the method involves attaching first and second primers 55, 57 to at least some of the biotin-containing linkers 16 within the flow channel 20 (see G); and introducing single stranded forms of the previously cleaved and fully adapted DNA sample fragments 56A, 56B into the flow cell 10.

While P5 and P7 primers are referenced, it is to be understood that other sequences may be used for the primers 55, 57, as long as the combination enables the desired amplification of the single stranded fully adapted DNA sample fragments 56A, 56B. The primers 55, 57 include biotin at the 5' end so that they can attach to the biotin-containing linkers 16 through streptavidin 60. Any of the avidin/streptavidin techniques described herein for attaching the transposome complexes 38B to the biotinylated polymeric hydrogel 30 may be used to attach the primers 55, 57.

Once the primers 55, 57 are attached, the single stranded fully adapted sample fragments 56A, 56B may be transported back to the flow channel 20, where each will seed in a depression 26 and undergo amplification as described in reference to Fig. 4. Sequencing may then be performed. After sequencing, the biotin streptavidin cleavage composition may again be introduced to remove the primers 55, 57, and amplicons and nascent strands attached thereto. This leaves the biotin-containing linker 16 within the depressions 26 ready for another cycle.

In the examples shown in Fig. 4 and Fig. 5, the single stranded fully adapted DNA sample fragments 56A, 56B may alternatively be prepared as described herein, and then introduced into another type of flow cell that has the primers 55, 57 attached to a non-biotinylated polymeric hydrogel 32 in the depressions 26. This is a single use flow cell, and the surface would not be repurposed after amplification and sequencing. In these examples, the 5' end functional group 48 of the transposome complexes 38A, 38B may be any functional group that can attach to the R^{A} groups of the polymeric hydrogel 32.

The flow cell 10 including the biotinylated polymeric hydrogel 30 may also be used for enrichment. Enrichment enables a target portion of the DNA sample 54, e.g., specific genetic variants in a given sample, to be isolated and analyzed. For this type of analysis, the target portion or region of interest may be enriched, e.g., which helps to separate the target portion from the remainder of the DNA sample 54. This enables the sequencing reads to be dedicated to the target portion. The enrichment techniques disclosed herein utilize on-flow cell hybridization and capture of the tagmented DNA fragments 58, 58' that correspond to the target portion. Examples are shown in Fig. 6, Fig. 7, and Fig. 8.

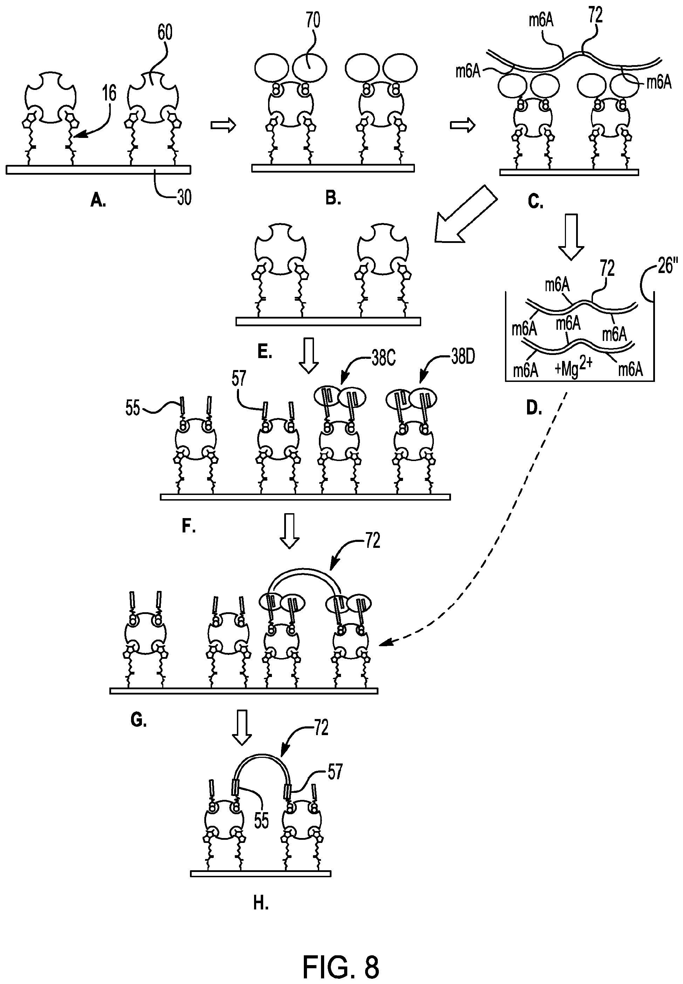

In each of the examples shown in Fig. 6 and Fig. 8, the plurality of the transposome complexes may include first transposome complexes 38C including a first amplification domain 45C, e.g., P7, and second transposome complexes 38D including a second amplification domain 49D, e.g., P5. These transposome complexes 38C, 38D are shown in more detail in Fig. 14C. In Fig. 14C, one of each complex 38C, 38D form a dimer, and in Fig. 6 and Fig. 8, two of the same complexes 38C or 38D form a dimer.

As shown in Fig. 14C, each of the first and second transposome complexes 38C, 38D includes the transposase enzyme 46 non-covalently bound to the transposon end 40C, 40D. In this example, each transposon end 40C, 40D is a double-stranded nucleic acid strand, one strand (e.g., ME) of which is part of a transferred strand 42C or 42D and the other strand (e.g., ME') of which is part of a non-transferred strand 44C or 44D. Any of the example strands for the transposon end 40 (e.g., ME and ME') described herein may be used.

In the example shown in Fig. 14C, the transferred strand 42C includes a 5' end functional group 48C (in this case biotin) that is capable of non-covalently attaching to avidin/streptavidin (shown at reference numeral 60 in Fig. 6), the first amplification domain 45C, and the sequencing primer sequence 47C that is attached to one strand of the transposon end 40C (which is positioned at the 3' end of the transferred strand 42C). In some examples, the transferred strand 42C includes an index sequence between the first amplification domain 45C and the sequencing primer sequence 47C. Similar to the transferred strand 42C, the transferred strand 42D includes a 5' end functional group 48D (in this case biotin) that is capable of covalently attaching to avidin/streptavidin, a second amplification domain 49D, and a sequencing primer sequence 47D that is attached to one strand (ME) of the transposon end 40D (which is positioned at the 3' end of the transferred strand 40D).

The first and second amplification domains 45C, 49D of the transposome complexes 38C, 38D have different sequences from each other (e.g., P7 and P5), but have the same sequence, respectively, as first and second primers (shown as 55 and 57 in I of Fig. 6) to be introduced to the biotinylated polymeric hydrogel 30. The first amplification domain 45C, the transposome complex 38C and the primer 55 together with the second amplification domain 49D, the transposome complexes 38D and the primer 57 enable the amplification of the DNA sample fragments 58, 58' generated during tagmentation. Examples of suitable sequences for the first amplification domain 45C/primer 55 and for the second amplification domain 49D/primer 57 include any of the examples set forth herein.

While not shown, it is to be understood that in each of the examples shown in Fig. 6 and Fig. 8, the plurality of the transposome complexes may alternatively include first transposome complexes 38E including a first amplification domain 45E, e.g., P7, and second transposome complexes 38F including a second amplification domain 49F, e.g., P5. These transposome complexes 38E, 38F are shown in more detail in Fig. 14D. In Fig. 14D, one of each complex 38E, 38F form a dimer, although it is to be understood that two of the same complexes 38E or 38F could form a dimer.

As shown in Fig. 14D, each of the first and second transposome complexes 38E, 38F includes the transposase enzyme 46 non-covalently bound to the transposon end 40E, 40F. In this example, each transposon end 40E, 40F is a double-stranded nucleic acid strand, one strand (e.g., ME) of which is part of a transferred strand 42E or 42F and the other strand (e.g., ME') of which is part of a non-transferred strand 44E or 44F. Any of the example strands for the transposon end 40 (e.g., ME and ME') described herein may be used.

In the transposome complex 38E, the transferred strand 42E includes a first amplification domain 45E and a sequencing primer sequence 47E that is attached to one strand ME of the transposon end 40E. In some examples, the transferred strand 42F includes an index sequence between the first amplification domain 45E and the sequencing primer sequence 47E. The strand ME of the transposon end 40E is positioned at the 3' end of the transferred strand 42E. Similar to the transferred strand 42E, the transferred strand 42F of the transposome complex 38F includes a second amplification domain 49F and a sequencing primer sequence 47F that is attached to one strand ME' of the transposon end 40F. The strand ME of the transposon end 40F is positioned at the 3' end of the transferred strand 42F.

The first and second amplification domains 45E, 49F of the transposome complexes 38E, 38F have different sequences from each other (e.g., P7 and P5), but have the same sequence, respectively, as first and second primers (shown as 55 and 57 in I of Fig. 6) to be introduced to the biotinylated polymeric hydrogel 30. The first amplification domain 45E, the transposome complex 38E and the primer 55 together with the second amplification domain 49F, the transposome complexes 38F and the primer 57 enable the amplification of the DNA sample fragments 58, 58' generated during tagmentation. Examples of suitable sequences i) for the first amplification domain 45E are the same as those set forth herein for the first amplification domain 45C/primer 55 and ii) for the second amplification domain 49F are the same as those set forth herein for the second amplification domain 49D/primer 57.

Similar to the sequencing primer sequences 47C, 47D, the sequencing primer sequences 47E, 47F have different sequences from each other that respectively bind to sequencing primers introduced into the flow cell after tagmentation and amplification.

The transposome complexes 38E, 38F are configured for asymmetric attachment to the flow cell surface (as shown in Fig. 11A). As such, one of the complexes, e.g., complex 38E, includes 3' end group 48E for attachment to the flow cell surface, and the other of the complexes, e.g., complex 38F, includes a 5' end group 48F for attachment to the flow cell surface. Thus, as depicted in Fig. 14D, the non-transferred strand 44E of the complex 38E includes the 3' end group 48E and the transferred strand 42F of the complex 38F includes the 5' end group 48F. The 3' end group 48E and the 5' end group 48F may be any functional group that is capable of covalently or non-covalently attaching, directly or indirectly, to surface functional groups of the polymeric hydrogel 30, 32 and thus will depend upon the surface functional groups of the polymer hydrogel 30, 32. In one example, the polymeric hydrogel 32 includes azide or tetrazine surface groups, and the 3' end group 48E and the 5' end group 48F each include a terminal alkyne (e.g., hexynyl) or an internal alkyne, where the alkyne is part of a cyclic compound (e.g., bicyclo[6.1.0]nonyne (BCN)). In another example, the biotinylated polymeric hydrogel 30 is present in the depressions 26 of the flow cell 10, and each of the 3' end group 48E and the 5' end group 48F is biotin. In these examples, additional streptavidin or avidin 60 is added to indirectly attach the biotin groups to one another as described herein. Still other reactive pairs (e.g., R^{A} of the polymeric hydrogel and 48) include tetrazine/TCO, amine/carboxylic acid, amines/alkyl halydes, thiol/alkene, or thiol/carboxylic acid. Tagmentation involving the transposome complexes 38E, 38F is performed may be performed in the same manner as described herein for the transposome complexes 38C, 38D.

Referring back to Fig. 6, in this example method, generating the fully adapted DNA sample fragments (e.g., 56C, 56D shown in D of Fig. 6) includes introducing a DNA sample 54 and a tagmentation buffer to the flow cell 10 (see C of Fig. 6); performing tagmentation of the DNA sample 54 using the plurality of transposome complexes 38C, 38D (see C of Fig. 6); removing a transposase enzyme 46 of each of the plurality of transposome complexes 38C, 38D (not shown); and performing an extension reaction (see C of Fig. 6). DNA sample 54 introduction, tagmentation, and transposase enzyme 46 removal may be performed as described herein in reference to Fig. 4.

When SDS or another chaotropic detergent has been used for transposase removal, the washing solution may be flushed through the flow channel prior to initiating the extension reaction. This removes the chaotropic detergent, which may interfere with downstream enzyme activity.

To initiate the extension reaction, an extension amplification mix is introduced into the flow cell 10. An example of the extension amplification mix includes nucleotides, a recombinase, a polymerase, and accessory proteins. The extension amplification mix may also include a buffer agent (e.g., Tris), enzymes, stabilizers, a metal co-factor, a surfactant (e.g., TWEEN polysorbates), and/or a co-solvent (e.g., glycerol, dimethylformamide, etc.). The ExAMP reagents available from Illumina Inc. are examples of suitable extension amplification mixes.

The flow cell 10 may be up to 60°C (e.g., at about 38°C) when the extension amplification mix is introduced.

At the outset of the extension reaction, the non-transferred strands 44C, 44D are dehybridized. Additional sequences (adapters) are added to the 3' ends of the partially adapted fragments (fragments 58, 58') by an extension reaction using the extension amplification mix. The extension reaction involves the addition of nucleotides in a template dependent fashion from the 3' ends of the DNA fragments 58, 58' using the respective transferred strands 42C, 42D as the template. This extension is represented by the arrows in C of Fig. 6. As such, the DNA fragment 58 is extended along the transferred strand 42D to generate complementary sections of the sequencing primer sequence 47D and the second amplification domain 49D attached to the DNA fragment 58; and the DNA fragment 58' is extended along the transferred strand 42C to generate complementary sections of the sequencing primer sequence 47C and the first amplification domain 45C attached to the DNA fragment 58'. The sequences resulting from the extension reaction render the partially adapted fragments 58, 58' (i.e., the tagmented fragments that have not been ligated, extended, etc.) fully adapted. At least some of the fully adapted fragments that are generated along the transposome complex 38D include the first amplification domain 45C (e.g., P7) at one end and a complement of the second amplification domain 49D (e.g., P5') at the other end. At least some of the fully adapted fragments that are generated along the transposome complex 38C include the second amplification domain 49D (e.g., P5) at one end and a complement of the first amplification domain 45C (e.g., P7') at the other end.

The fully adapted DNA fragments are then cleaved by introducing a suitable cleaving agent for the cleavage site 52. Cleavage may be performed as described in reference to Fig. 4. The double stranded, fully adapted DNA fragments are released from the flow cell surface.

The method shown in Fig. 6 then includes generating an enriched sample by denaturing the fully adapted DNA sample fragments to generate single stranded, fully adapted DNA sample fragments 56C, 56D (see D), wherein some of the single stranded, fully adapted DNA sample fragments 56C, 56D include a targeted region (i.e., a region of interest for analysis); transporting the single stranded, fully adapted DNA sample fragments 56C, 56D to an enrichment receptacle 26‴; and introducing biotinylated capture probes 62 to the enrichment receptacle 26‴, whereby the some of the single stranded, fully adapted DNA sample fragments 56C, 56D with the targeted region respectively hybridize to the biotinylated capture probes 62 to form enriched complexes 64 (as shown in D of Fig. 6), and some other of the single stranded, fully adapted DNA sample fragments 56 without the targeted regions remain unattached. The enrichment receptacle 26‴ may be a separate compartment on the flow cell 10, or may be off of the flow cell 10, such as in a cartridge on the instrument that receives the flow cell 10.

The biotinylated capture probe 62 includes i) one or more single stranded deoxyribonucleic acid sequences that is/are complementary to the targeted region(s), and ii) biotin at an end for subsequent attachment with the avidin/streptavidin 60 on the flow cell surface. In its simplest form, the biotinylated capture probe 62 includes a single stranded sequence that is complementary to a target region of the DNA sample, with biotin at the end.

As shown in Fig. 6, one example of a biotinylated capture probe 62 is a polynucleotide bottlebrush polymer structure. This polynucleotide bottlebrush polymer structure is described in International Publication No. WO 2022/220748, which is incorporated herein by reference in its entirety. In short, a polynucleotide bottlebrush polymer includes a substantially linear polynucleotide backbone, including a sequence of nucleotides connected to one another by phosphodiester bonds, and polynucleotide side chains that are connected to a nucleobase of a nucleotide of the polynucleotide backbone by one or more covalent bonds or by one or more non-covalent bonds. The sequence of the side chain may be complementary to the targeted region, and thus the side chains may be used as probes for hybridizing only those single stranded, fully adapted DNA sample fragments 56C, 56D containing the targeted region. In some examples, the side chains include coordinated complementary sequences in order to enhance the enrichment strength. In other examples, the side chains may be interlocking probes. In still other examples, the side chains may be conjugated to a peptide rather than a linear polynucleotide backbone.

The enrichment receptacle 26‴ is heated, and the biotinylated capture probe 62 (e.g., one or more side chains thereof) will hybridize to the target region of some of the single stranded, fully adapted DNA sample fragments 56C, 56D. This forms the enriched complexes 64.

While not shown in Fig. 6, after cleaving the fully adapted DNA sample fragments 56, 56C, 56D, the biotin-containing linker 16 remains attached to the surface within the depressions 26. The 5' end biotin 48 also remains attached through the streptavidin 60. Thus, after cleavage and prior to using the flow cell 10 amplification of the enriched sample, the method further comprises removing streptavidin 60 from the biotin-containing linker 16. Streptavidin 60 removal may be performed as described in Fig. 4. The resulting surface is similar to F in each Fig. 4 and Fig. 5.

The method shown in Fig. 6 then includes replenishing streptavidin 60 in the flow channel 20 (see E in Fig. 6); transporting the enriched sample (from the enrichment receptacle 26"') to the flow channel 20 including the replenished streptavidin 60, whereby the enriched complexes 64 attach to the biotin-containing linkers 16 (through the biotin on the probe 62) and the single stranded, fully adapted DNA sample fragments 56 without the targeted regions remain unattached; introducing a wash solution to the flow channel 20 to remove the single stranded, fully adapted DNA sample fragments 56 without the targeted regions; releasing the single stranded, fully adapted DNA sample fragments 56C, 56D with the targeted region from the biotinylated capture probes 62; and transporting the single stranded fully adapted DNA sample fragments 56C, 56D with the targeted region to a holding receptacle 26" (see G in Fig. 6).

In Fig. 6, at F, the biotin of the enriched complexes 64 attaches to the surface bound avidin/streptavidin 60, and the non-complexed fragments 56 remain free in solution. These free and non-complexed fragments 56 are removed from the flow channel 20 by flushing a washing solution through the flow channel 20. The enriched complexes 64, and thus the fragments 56C, 56D with the target region, remain in the flow channel 20. By removing the fragments 56 that do not include the target region, the remaining sample is enriched.

The bound single stranded, fully adapted DNA sample fragments 56C, 56D (with the targeted region) can then be released from the biotinylated capture probes 62; and transported to the holding receptacle 26" (see G in Fig. 6). The release involves dehybridization from the probe 62, and may be performed in the presence of NaOH or Tris-HCl and heat. While the single stranded, fully adapted DNA sample fragments 56C, 56D are removed and moved to the receptacle 26", the probes 62 will remain attached to the surface bound streptavidin 60.

While the fragments 56C, 56D are in the holding receptacle 26", the method further comprises removing streptavidin 60 from the biotin-containing linker 16 in the flow channel 20 (see H in Fig. 6); and attaching first and second primers 55, 57 to at least some of the biotin-containing linkers 16 within the flow channel 20 (see I in Fig. 6). Both of these processes may be performed as described herein in reference to Fig. 4. It is to be understood that the primer 55, 57 are biotinylated for attachment to the linker bound avidin/streptavidin 60.

The fragments 56C, 56D are then transported from the holding receptacle 26" back to the flow channel 20, where they will hybridize to one of the primers 55, 57 and undergo amplification as described herein. Two amplicons of one of the single stranded, fully adapted DNA sample fragments 56C, 56D are shown at J in Fig. 6.

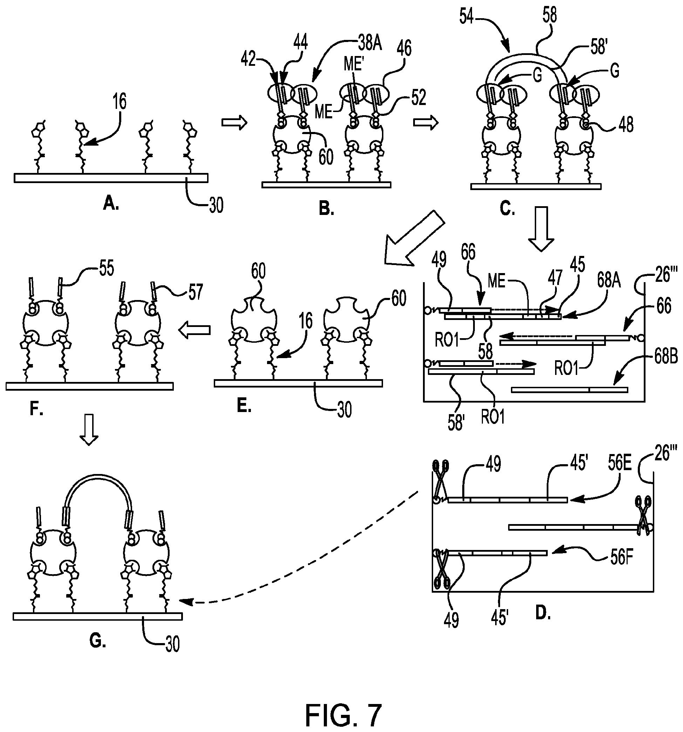

Referring now to Fig. 7, another example of enrichment is depicted. This may be desirable for blood, saliva, or small cancer panels.

In this example, the biotinylated polymeric hydrogel 30 (within biotin-containing linkers 16) are present in the depressions 16 of the flow cell 10 (see A of Fig. 7). In this example, each of the plurality of the transposome complexes 38A is of a single type including the transposon end 40 with a portion of a transferred strand 42 hybridized to a non-transferred strand 44. The transposome complexes 38A are those described in reference to Fig. 4, can be immobilized on the flow cell 10 using avidin/streptavidin 60 as described herein.

In this example, tagmentation forms partially adapted DNA sample fragments 58, 58', and thus the gap G (shown in Fig. 7 at C) exists between the 3' end of each DNA fragment strand 58, 58' and the 5' end of the corresponding non-transferred strand 44. The formation of these fragments 58, 58' involves introducing a DNA sample 54 and a tagmentation buffer to the flow channel 20; performing tagmentation of the DNA sample 54 using the plurality of transposome complexes 38A; and removing the transposase enzyme 46 of each of the plurality of transposome complexes 38A. The resulting fragments are partially adapted with the first amplification domain 45 (e.g., P7) of the transferred strand 42 of the transposome complex 38A.

Without performing gap fill ligation, the partially adapted fragments (with the non-transferred strands 42 still attached) may be cleaved at cleavage site 52 using a cleaving agent as disclosed herein. The method then further includes denaturing the partially adapted DNA sample fragments from each other and to remove the non-transferred strands 44 to generate single stranded partially adapted DNA sample fragments 68A, 68B (shown at D in Fig. 7), wherein some of the single stranded partially adapted DNA sample fragments 68A include a targeted region (shown as ROI (region of interest) in D in Fig. 7); transporting the single stranded, partially adapted DNA sample fragments 68A, 68B to an enrichment receptacle 26‴, the enrichment receptacle 26‴ including surface bound adapters 66 having a second amplification domain 49 (e.g., P5) and a region complementary to the targeted region, whereby the some of the single stranded partially adapted DNA sample fragments 68A with the targeted region ROI respectively hybridize to the surface bound adapters 66, and some other of the single stranded partially adapted DNA sample fragments 68B without the targeted regions ROI remain unattached (see D); introducing a wash solution to the enrichment receptacle 26‴ to remove the single stranded partially adapted DNA sample fragments 68B without the targeted regions ROI; and performing an extension reaction along the surface bound adapters 66, thereby generating fully adapted DNA sample fragments (see bottom of D). The enrichment receptacle 26‴ may be on the flow cell 10 or off of the flow cell, e.g., in a cartridge, as described herein.

In this example, the second amplification domain 49 has the same sequence as the primer 57 (e.g., P5) that is to be introduced into the flow cell 10, and the resulting fully adapted fragments 56E, 56F will include a complement 45' of the first amplification domain 45 (e.g., P7) of the transposome complex 38A. Thus, the fully adapted fragments56E, 56F shown in the bottom part of D include 45' (e.g., P7'), which can hybridize to the flow cell primer 55 (e.g., P7) to initiate amplification. In the enrichment receptacle 26"', the extension reaction may be performed as described herein. The resulting fully adapted fragments 56E, 56F can then be cleaved from the receptacle 26‴.

While the fragments 56E, 56F are in the enrichment receptacle 26"', the method further comprises removing streptavidin 60 from the biotin-containing linker 16 in the flow channel 20; and attaching first and second primers 55, 57 (e.g., P7, P5) to at least some of the biotin-containing linkers 16 within the flow channel 20 (see F in Fig. 7). Both of these processes may be performed as described herein in reference to Fig. 4. In particular, streptavidin 60 is first removed to also remove the 5' end biotin that remains after cleavage of the partially adapted DNA fragments. Then, the streptavidin 60 is replenished (see E in Fig. 7) prior to primer 55, 57 attachment. It is to be understood that the primer 55, 57 are biotinylated for attachment to the linker bound avidin/streptavidin 60.

After enrichment (D in Fig. 7) and primer 55, 57 attachment (F in Fig. 7), the fragments 56E, 56F are then transported from the enrichment receptacle 26‴ back to the flow channel 20, where they will hybridize to one of the primers P7, P5 and undergo amplification as described herein.

The example methods shown in Fig. 8 includes capturing a plurality of biotinylated dDpn1 70 at a plurality of biotin-containing linkers 16 attached to a surface within a flow channel 20 of a flow cell 10 (A and B in Fig. 8); introducing a DNA sample to the flow cell 10, whereby methylated bacterial DNA 72 within the DNA sample is captured by the biotinylated dDpn1 70 (C in Fig. 8); introducing a wash solution to the flow channel 20 to remove uncaptured DNA sample therefrom (not shown); releasing the methylated bacterial DNA 72 from the biotinylated dDpn1 70 such that the biotin-containing linker 16 remains attached to the surface (E in Fig. 8); transporting the released methylated bacterial DNA 72 to a holding receptacle 26" (D in Fig. 8); while the released methylated bacterial DNA 72 is in the holding receptacle 26": replenishing streptavidin 60 from the biotin-containing linker 16 (replacing or removing and replacing the streptavidin used in A-C); introducing a plurality of biotinylated transposome complexes 38C, 38D to the flow channel 20, whereby the plurality of biotinylated transposome complexes 38C, 38D attach to some of the biotin-containing linkers 16 (F in Fig. 8); and introducing a plurality of biotinylated primers 55, 57 to the flow channel 20, whereby the plurality of biotinylated primers 55, 57 attach to some other of the biotin-containing linkers 16; and transporting the released methylated bacterial DNA 72 back to the flow channel 20 containing the biotinylated transposome complexes 38C, 38D and the biotinylated primers 55, 57 for tagmentation and amplification (G and H in Fig. 8).

The biotinylated dDpn1 70 is attached to the flow cell surface via streptavidin 60 and the biotin-containing linkers 16. When DNA sample is introduced into the flow cell 10, methylated bacterial DNA 72 within the DNA sample is captured by the biotinylated dDpn1 70.

After methylated bacterial DNA 72 capture, any of the wash solutions disclosed herein may be used to remove uncaptured DNA sample from the flow cell 10.

Once the uncaptured DNA sample and wash solution are removed, the methylated bacterial DNA 72 may then be released from the biotinylated dDpn1 70 using a solution containing Mg²⁺, proteinase K, or SDS. Proteinase K or SDS is likely to release the biotinylated dDpn1 70 and the methylated bacterial DNA 72. In the latter instances, the biotinylated dDpn1 70 would be separated from the methylated bacterial DNA 72.

In one specific example, Mg²⁺ is used to release the captured methylated bacterial DNA 72. In this example, after the methylated bacterial DNA 72 is released, it is transported to the holding receptacle 26" while the biotinylated dDpn1 70 is removed. In one example, the biotinylated dDpn1 70 is removed with the streptavidin 60. In this example, the biotin streptavidin cleavage composition may be introduced into the flow cell 10, which will break the biotin-streptavidin interactions, thus releasing the biotinylated dDpn1 70 from the streptavidin 60 and releasing the streptavidin from the biotin-containing linker 16. In another example, the biotinylated dDpn1 70 may include another cleavable group, and a cleaving agent for that group may be used to remove the biotinylated dDpn1 70. As examples, the cleaving agent may be an enzymatic cleaving agent or an acid. Proteinase K or SDS may be used to remove the biotinylated dDpn1 70.

The replenishment of the streptavidin 60, introduction of the transposome complexes 38C, 38D and the biotinylated primers 55, 57, tagmentation, and amplification may be performed as described herein.

### Light Activating Flow Cell Surface

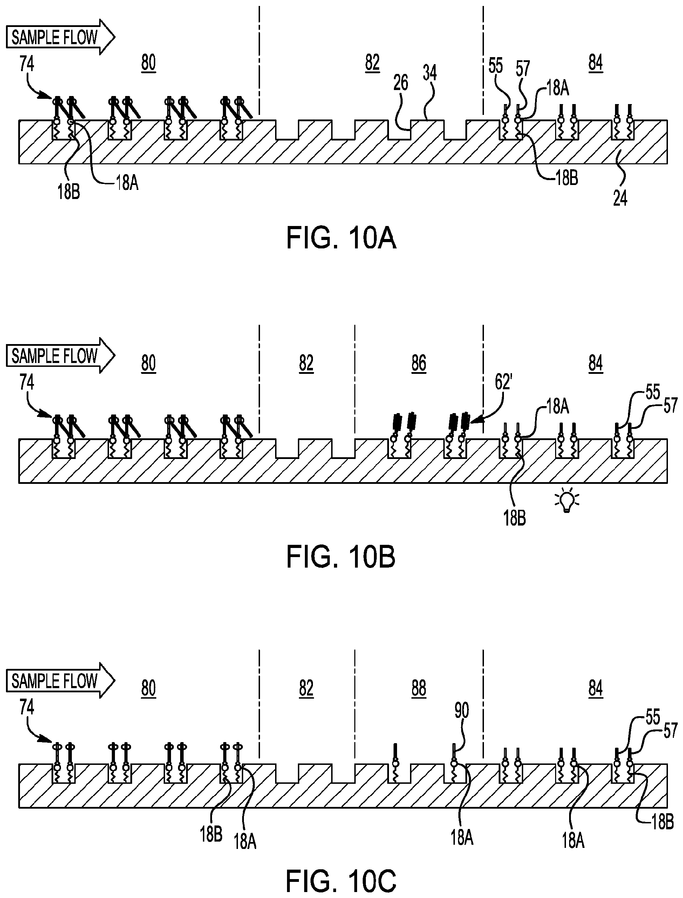

Other examples of the flow cell 10 include the visible light responsive member 18, which enables at least a portion of the flow cell surface to be selectively activated at a desirable time for a desirable reaction. These flow cells 10 may be used in a method that includes introducing a tagmentation entity (e.g., transposome complexes 38), having a first visible light responsive member 18A attached thereto, to a flow cell 10 having a second visible light responsive member 18B attached thereto; and exposing a predetermined area of the flow cell 10 to visible light while the tagmentation entity is present in the flow cell 10, thereby coupling the first and second visible light responsive members 18A, 18B and attaching the tagmentation entity to the flow cell 10 at the predetermined area. Several example methods are shown in Fig. 9A through Fig. 9D and Fig. 10A through Fig. 10C.

In these examples, the tagmentation entity 74 may be any of the transposome complexes 38A, or 38B, or 38C and 38D, or 38E and 38F described herein. The process that is performed to generate the fully adapted DNA fragments 56 (e.g., gap fill ligation, extension reaction, etc.) will depend upon the type of transposome complexes 38A, or 38B, or 38C and 38D, or 38E and 38F that is used.

Also in these examples, the visible light responsive members 18A, 18B may be any examples of the visible light responsive members 18 described herein in reference to Fig. 3A through Fig. 3H. It is to be understood that the visible light responsive members 18A may be used in place of the 5' end functional group 48 in the complex 38A or 38B; the visible light responsive members 18A may be used in place of the 5' end functional groups 48C, 48D in the respective complexes 38C, 38D; or the visible light responsive members 18A may be used in place of the 5' end functional group 48F of the complex 38F and the 3' end functional group 48E of the complex 38E.

In the examples shown in Fig. 9A through Fig. 9D, the flow cell 10 is similar to that shown in Fig. 2A (although the base support 22 is not shown) and includes the visible light responsive members 18B attached to the polymeric hydrogel 32 in the depressions 26. In this example, the flow cell 10 has the same surface chemistry in each of the depressions 26. Alternatively, the flow cell 10 shown in Fig. 2B may be used, and the visible light responsive members 18B may be positioned along the lane 36. While not shown, each flow cell depression 26 also has the primers 55, 57 attached to the polymeric hydrogel 32.

When the tagmentation entity 74 is introduced into the flow channel 20, a specific region (e.g., region 76A in Fig. 9A, region 76B in Fig. 9D) of the flow cell 10 is exposed to visible light (from light source 78), and the exposed visible light responsive members 18A, 18B undergo a coupling reaction to attach the tagmentation entity 74 within the depressions 26. Any regions (e.g., 76B, 76C in Fig. 9A) of the flow cell 10 that are not exposed to visible light will not attach the tagmentation entity 74, and thus can be activated for a subsequently introduced sample. This allows different DNA samples to be attached within different regions 76A, 76B, 76C of a single flow channel 20, and thus enables spatial indexing to be used for sample identification. The data generated during sequencing can be de-multiplexed using analysis of the different regions 76A, 76B, 76C.

In the example shown in Fig. 10A through Fig. 10C, the flow cell 10 is similar to that shown in Fig. 2A (except that the base support 22 is not shown) and includes the visible light responsive members 18B attached to the polymeric hydrogel 32 either in some of the depressions 26. Alternatively, the flow cell 10 shown in Fig. 2B may be used, and the visible light responsive members 18B may be positioned along the lane 36.

In Fig. 9A, the tagmentation entity 74 includes the transposome complexes 38E and 38F having the visible light responsive member 18A at the 5' end of the transferred strand 49F and the 3' end of the non-transferred strand 44E instead of biotin or another hydrogel linking functional group. Thus, the transposome complexes 38E and 38F can be selectively attached to a predetermined region 76A, 76B, 76C of the flow cell surface that is exposed to visible light when the transposome complexes 38E and 38F are introduced.

As shown in Fig. 9A, the tagmentation entity 74 is introduced into the flow channel 20. The tagmentation entity 74 may be introduced in a suitable liquid (water, buffer etc.) through the inlet. In one example, a pump of a sequencing instrument, in which the flow cell 10 is operatively positioned, may be used to transport the tagmentation entity 74 into the flow channel 20.

In the example shown in Fig. 9A, when the tagmentation entity 74 is introduced into the flow channel 20, the specific region 76A of the flow cell 10 is exposed to visible light. Any suitable visible light source 78 may be used to illuminate the desired region 76A, 76B, 76C of the flow cell 10. The visible light source 78 may be a laser, a light emitting diode, an incandescent light bulb, or the like. The visible light source 78 may be part of the sequencing instrument or a separated automated or manually operated light source. The wavelength of light emitted is within the visible range, i.e., about 400 nm to about 700 nm. The exposed visible light responsive members 18A, 18B are activated and able to undergo a coupling reaction to attach the tagmentation entity 74 to the depressions 26 or the portion of the lane 36. In Fig. 9A, the transposome complexes 38E, 38F are asymmetrically attached (via their 3' and 5' ends, respectively).

The light responsive members 18A, 18B are not active unless they are exposed to visible light, and thus the light responsive members 18A, 18B present in any regions 76B, 76C of the flow cell 10 that are not exposed to visible light will not attach the tagmentation entity 74. In Fig. 9A, the light visible light source 78 is not used at regions 76B, 76C, and thus the light responsive members 18A, 18B in those regions 76B, 76C are not activated. Thus, these regions 76B, 76C can be used for a subsequently introduced sample.

After coupling of the tagmentation entity 74 in the desired region 76A, the remaining unbound tagmentation entities 74 are removed from the flow channel 20 using an example of the washing solution set forth herein.

In the example shown in Fig. 9A through Fig. 9D, the attached tagmentation entity 74 (e.g., transposome complexes 38E, 38F) may undergo tagmentation (Fig. 9B) and transposase 46 inactivation (Fig. 9C) before any additional tagmentation entities 74 are introduced (Fig. 9D).

In Fig. 9B, a first DNA sample 54 is introduced into the flow channel 20. While the DNA sample 54 is present in the flow channel 20, the temperature is raised to initiate tagmentation. Because the transposome complexes 38E, 38F are only present in the region 76A, tagmentation will take place only in the region 76A. Tagmentation may be performed as described herein.

In Fig. 9C, transposase 46 inactivation is performed. Transposase 46 inactivation involves removing the transposase 46 from each transposome complex 38E, 38F using any of the methods described herein (e.g., using sodium dodecyl sulfate (SDS) or proteinase or another chaotropic agent, or by heating the flow cell to about 60°C). This renders the transposome complexes 38E, 38F (or other complex that may be used as the tagmentation entity 74) inactive for participation with subsequently introduced DNA samples 54.

At each area/region 76A, 76B, 76C, tagmentation and transposase 46 inactivation may be performed before performing a suitable reaction for generating fully adapted DNA sample fragments and ultimately single stranded, fully adapted DNA sample fragments 56 (not shown in Fig. 9A through Fig. 9D). Fig. 9D illustrates the introduction of another batch of tagmentation entities 74 (e.g., transposome complexes 38E, 38F). Because the tagmentation entities 74 are introduced sequentially and are attached only in the light activated regions 76A, 76B, or 76C, the tagmentation entities 74 (in this example, any of the complexes 38 described herein) in a particular batch may include an index sequence 51 in the transferred strand 42 and/or in an adapter 50 introduced to the flow cell. This enables standard indexing in addition to the spatial indexing.

Once all of the desired regions 76A, 76B, 76C have the desired tagmentation entity 74 attached, the desired DNA sample 54 tagmented, and the transposase enzymes 46 removed, the fully adapted DNA sample fragments may be generated using gap fill ligation or an extension reaction depending upon the transposome complexes 38A, or 38B, or 38C and 38D, or 38E and 38F. These processes may be respectively performed as described herein.

As noted above, each depression 26 may be configured with primers 55, 57 for amplification and cluster generation of the fully adapted DNA sample fragments 56. These processes may be performed as described herein.

If it is desirable, cleavage of the forward and/or reverse fully adapted DNA sample fragments may take place, followed by denaturation. If desirable for a particular workflow, the cleaved and single stranded fully adapted DNA sample fragments 56 may be transported to another area, e.g., on the flow cell 10 off of the flow cell 10, for enrichment as described herein.

After amplification and cluster generation, sequencing may then be performed. In one example, sequencing by synthesis is performed by introducing a sequencing primer followed by an incorporation mix including labeled nucleotides. Optical imaging may be used to detect each instance of nucleotide incorporation.

The examples shown in Fig. 10A through Fig. 10C depict different methods in which the visible light activated surface may be used. The flow cell 10 is divided into different areas 80, 82, 84, etc., some of which are visible light activated. In each of these figures, the tagmentation entities 74 are some example of the transposome complex 38A or 38B or 38C and 38D or 38E and 38F disclosed herein.

In Fig. 10A, the different areas of the flow cell channel 20 include a tagmentation area 80, a denaturation area 82, and a clustering area 84. In this example, some or all of the depressions 26, or a portion or all of the lane 36 may be functionalized with the visible light responsive member 18B. Any area that is to exhibit coupling of a tagmentation entity (e.g., transposome complexes 38 or primers 55, 57) may include the visible light responsive member 18B. In the example shown in Fig. 10A, both the tagmentation area 80 and the clustering area 84 include the visible light responsive members 18B.

When transposome complexes 38A or 38B or 38C and 38D, or 38E and 38F (which include the visible light responsive member 18A) are to be attached in the area 80, a fluid containing the desired transposome complexes 38A, or 38B, or 38C and 38D, or 38E and 38F may be introduced into the flow cell 10 and the tagmentation area 80 may be exposed to visible light using any of the light sources 78 described herein (not shown in Fig. 10A). The visible light responsive members 18A, 18B that are exposed to the visible light will undergo a coupling reaction to attach the transposome complexes 38A, or 38B, or 38C and 38D, or 38E and 38F within the depressions 26 or the portion of the lane 36 in the tagmentation area 80. A DNA sample 54 (not shown in Fig. 10A) may then be introduced for tagmentation and any other processes as described herein to generate fully adapted DNA fragments (not shown in Fig. 10A).

When primers 55, 57 are to be attached in the area 84, a fluid containing the primers 55, 57 (having the visible light responsive member 18B at the 5' end) may be introduced into the flow cell 10 and the clustering area 84 may be exposed to visible light. The visible light responsive members 18A, 18B that are exposed to the visible light will undergo a coupling reaction to attach the primers 55, 57 within the depressions 26 or the portion of the lane 36 in the clustering area 84.

It is to be understood that transposome complex 38 attachment and primer 55, 57 attachment may take place in any order, as long as transposome complex 38 attachment takes place before DNA sample 54 introduction.

Once fully adapted fragments are generated in the tagmentation area 80 and once the primers 55, 57 are attached in the clustering area 84, a cleaving agent may be introduced into the flow channel 20 to cleave the fully adapted fragments. Flow may be directed toward the denaturation area 82, where the fully adapted fragments are denatured to form single stranded fully adapted fragments (e.g., 56) which then move to the clustering area 84 where they are seeded and amplified.

In Fig. 10B, the different areas of the flow cell channel 20 include a tagmentation area 80, a denaturation area 82, an enrichment area 86, and a clustering area 84.

When transposome complexes 38A or 38B or 38C and 38D, or 38E and 38F (which include the visible light responsive member 18A) are to be attached, a fluid containing the transposome complexes 38A or 38B or 38C and 38D, or 38E and 38F (which include the visible light responsive member 18A) may be introduced into the flow cell 10 and the tagmentation area 80 may be exposed to visible light using any of the light sources 78 described herein (not shown in Fig. 10B). The visible light responsive members 18A, 18B that are exposed to the visible light will undergo a coupling reaction to attach the transposome complexes 38A, or 38B, or 38C and 38D, or 38E and 38F within the depressions 26 or the portion of the lane 36 (not shown in Fig. 10B) in the tagmentation area 80. A DNA sample 54 (not shown in Fig. 10B) may then be introduced for tagmentation and any other processes as described herein to generate fully adapted DNA fragments (also not shown in Fig. 10B).

When probes 62' (which are similar to probes 62, except that the visible light responsive member 18B is positioned at the end instead of biotin) are to be attached, a fluid containing the probes 62' may be introduced into the flow cell 10 and the enrichment area 86 may be exposed to visible light. The visible light responsive members 18A, 18B that are exposed to the visible light will undergo a coupling reaction to attach the probes 62' within the depressions 26 or the portion of the lane 36 in the enrichment area 86.

Once fully adapted fragments are generated in the tagmentation area 80 and the probes 62' are attached in the enrichment area 86, but before the primers 55, 57 are attached in the clustering area 84, a cleaving agent may be introduced into the flow channel 20 to cleave the fully adapted fragments. Flow may be directed toward the denaturation area 82, where the fully adapted fragments are exposed to heat and denatured to form the single stranded fully adapted fragments (e.g., 56A, 56B, etc.) which then move to the enrichment area 86, where fragments including the target region are hybridized to the probes 62'. A wash solution may be introduced to flush out any fragments that do not include the target region and thus are not attached within the enrichment area 86.

While the fragments including the target region are hybridized to the probes 62', the primers 55, 57 may be introduced into the flow cell channel 20. A fluid containing the primers 55, 57 (having the visible light responsive member 18B attached at the 5' end) may be introduced into the flow cell 10 and the clustering area 84 may be exposed to visible light. The visible light responsive members 18A, 18B exposed to visible light from the light source 78 undergo a coupling reaction to attach the primers 55, 57 within the depressions 26 or the portion of the lane 36 in the clustering area 84.

Once the primers 55, 57 are attached, the fragments including the target region may be dehybridized from the probes 62' and transported to the clustering area 84 where they are seeded and amplified.

In Fig. 10C, the different areas of the flow cell channel 20 include a tagmentation area 80, a denaturation area 82, an amplicon hybridization and extension area 88, and a clustering area 84. In the example shown in Fig. 10C, the transposome complexes 38A or 38C and 38D or 38E and 38F may be used.

When transposome complexes 38A or 38C and 38D, or 38E and 38F (which include the visible light responsive member 18A) are to be attached, a fluid containing the transposome complexes 38A or 38C and 38D, or 38E and 38F (which include the visible light responsive member 18A) may be introduced into the flow cell 10 and the tagmentation area 80 may be exposed to visible light using any of the light sources 78 described herein (not shown in Fig. 10C). The visible light responsive members 18A, 18B that are exposed to the visible light will undergo a coupling reaction to attach the transposome complexes 38A or 38C and 38D, or 38E and 38F within the depressions 26 or the portion of the lane 36 in the tagmentation area 80. A DNA sample 54 may then be introduced for tagmentation and any other processes as described herein to generate partially adapted DNA fragments (e.g., 58, 58').

The amplicon hybridization and extension area 88 is an area where the partially adapted DNA fragments can be hybridized and extended to introduce suitable adapters for amplification. Specific sequences 90 are introduced to the surface of amplicon hybridization and extension area 88. These sequences 90 have a portion that can hybridize to the partially adapted fragment, a portion that is a complement of the adapter that is to be added, and have the light responsive member 18A at the 5 end. A fluid containing the sequences 90 may be introduced into the flow cell 10 and the amplicon hybridization and extension area 88 may be exposed to visible light. The visible light responsive members 18A, 18B undergo a coupling reaction to attach the sequences 90 within the depressions 26 or the portion of the lane 36 in the amplicon hybridization and extension area 88.

Once the partially adapted fragments are generated in the tagmentation area 80 and the sequences 90 are attached in the amplicon hybridization and extension area 88, but before the primers 55, 57 are attached in the clustering area 84, a cleaving agent may be introduced into the flow channel 20 to cleave the partially adapted fragments. Flow may be directed toward the denaturation area 82, where the partially adapted fragments are heated and denatured to form single strands, which then move to the amplicon hybridization and extension area 88, where the single stranded partially adapted fragments are hybridized to the sequences 90. An extension mix may be introduced to add the adapters and extend the partially adapted fragments into fully adapted fragments.

While the fully adapted fragments are hybridized to the sequences 90, the primers 55, 57 may be introduced into the flow cell channel 20. A fluid containing the primers 55, 57 (having the visible light responsive member 18A at the 5' end) may be introduced into the flow cell 10 and the clustering area 84 may be exposed to visible light. The exposed visible light responsive members 18A, 18B undergo a coupling reaction to attach the primers 55, 57 within the depressions 26 or the portion of the lane 36 in the clustering area 84.

Once the primers 55, 57 are attached, the fully adapted fragments may be dehybridized from the sequences 90 and transported to the clustering region 84 where they are seeded and amplified.

The examples shown in Fig. 9A through Fig. 9C enable at least spatial separation of DNA samples (thus using the surface as an indexing strategy), and the examples shown in Fig. 10A through Fig. 10C depict a sequence of workflow steps that can be performed along a single flow channel 20 (e.g., tagmentation in one region/area, enrichment in another region/area, etc.).

### Activating Flow Cell Surfaces with a Protective Coating

Fig. 11A through Fig. 11D depict the selective activation of flow cell surface chemistry to enable spatial indexing. The example shown in Fig. 11A through Fig. 11D utilizes a removable coating 92 for selective exposure of surface bound transposome complexes 38A, or 38B, or 38C and 38D, or 38E and 38F. In this example, any of the transposome complexes 38A, or 38B, or 38C and 38D, or 38E and 38F may be used, and the transposome complexes 38A, or 38B, or 38C and 38D, or 38E and 38F may include biotin or another suitable linking functional group that attaches to the polymeric hydrogel 30 or 32 present in the depressions 26. For ease of illustration, the transposome complexes 38E, 38F are shown in Fig. 11A through Fig. 11D.

The flow cell 10 is similar to that shown in Fig. 2A (although the base support 22 is not shown) and includes the transposome complexes 38E, 38F attached to the polymeric hydrogel 30, 32 in the depressions 26. In this example, the flow cell 10 has the same surface chemistry in each of the depressions 26. Alternatively, the flow cell 10 shown in Fig. 2B may be used, and the transposome complexes 38E, 38F may be positioned along the lane 36. While not shown, each flow cell depression 26 also has the primers 55, 57 attached to the polymeric hydrogel 30, 32.

The removable coating 92 overlies the depressions 26 (and any surface chemistry therein) and the interstitial regions 34. The removable coating 92 may be any material that melts, collapses, softens, solubilizes, or permeabilizes upon exposure to a predetermined stimulus, such as light, heat, or a pH change.

Removable coatings 92 that are responsive to light are made of photocleavable materials.

In some examples, the light removable coating is a hydrophilic polymer cross-linked with a photo-cleavable cross-linker, an acid-labile cross-linker, and/or a heat-labile crosslinker. In one specific example, the hydrophilic polymer is a polyvinyl alcohol/polyethylene glycol graft copolymer, and the photo-cleavable cross-linker is a coumarin or o-nitrobenzyl moiety. The coumarin or o-nitrobenzyl moieties may covalently conjugate with poly(ethylene glycol) of the polyvinyl alcohol/polyethylene glycol graft copolymer. The coumarin or o-nitrobenzyl is cleavable upon exposure to ultraviolet (UV) (100 nm to 400 nm) or blue light (450 nm to 495 nm), which leads to decreased cross-linking density of the coating. Thus, upon light exposure, the cleaved polymer coating becomes hydrophilic and washable by an aqueous solution. In another example, the hydrophilic polymer is a polyvinyl alcohol/polyethylene glycol graft copolymer, and the photo-cleavable cross-linker is an acid-labile cross-linker. In this example, the acid-labile cross-linker is an acetal moiety. With the acid-labile cross-linker, an acid species can be generated in the presence of a photoacid generator (PAG) and upon exposure to UV light, which changes the pH and cleaves the cross-linker, thus rendering the polymeric coating more soluble (and easily removable via rinsing).

In other examples, the light removable coating is a hydrophilic polymer capped with a photo-cleavable hydrophobic group or an acid-labile group. The photo-cleavable hydrophobic groups or acid-labile groups are protective groups on the polymer side chains that render the polymer more hydrophobic. These examples undergo a phase transition from hydrophobic to hydrophilic upon exposure to light. In an example, the hydrophilic polymer is polymethacrylic acid, polyphenol, polyvinyl alcohol, or polyvinyl alcohol/polyethylene glycol graft copolymer, which is functionalized with the photo-cleavable hydrophobic group, such as a coumarin or o-nitrobenzyl moiety. These photo-cleavable groups are generally hydrophobic, which prevents the coating 92 from being removed and washed away by an aqueous solution. Upon exposure to UV or visible light, these hydrophobic groups will be cleaved, returning the removable coating 92 back to its hydrophilic form. In another example, the hydrophilic polymer is polymethacrylic acid, polyphenol, polyvinyl alcohol, or polyvinyl alcohol/polyethylene glycol graft copolymer, which is functionalized with an acid-labile group. An acid species can be generated in the presence of a photoacid generator (PAG) and upon exposure to UV light, which cleaves the acid-labile group. In one example, the cleavage of tert-butyl carbonate groups leads to the hydrophilic polyphenol coating, which is washable and removable by an aqueous solution.

In still other examples, the light removable coating is a thermo-responsive polymer and a photo-thermal additive (or photo-thermal filler). The thermo-responsive polymer may be selected from the group consisting of polylactic acid, poly(lactic-co-glycolic) acid, polycaprolactone, agarose, wax, poly(acrylamide-co-acrylonitrile), poly(N-isopropylacrylamide), cyclodextrins, polyethylene glycol homopolymer, polyethylene glycol graft copolymer, polyethylene block copolymer, and a combination thereof. The photo-thermal additive or photo-thermal filler included in the composite may be nano-sized or any micro-sized structures that absorb light (e.g., photonic) energy at a certain wavelength range and convert the absorbed energy to heat. The photo-thermal filler (or photo-thermal additive) may be selected from the group consisting of gold nanoparticles, silver nanoparticles, iron oxide nanoparticles, polypyrrole particles, graphene sheets, carbon nanotubes, carbon nanodots, black phosphorus particles, azobenzene particles, and combinations thereof. Upon exposure to UV or visible light, energy absorbed by the photo-thermal additive(s) is converted to heat, which induces a phase transition in the polymer matrix (due to the presence of the thermo-responsive polymer). This phase transition causes this light removable coating to soften or dissolve. The softened or dissolved coating can then be removed using an aqueous solvent.

Still other light removable coatings are light and pH responsive. With these coatings, exposure to light induces proton release. Examples of these light removable coatings include 2-Naphthol-6,8-disulfonic acid or 1,2-Naphthoquinone-2-diazide-5-sulfonic acid.

Heat removable coatings are made of thermo-responsive materials. As an example, the thermo-responsive material may be a polymer, a wax, or a myristic acid that melts upon reaching a certain temperature. As another example, the thermo-responsive material may be a polymer that transitions from a hydrophobic state to a hydrophilic state upon reaching a certain temperature, e.g., UCST (upper critical solution temperature) polymers, such as poly(acrylamide-co-acrylonitrile). As yet another example, the thermo-responsive material may be a polymer that transitions form a hydrophilic state to a hydrophobic state upon reaching a certain temperature, e.g., LCST (lower critical solution temperature) polymers, such as poly(N-isopropylacrylamide) or PNIPAAm, which has a transition temperature of about 32°C-44°C. In a specific example, the heat removable coating may include a polymer that is selected from the group consisting of polylactic acid, poly(lactic-co-glycolic) acid, polycaprolactone, agarose, wax, poly(acrylamide-co-acrylonitrile), poly(N-isopropylacrylamide), cyclodextrins, polyethylene glycol homopolymer, polyethylene glycol graft copolymer, polyethylene block copolymer, and a combination thereof. Another suitable thermo-responsive material is made up of a hydrophilic polymer cross-linked with the heat-labile cross-linker. An example of the heat-labile cross-linker is disuccinimidyl adipate (DSA). DSA is a homobifunctional cross-linker with two NHS ester groups. The NHS esters react with primary amines to form stable amide bonds. DSA reacts with primary amines, creating a covalent linkage through amide bond formation. DSA contains a linker that is susceptible to hydrolysis under elevated temperatures. As still another example, the thermo-responsive material may be a gel, such as hydroxypropyl methylcellulose, which has a viscosity linked to temperature.

pH change removable coatings are made of, or include, pH responsive materials. Alternatively, the pH responsive material may be adjacent to the removable coating. Examples of polymers that are soluble in aqueous solutions having different pH levels include chitosan, which is soluble under acidic conditions, and copolymers of non-water soluble monomers with amine functional monomers, which are tunable to dissolve at different pHs depending on the content of the amino functional monomer. One example of the copolymer is dimethylaminoethyl methacrylate, which is soluble under basic conditions. Other examples of pH responsive polymers are those that are commercially available under the tradename EUDRAGIT^{®} from Evonik. As described herein, light and/or heat may be used to generate a local pH change.

The method shown in Fig. 11A through Fig. 11D involves introducing a DNA sample 54 to a flow cell 10 (Fig. 11A); exposing a sub-set of the transposome complexes 38A, or 38B, or 38C and 38D, or 38E and 38F at a predetermined area 76A, 76B, 76C of the flow cell 10 by removing a portion of the removable coating 92 from the predetermined area 76A, 76B, 76C (Fig. 11B); and initiating tagmentation of at least a portion of the DNA sample 54 at the predetermined area 76A, 76B, 76C (Fig. 11B).

In Fig. 11A, a first DNA sample 54 is introduced into the flow channel 20. The first DNA sample 54 may be introduced in a suitable liquid (water, buffer etc.) that is introduced through an opening (inlet, outlet) so that it flows in a desirable direction. In one example, a pump of a sequencing instrument, in which the flow cell 10 is operatively positioned, may be used to transport the first DNA sample 54 into the flow channel 20. Because the removable coating 92 overlies the transposome complexes 38E, 38F, the DNA sample 54 cannot bind anywhere.

While the DNA sample 54 is present in the flow channel 20, the predetermined area 76A of the flow cell 10 is exposed to the stimulus that melts, solubilizes, or permeabilizes the removable coating 92. This is depicted in Fig. 11B. As described herein, the removable coating 92 may be susceptible to light, heat, or a pH change.

When light is the stimulus, any suitable visible light source 78 may be used to illuminate the desired area/region 76A, 76B, 76C of the flow cell 10. While the light source 78 is shown in Fig. 11B and Fig. 11D, it is to be understood that this may be replaced with a heating mechanism.

When heat is the stimulus, any suitable heating mechanism may be used. The heating mechanism may be included in a complementary metal oxide semiconductor chip that coupled to the flow cell substrate 12, 14, included as part of the lid of the flow cell 10, or included/embedded in the flow cell substrate 12, 14, or deposited within the depressions 26. Examples of the heating mechanism may include one or more electrode materials that are capable of converting electricity to a suitable amount of thermal energy to heat up the desired portion of the removable coating 92. In other examples, an external heating mechanism may be used, such as a heater of a sequencing instrument in which the flow cell 10 is positioned.

In some instances when heat is the removal mechanism, the other area(s) 76B, 76C of the flow channel 20 may be actively cooled while the area 76A is heated. Localized cooling may be performed with an active cooling system of an external heater (e.g., a Peltier). It is to be understood that while localized cooling may be used, it may not be used when the heating is localized to the desired area 76A. In other words, the heating mechanism can be used to locally heat an area where the coating 92 is to be removed, and the other area(s) may remain unheated.

When a pH change is the stimulus, the pH change may be initiated by altering the temperature at the predetermined area 76A, 76B, 76C. In some examples, pH levels decrease with an increase in temperature, and thus the heating mechanism may be used to induce a localized change in pH. In an example, a buffer that decreases pH with an increase in temperature, such as an acetate buffer, a citrate buffer, or a phosphate buffer, may be added to the flow cell 10 prior to localized heating. In another example, a buffer that increases pH with an increase in temperature, such as a borate buffer, may be added to the flow cell 10 prior to localized heating. In still another example, a buffer that can increase or decrease pH with an increase in temperature, such as a glycine buffer, may be added to the flow cell 10 prior to localized heating. In other examples, an acid (or another source of pH-lowering H+ ions) may be generated in the vicinity of an electrode that is adjacent to the predetermined area 76A (e.g., in the lid, substrate 12, 14, etc.), and the acid or source of pH-lowering H+ ions lowers the pH. In still other examples, an acid (or another source of pH-lowering H+ ions) may be generated when the predetermined area 76A is exposed to light. The light exposure induces a photocatalytic reaction, and thus proton release in the desired area for coating removal. Thus, the pH change is coupled with a temperature change or light exposure, which enables the spatial control.

Exposure of a portion of the removable coating 92 to the stimulus melts, solubilizes, or permeabilizes the portion of the removable coating 92 located at the predetermined area 76A. The removal of the portion of the removable coating 92 exposes the transposome complexes 38E, 38F at the area/region 76A. This allows the DNA sample 54 to bind at these transposome complexes 38E, 38F. Tagmentation may be then initiated in the area/region 76A. Tagmentation may be performed as described herein.

After tagmentation is performed in the desired region 76A, the flow channel 20 may be washed using an example of the washing solution set forth herein to remove the melted, solubilized, or permeabilized portion of the removable coating 92 and tagmentation reagents.

In Fig. 11C, transposase enzyme 46 removal is performed. The transposase 46 from each transposome complex 38E, 38F may be removed using any of the methods described herein (e.g., using sodium dodecyl sulfate (SDS) or proteinase or another chaotropic agent, or by heating the flow cell to about 60°C). This renders the transposome complexes 38E, 38F inactive for participation with subsequently introduced DNA samples, e.g., second DNA sample 54B in Fig. 11D.

The processes of DNA sample 54 addition, triggering the localized removal of the removable coating 92 in another predetermined area, e.g., 76B or 76C, tagmentation, and removal of the transposase enzyme 46 can then be repeated for as many DNA samples 54 and areas as desired and/or possible within a given flow channel 20. This will allow multiple DNA samples 54, 54B to be tagmented in the respective areas 76A, 76B, etc., which will persist through clustering. The addition of the second DNA sample 54B, the removal of the second portion of the removable coating 92 in predetermined area 76B, and the tagmentation of the second DNA sample 54B is shown in Fig. 11D.

The repeated processes are performed before performing a suitable reaction for generating fully adapted DNA sample fragments (not shown in Fig. 11A through Fig. 11D). Once all of the desired regions/areas 76A, 76B, 76C have the desired DNA sample 54 tagmented and the transposase enzymes 46 removed, the fully adapted DNA sample fragments may be generated using gap fill ligation or an extension reaction depending upon the transposome complexes 38A, or 38B, or 38C and 38D, or 38E and 38F that is used. These processes may be respectively performed as described herein.

As noted above, each depression 26 may be configured with primers 55, 57 for amplification and cluster generation. Amplification and cluster generation may be performed as described herein. Cleavage of either the forward or reverse fully adapted DNA sample fragments may then be performed. Denaturation will release the cleaved fragment, leaving the other (e.g., reverse or forward) single stranded fully adapted fragments (e.g., 56A, 56B, etc.) attached to the flow cell surface.

Sequencing may then be performed. In one example, sequencing by synthesis is performed by introducing a sequencing primer followed by an incorporation mix including labeled nucleotides. Optical imaging may be used to detect each instance of nucleotide incorporation.

### Activating Flow Cell Surfaces with Encapsulated Tagmentation Entity

Fig. 12A through Fig. 12D depict the selective release of a tagmentation entity 74 to enable spatial indexing. The example shown in Fig. 12A through Fig. 12D utilizes an encapsulated complex 94 for selective exposure of surface bound transposome complexes 38A, or 38B, or 38C and 38D, or 38E and 38F to activation chemistry. In this example, any of the transposome complexes 38A, or 38B, or 38C and 38D, or 38E and 38F may be used, and the transposome complexes 38A, or 38B, or 38C and 38D, or 38E and 38F may include biotin or another suitable linking functional group that attaches to the polymeric hydrogel 30 or 32 present in the depressions 26. For ease of illustration, the transposome complexes 38E, 38F are shown in Fig. 12A through Fig. 12D.

The flow cell 10 is similar to that shown in Fig. 2A (although the base support 22 is not shown) and includes the transposome complexes 38E, 38F attached to the polymeric hydrogel 30, 32 in the depressions 26. In this example, the flow cell 10 has the same surface chemistry (e.g., transposome complexes 38E, 38F) in each of the depressions 26. Alternatively, the flow cell 10 shown in Fig. 2B may be used, and the transposome complexes 38E, 38F may be positioned along the lane 36. While not shown, each flow cell depression 26 also has the primers 55, 57 attached to the polymeric hydrogel 30, 32.

The encapsulated complex includes an example of the tagmentation entity 74 surrounded by a coating material 98. In this example, the tagmentation entity 74 is a catalytic metal ion 96, such as Mg²⁺, Mn²⁺, etc. that catalyzes tagmentation.

The coating material 98 is removable upon exposure to a release mechanism/stimulus, such as temperature, light, or pH. Any of the temperature, light, or pH responsive materials set forth herein for the removable coating 92 may be used for the coating material 98. While the light source 78 is shown in Fig. 12A and Fig. 12D, it is to be understood that this may be replaced with a heating mechanism.

Another example of a temperature responsive coating material is a temperature sensitive wax or fatty acid; and triggering release of the catalytic metal ion 96 involves heating the predetermined area 76A, 76B, 76C to above a melting temperature of the temperature sensitive wax. Some examples of suitable temperature sensitive waxes include myristic acid, palmitic acid, paraffin wax, spermaceti or soy wax, each of which melts at a temperature ranging from about 40°C to about 50°C. Other examples of suitable temperature sensitive waxes include stearic acid, beeswax, microcrystalline polyethylene wax, or carnauba wax, each of which melts at a temperature ranging from about 60°C to about 80°C. In these examples, the predetermined area 76A, 76B, 76C is heated to the desired temperature to melt the wax and release the catalytic metal ion 96. Still another example of a temperature responsive coating material is a temperature sensitive polymer; and triggering release of the catalytic metal ion 96 involves heating the flow cell 10 above a solubilization temperature of the temperature sensitive polymer. Examples of suitable temperature sensitive polymers include UCST polymers, i.e. poly(acrylamide-co-acrylonitrile), hydroxypropyl methylcellulose, agarose, or gelatin, each of which solubilizes at a temperature greater than 30°C. Other examples of suitable temperature sensitive polymers include LCST polymers, i.e., poly(N-isopropylacrylamide), methylcellulose, or poloxamer, each of which solubilizes at a temperature less than 30°C. In these examples, the predetermined area 76A, 76B, 76C is heated to the desired temperature to solubilize the polymer and release the catalytic metal ion 96.

Some specific examples of suitable pH sensitive polymers for the coating material include EUDRAGIT^{®} L100 (methacrylic acid copolymer, type B from Evonik, Inc.) or KOLLICOAT^{®} MAE-100 (methacrylic acid copolymer, type A from BASF Corp.), each of which is soluble at pH greater than 6. Other examples of suitable pH sensitive polymers include EUDRAGIT^{®} RL/RS 100 (methacrylic acid copolymers from Evonik, Inc.) or carboxymethyl cellulose, each of which is soluble at pH greater than 8. Still other examples of suitable pH sensitive polymers include EUDRAGIT^{®} E (amino dimethyl methacryate copolymer from Evonik, Inc.) or chitosan, each of which is soluble at pH less than 3. In these examples, the pH of the predetermined region 76A, 76B, 76C is adjusted to be lower than the pH at which the polymer is sensitive (e.g., if solubility is pH X or lower) or to be higher than the pH at which the polymer is sensitive (e.g., if solubility is pH X or higher), and the polymer solubilizes. Solubilization of the polymer releases the catalytic metal ion 96.

The encapsulated complex 94 may be formed by generating a dispersion of the coating material 98, spray coating the dispersion in a fluidized bed onto the catalytic metal ion 96, and allowing the complex to dry. Alternative forms of drying could be used, such as freeze drying, vacuum drying, or microwave assisted drying. The dried product could subsequently be powderized into small matrix particles containing a mixture of the trigger material and the catalytic metal. Other suitable coating techniques, such a pan coating, spray drying, etc., may be used that effectively form a film of the coating material 98 over the catalytic metal ion 96.

The method shown in Fig. 12A through Fig. 12D involves introducing a DNA sample 54 to a flow cell 10 whereby the DNA sample 54 binds to the transposome complexes 38A, or 38B, or 38C and 38D, or 38E and 38F across the flow cell 10; introducing an encapsulated complex 94 into the flow cell 10, the encapsulated complex 94 including a catalytic metal ion 96 surrounded by a coating material 98; and selectively releasing the catalytic metal ion 96 from the encapsulated complex 94 at a predetermined area 76A of the flow cell 10 by exposing the encapsulated complex to a mechanism that initiates removal of the coating material 98, thereby initiating tagmentation of a portion of the DNA sample 54 in the predetermined area 76A while leaving another portion of the DNA sample 54 untagmented in another area 76B, 76C.

In Fig. 12A, a first DNA sample 54 is introduced into the flow channel 20. The first DNA sample 54 may be introduced in a suitable liquid (water, buffer etc.) through the inlet. In one example, a pump of a sequencing instrument, in which the flow cell 10 is operatively positioned, may be used to transport the first DNA sample 54 into the flow channel 20. The DNA sample 54 can bind to the transposome complexes 38E, 38F in each of the predetermined areas 76A, 76B, 76C, but tagmentation does not occur in each of the predetermined areas 76A, 76B, 76C.

While the DNA sample 54 is present in the flow channel 20, the predetermined area 76A of the flow cell 10 is exposed to the stimulus that melts, solubilizes, or permeabilizes the coating material 98. The removal of the coating material 98 from the encapsulated complex 94 releases the catalytic material 96 in the predetermined area 76A. This is depicted in Fig. 12A. As described herein, the coating material 98 may be susceptible to light, heat, or a pH change. These stimuli may be applied as described herein for the removable coating 92. When the coating material 98 is removed, the release of the catalytic metal ion 96 is localized so that it catalyzes tagmentation in the predetermined area 76A, but not in the other areas 76B, 76C. Tagmentation may be performed as described herein. When heat is the trigger, the other areas 76B, 76C may be cooled in order to keep the catalytic metal ion 96 from being released prematurely.

After tagmentation is performed in the desired region 76A, the flow channel 20 may be washed using an example of the washing solution set forth herein to remove the melted, solubilized, or permeabilized portion of the coating material 98 and tagmentation reagents.

In Fig. 12B, transposase enzyme 46 removal is performed. The transposase enzyme 46 from each transposome complex 38E, 38F may be removed using any of the methods described herein (e.g., using sodium dodecyl sulfate (SDS) or proteinase or another chaotropic agent, or by heating the flow cell to about 60°C). This process releases bound but untagmented DNA sample 54 from the inactive transposome complexes 38E, 38F in areas 76B, 76C. The tagmented fragments 58, 58' remain attached in the area 76A, as they have been ligated to respective 3' ends of the transferred strands 42E, 42F. The released transposase enzymes 46 and untagmented DNA sample 54 may be removed from the flow cell 10.

This example method then involves reintroducing transposase enzymes 46. This is shown in Fig. 12C. The transposase enzymes 46 bind to the transposon ends 40E, 40F to reform the transposome complexes 38E, 38F. This renders the transposome complexes 38E, 38F in the areas 76B, 76C ready for participation with subsequently introduced DNA samples, e.g., second DNA sample 54B in Fig. 12D.

The processes of DNA sample 54 addition, triggering the localized release of the catalytic metal 96 in another predetermined area, e.g., 76B or 76C, tagmentation, removal of the transposase enzyme 46, the catalytic metal ion 96, and the bound but untagmented DNA sample 54, and reintroduction of transposase enzymes 46 can then be repeated for as many DNA samples 54 and areas as desired and/or possible within a given flow channel 20. This will allow multiple DNA samples 54, 54B to be tagmented in the respective areas 76A, 76B, etc., which will persist through clustering. The addition of the second DNA sample 54B, the localized release of the catalytic metal 96 in the predetermined area 76B, and the tagmentation of the second DNA sample 54B is shown in Fig. 12D.

The repeated processes are performed before performing a suitable reaction for generating fully adapted DNA sample fragments (not shown in Fig. 12A through Fig. 12D). Once all of the desired regions/areas 76A, 76B, 76C have the desired DNA sample 54 tagmented and the transposase enzymes 46 removed one final time, the fully adapted DNA sample fragments may be generated using gap fill ligation or an extension reaction depending upon the transposome complexes 38A, or 38B, or 38C and 38D, or 38E and 38F that is used. These processes may be respectively performed as described herein.

As noted above, each depression 26 may be configured with primers 55, 57 for amplification and cluster generation. Amplification and cluster generation may be performed as described herein. Cleavage of either the forward or reverse fully adapted DNA sample fragments may then be performed. Denaturation will release the cleaved fragment, leaving the other (e.g., reverse or forward) single stranded fully adapted fragments (e.g., 56A, 56B, etc.) attached to the flow cell surface.

Sequencing may then be performed. In one example, sequencing by synthesis is performed by introducing a sequencing primer followed by an incorporation mix including labeled nucleotides. Optical imaging may be used to detect each instance of nucleotide incorporation.

### Heat Activating Flow Cell Surfaces

Fig. 13A through Fig. 13D depict the selective activation of the surface bound tagmentation entity 74 to enable spatial indexing. The example shown in Fig. 13A through Fig. 13D utilizes heat to selectively activate surface bound transposome complexes 38A, or 38B, or 38C and 38D, or 38E and 38F. In this example, any of the transposome complexes 38A, or 38B, or 38C and 38D, or 38E and 38F may be used, and the transposome complexes 38A, or 38B, or 38C and 38D, or 38E and 38F may include biotin or another suitable linking functional group that attaches to the polymeric hydrogel 30 or 32 present in the depressions 26. For ease of illustration, the transposome complexes 38E, 38F are shown in Fig. 13A through Fig. 13D.

The flow cell 10 is similar to that shown in Fig. 2A (although the base support 22 is not shown) and includes the transposome complexes 38E, 38F attached to the polymeric hydrogel 30, 32 in the depressions 26. In this example, the flow cell 10 has the same surface chemistry (e.g., transposome complexes 38E, 38F) in each of the depressions 26. Alternatively, the flow cell 10 shown in Fig. 2B may be used, and the transposome complexes 38E, 38F may be positioned along the lane 36. While not shown, each flow cell depression 26 also has the primers 55, 57 attached to the polymeric hydrogel 30, 32.

The method shown in Fig. 13A through Fig. 13D involves selectively activating a sub-set of transposome complexes 38E, 38F in a predetermined area 76A, 76B, 76C of a flow cell 10 by heating the predetermined area 76A to an activation temperature and cooling another area 76B, 76C of the flow cell below the activation temperature; and introducing a DNA sample 54 to the flow cell 10, whereby a portion of the DNA sample 54 binds to and is tagmented by the sub-set of transposome complexes 38E, 38F in the predetermined area 76A and another portion of the DNA sample 54 binds to, but remains untagmented by, the transposome complexes 38E, 38F in the other area 76B, 76C.

In Fig. 13A, the area 76A of the flow cell 10 where tagmentation is to be initiated is exposed to an activation temperature of the transposome complexes 38E, 38F. In an example, the activation temperature ranges from about 20°C to about 55°C. Localized heating may be accomplished using any of the heating mechanisms described herein.

While localized heating is performed, localized cooling may also be performed in the other areas 76B, 76C where tagmentation is not to be initiated. In an example, the cooling temperature is at or below 18°C. Localized cooling may be accomplished using any of the cooling mechanisms described herein. Cooling to the lower temperature helps to inhibit the transposome complex 38E, 38F activation and prevent tagmentation in the areas 76B, 76C where tagmentation is undesirable.

Once the desired area 76A is heated and the other area(s) 76B, 76C is/are cooled (if desired), the first DNA sample 54 is introduced into the flow channel 20. The first DNA sample 54 may be introduced in a suitable liquid (water, buffer etc.) through the inlet. In one example, a pump of a sequencing instrument, in which the flow cell 10 is operatively positioned, may be used to transport the first DNA sample 54 into the flow channel 20. The DNA sample 54 can bind to the transposome complexes 38E, 38F in each of the predetermined areas 76A, 76B, 76C, but tagmentation only occurs in the heated area, e.g., area 76A in Fig. 13A, where the transposome complexes 38E, 38F have been activated. Tagmentation may be performed as described herein.

After tagmentation is performed in the desired region 76A, the flow channel 20 may be washed using an example of the washing solution set forth herein to remove tagmentation reagents.

The method shown in Fig. 13A through 13D then further includes removing a transposase enzyme 46 of each of the transposome complexes 38E, 38F, thereby releasing the other portion of the DNA sample 54 and whereby the portion of the DNA sample (e.g., tagmented fragments 58, 58') remains attached in the predetermined area 76A; washing the removed transposase enzymes 46 and the released DNA sample 54 from the flow cell 10; and introducing fresh transposase enzymes 46 to the flow cell 10, thereby reforming transposome complexes 38E, 38F in the other area 76B or 76C.

In Fig. 13B, transposase removal is performed. The transposase enzyme 46 from each transposome complex 38E, 38F may be removed using any of the methods described herein (e.g., using sodium dodecyl sulfate (SDS) or proteinase or another chaotropic agent, or by heating the flow cell to about 60°C). This process releases bound but untagmented DNA sample 54 from the inactive transposome complexes 38E, 38F in the cooled areas 76B, 76C. The tagmented fragments 58, 58' remain attached in the heated area 76A, as they have been ligated to respective 3' ends of the transferred strands 42E, 42F. The released transposase enzymes 46 and untagmented DNA sample 54 may be removed from the flow cell 10 using the wash solution.

As mentioned, this example method then involves reintroducing transposase enzymes 46. This is shown in Fig. 13C. The transposase enzymes 46 bind to the transposon ends 40E, 40F to reform the transposome complexes 38E, 38F. This renders the transposome complexes 38E, 38F in the areas 76B, 76C ready for participation with subsequently introduced DNA samples, e.g., second DNA sample 54B in Fig. 13D.

The processes of localized heating and cooling, DNA sample 54 addition, removal of the transposase enzyme 46 and bound but untagmented DNA sample 54, and reintroduction of transposase enzymes 46 can then be repeated for as many DNA samples 54 and areas as desired and/or possible within a given flow channel 20. This will allow multiple DNA samples 54, 54B to be tagmented in the respective areas 76A, 76B, etc., which will persist through clustering. The addition of the second DNA sample 54B and localized heating is shown in Fig. 13D.

The repeated processes are performed before performing a suitable reaction for generating fully adapted DNA sample fragments (not shown in Fig. 13A through Fig. 13D). Once all of the desired regions/areas 76A, 76B, 76C have the desired DNA sample 54 tagmented and the transposase enzymes 46 removed one final time, the fully adapted DNA sample fragments may be generated using gap fill ligation or an extension reaction depending upon the transposome complexes 38A, or 38B, or 38C and 38D, or 38E and 38F that is used. These processes may be respectively performed as described herein.

As noted above, each depression 26 may be configured with primers 55, 57 for amplification and cluster generation. Amplification and cluster generation may be performed as described herein. Cleavage of either the forward or reverse fully adapted DNA sample fragments may then be performed. Denaturation will release the cleaved fragment, leaving the other (e.g., reverse or forward) single stranded fully adapted fragments (e.g., 56A, 56B, etc.) attached to the flow cell surface.

Sequencing may then be performed. In one example, sequencing by synthesis is performed by introducing a sequencing primer followed by an incorporation mix including labeled nucleotides. Optical imaging may be used to detect each instance of nucleotide incorporation.

### Selective Capture of DNA Samples

The top view portion of Fig. 15A depicts different regions/areas 76A, 76B, 76C, 76D, 76E, 76F of a lane 36 of a flow cell 10. As shown in Fig. 15B, each region/area 76A, 76B, 76C, 76D, 76E, and 76F includes a different type of target primer 100A, 100B, 100C, 100D, 100E, 100F that enables at least spatial indexing.

The flow cell lane 36 is similar to that shown in Fig. 2A, and may be the only lane of a flow cell 10, or may be one of multiple lanes 36 of the flow cell 10. To briefly reiterate, the flow cell lane 36 is formed in the multi-layer substrate 12, and includes depressions 26 defined in the patterned layer 24. Each depression 26 includes the polymeric hydrogel 32 therein, and an amplification primer set (i.e., primers 55 and 57) attached to the polymeric hydrogel 30 or 32. Alternatively, the flow cell lane 36 shown in Fig. 2B may be used, and the primers 55 and 57 may be attached to the polymeric hydrogel 30 or 32 along the lane 36.

In these examples, the flow cell lane 36 is divided into at least two regions/areas 76A, 76B. The number of regions/areas 76A, 76B depends upon the number of different samples that are to be introduced to the flow cell lane 36. The number of regions/areas 76A, 76B is also limited by the dimensions of the lane 36.

The regions/areas 76A, 76B, 76C, 76D, 76E, 76F are defined by the particular type of target primer, see reference numerals 100A, 100B, 100C, 100D, 100E, 100F in Fig. 15B, that is attached to the polymeric hydrogel 30 or 32 in the region/area 76A, 76B, etc. As used herein, the reference numeral 100 collectively refers to all of the target primers, while the reference numerals 100A, 100B, 100C, 100D, 100E, 100F specifically refers to those in the corresponding region/area 76A, 76B, 76C, 76D, 76E, 76F. Similarly, the reference numeral 102 collectively refers to all of the spatial tags, while the reference numerals 102A, 102B, 102C, 102D, 102E, 102F specifically refers to the spatial tag that is complementary to the corresponding target primers 100A, 100B, 100C, 100D, 100E, 100F.

Each target primer 100 in a given region 76A, 76B, 76C, 76D, 76E, or 76F of the flow cell 10 has the same sequence as each other target primer 100 in that region 76A, 76B, 76C, 76D, 76E, or 76F, and this sequence is complementary to the sequence of a spatial tag 102 that is to be hybridized thereto. The target primers 100D in one region 76D are orthogonal to the target primers 100A, 100B, 100C, 100E, and 100F in each other region 76A, 76B, 76C, 76E, and 76F. By "orthogonal" when the term is used to describe the target primers 100, it is meant that the target primers 100D in one region 76D have a different oligonucleotide sequence than the target primers 100A, 100B, 100C, 100E, and 100F in each other region 76A, 76B, 76C, 76E, and 76F, and thus the target primers 102A, 102B, 102C, 102D, 102E, 102F in the respective regions 76A, 76B, 76C, 76D, 76E, 76F are capable of hybridizing only to respective complementary spatial tags 102A, 102B, 102C, 102D, 102E, 102F.

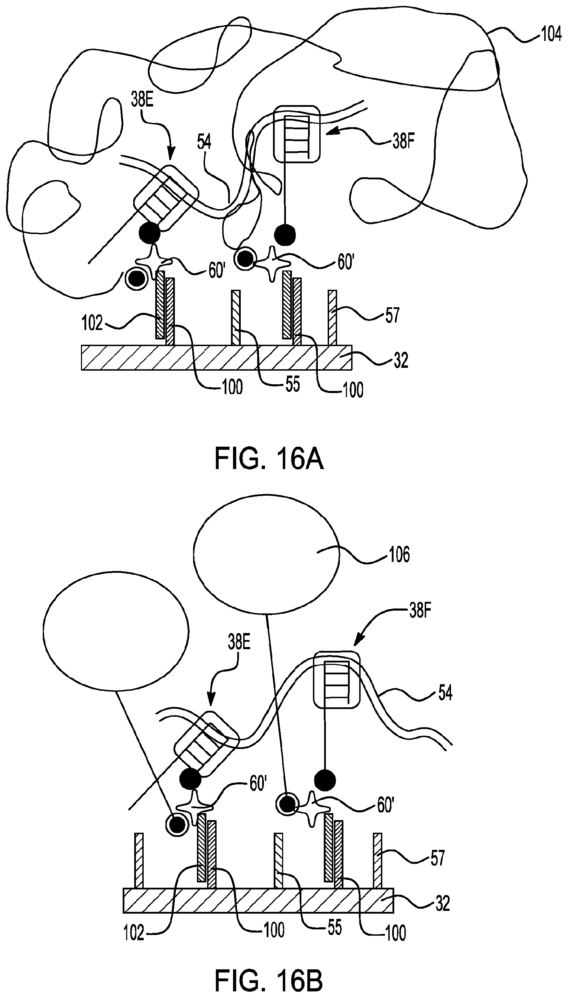

The 5' end of the target primers 100 includes a functional group that attaches it to the polymeric hydrogel 30 or 32. In the method that utilizes the passivation component (see 104 and 106 in Fig. 16A and Fig. 16B), it may be undesirable to use the biotinylated polymeric hydrogel 30, as the passivation component may include biotin as its attachment molecule. In other instances when the biotinylated polymer 30 is used, the 5' end of each of the target primers 100 is biotin, and avidin or streptavidin 60 may be used to attach the target primers 100A, 100B, etc. in the desired areas 76A, 76B, etc. When the polymeric hydrogel 32 is used, the 5' end of each of the target primers 100 may be any suitable functional group that can covalently attach to the R^{A} groups of the polymeric hydrogel 32.

The different groups of target primers 100A, 100B, etc. may be sequentially dispensed in the region/area 76A, 76B, etc. using a high precision coating method. In one example, high precision coating is achieved using a precision gantry tool. In other examples, the high precision coating method is performed using stripe coating or patch coating with a slot-die coating tool. The high precision coating method may further be used with other coating methods, such as spray coating or jetting, e.g., via inkjet. In an example, a first group of target primers 100A may be dispensed at the region 76A, and allowed to incubate so they graft to the polymeric hydrogel 30 or 32. When biotin-avidin/streptavidin-biotin linkages are used, the avidin/streptavidin 60 may be attached or introduced in accordance with any of the examples set forth herein.

The flow cell 10 including the lane 36 shown in Fig. 15A may be part of a kit. The kit includes at least two fluids, each of which is specifically designed for use with the target primers 100A, 100B, etc. in one of the regions 76A, 76B, etc. The number of fluids in the kit corresponds with the number of areas 76A, 76B, etc. that are defined in the lane 36. For example, a kit including the flow cell 10 with the lane 36 shown in Fig. 15 would include six different fluids, one for each of the areas 76A, 76B, 76C, 76D, 76E, 76F.

Each fluid in the kit includes a liquid carrier and a first plurality of transposome complexes 38A, or 38B, or 38C and 38D, or 38E and 38F dispersed in the liquid carrier, where each of the transposome complexes 38A, or 38B, or 38C and 38D, or 38E and 38F includes a spatial tag 102 that is complementary to the target primers 100 attached in one of the regions 76A, 76B, 76C, 76D, 76E, or 76F. For example, the fluid that is to be used with the region 76D includes transposome complexes 38E and 38F which includes spatial tags 102D whose sequences are complementary to the target primers 100D. As one specific example, the spatial tags 10D may be attached to each of the 3' end of the non-transferred strand (e.g., at 48E in Fig. 14D) and to the 5' end of the transferred strand (at 48F in Fig. 14D) so that these complexes 38E, 38F directly hybridize to the target primers 100D. In one example, the flow cell 10 has two regions 76A, 76B defined in the lane 36, and the kit includes a first fluid including a first liquid carrier; and a first plurality of transposome complexes 38A, or 38B, or 38C and 38D, or 38E and 38F dispersed in the first liquid carrier, each of the first plurality of transposome complexes 38A, or 38B, or 38C and 38D, or 38E and 38F including a first spatial tag 102A, that is complementary to the target primer 100A attached within the depressions 26 located at the first region 76A of the flow cell 10; and a second fluid including a second liquid carrier; and a second plurality of transposome complexes 38A, or 38B, or 38C and 38D, or 38E and 38F dispersed in the second liquid carrier, each of the second plurality of transposome complexes 38A, or 38B, or 38C and 38D, or 38E and 38F including a second spatial tag 102B, that is complementary to the target primer 100B attached within the depressions 26 located at the second region 76B of the flow cell 10.

The liquid carrier of the respective fluids may be water. A buffer and/or salt may be added to the carrier liquid. The buffer has a pH ranging from 5 to 12. Example of neutral buffers include Tris(hydroxymethyl) aminomethane (Tris or TRIS) buffers, such as Tris-HCI or Tris-EDTA, or a carbonate buffer (e.g., 0.25 M to 1 M). Sodium sulfate (e.g., 1 M to 2 M) is a suitable salt that may be used. The transposome complexes 38A, or 38B, or 38C and 38D, or 38E and 38F may be included in the carrier liquid in a concentration ranging from about 0.1 µM to about 1 µM.

As mentioned, any of the transposome complexes 38A, or 38B, or 38C and 38D, or 38E and 38F described herein may be used. In this example, each of the transposome complexes 38A, or 38B, or 38C and 38D, or 38E and 38F further includes one of the spatial tags 102A, 102B, 102C, 102D, 102E, or 102F. The spatial tag 102 is an oligonucleotide primer that is complementary to the target primer 100 contained within the region 76A, 76B, etc. of the flow cell 10 with which the fluid (containing the transposome complexes 38A, or 38B, or 38C and 38D, or 38E and 38F and spatial tags 102) is to be used. Thus, the spatial tag 102A, 102B, etc. used in each fluid will depend upon the corresponding region 76A, 76B, etc. and the target primers 100A, 100B, etc. in that region 76A, 76B, etc.

When the transposome complex 38A (Fig. 14A) or the transposome complex 38B (Fig. 14B) is used, the spatial tag 102 is covalently or non-covalently attached to the 5' end of the transferred strand 42. When the transposome complexes 38C and 38D are used (Fig. 14C), spatial tags 102 are respectively covalently or non-covalently attached to the 5' end of each of the transferred strands 42C and 42D of the complexes 38C and 38D. When the transposome complexes 38E and 38F are used (Fig. 14D), spatial tags 102 are attached to the 3' end of the non-transferred strand 44E of the complex 38E and to the 5' end of the transferred strand 42F of the complex 38F. In the example shown in Fig. 15B, the spatial tags 102A, 102B, 102C, 102D, 102E, 102F are attached to the transposome complexes 38E and 38F through biotin-avidin/streptavidin-biotin interaction, where the end groups 48E, 48F are both biotin, the 5' ends of the spatial tags 102 are biotin, and avidin/streptavidin 60' attached the biotins. Alternatively, the spatial tags 102A, 102B, 102C, 102D, 102E, 102F are attached to the transposome complexes 38E and 38F through biotin-avidin/streptavidin interaction, where the end groups 48E, 48F are both biotin, and avidin/streptavidin 60' is covalently attached to a thiol-modified oligonucleotide at the 5' end of the spatial tag 102.

An example method for making the lane 36 shown in Fig. 15A and Fig. 15B includes attaching an amplification primer set (e.g., primers 55, 57) along the flow cell lane 36 (e.g., to the polymeric hydrogel 30 or 32 in the lane 36 or in depressions 26); and sequentially attaching orthogonal target primers 100 (e.g., 100A, 100B, etc.) in at least two different regions 76A, 76B, etc. of the flow cell lane 36.

Two example methods for using the flow cell lane 36 of Fig. 15A and Fig. 15B will now be described. At the outset of each of the methods, the flow cell 10 with the regions 76A, 76B, etc. defined in the lane 36 is operatively positioned within a sequencing instrument.

One example method includes i) generating fully adapted first DNA sample fragments in the first region 76A of the flow cell 10 by introducing the first fluid (including the first plurality of transposome complexes 38A, or 38B, etc.) into the flow cell 10 at a hybridization temperature, whereby the first spatial tags 102A respectively hybridize to the target primers 100A attached within the depressions 26 located at the first region 76A of the flow cell 10, introducing a first DNA sample 54 into the flow cell 10, whereby the first DNA sample 54 is tagmented by the first plurality of transposome complexes 38A, or 38B, etc., removing a transposase enzyme 46 of each of the first plurality of transposome complexes 38A, or 38B, etc., and performing gap fill ligation or an extension reaction; and ii) generating fully adapted second DNA sample fragments in the second region 76B of the flow cell 10 by introducing the second fluid into the flow cell 10 at a hybridization temperature, whereby the second spatial tags 102B respectively hybridize to the target primers 100B attached within the depressions 26 located at the second region 76B of the flow cell 10, introducing a second DNA sample 54B into the flow cell 10, whereby the second DNA sample 54B is tagmented by the second plurality of transposome complexes 38A, or 38B, etc., removing a transposase enzyme 46 of each of the second plurality of transposome complexes 38A, or 38B, etc., and performing gap fill ligation or an extension reaction.

At the outset of this example method, the first fluid (including the first plurality of transposome complexes 38A, or 38B, etc. with the first spatial tag 102A) is introduced into the flow cell lane 36. The first fluid may be introduced through the inlet. In one example, a pump of the sequencing instrument may be used to transport the first fluid into the lane 36.

The temperature of the flow cell lane 36 is at or is brought to a hybridization temperature so that the first spatial tags 102A respectively hybridize to the complementary target primers 100A located at the first region 76A. Because the target primers 100B, 100C, 100D, 100E, and 100F located in other regions 76B, 76C, 76D, 76E, and 76F are not complementary to the first spatial tags 100A, the first plurality of transposome complexes 38A, or 38B, etc. will not bind in these regions 76B, 76C, 76D, 76E, and 76F.

The first DNA sample 54 is then introduced into the flow cell lane 36 as described herein (e.g., with a tagmentation buffer). Because the first plurality of transposome complexes 38A, or 38B, etc. is the only group of transposome complexes 38A, or 38B, etc. within the lane 36, the first DNA sample 54 will be tagmented by the first plurality of transposome complexes 38A, or 38B, etc. Tagmentation may be performed as described herein.

In this example method, the transposase enzyme 46 of each of the first plurality of transposome complexes 38A, or 38B, etc. is then removed. Transposase enzyme 46 removal may be performed using any of the methods described herein (e.g., using sodium dodecyl sulfate (SDS) or proteinase or another chaotropic agent, or by heating the flow cell to about 60°C). The released transposase enzymes 46 may be removed from the flow cell 10 using the wash solution.

The fully adapted DNA fragments 56 may be formed in the first region 76A using performing gap fill ligation or an extension reaction as described herein, and the process used will depend upon the transposome complex 38A, or 38B, or 38C and 38D, or 38E and 38F that used.

All of these processes can be performed with a second fluid (including the second plurality of transposome complexes 38A, or 38B, etc. with the second spatial tag 102B) and a second DNA sample 54B to generate fully adapted second DNA sample fragments in the second region 76B. The processes can then again be repeated for as many fluids, DNA samples, and areas/regions 76C, 76D, 76E, 76F as desired and/or possible within a given flow cell lane 36. This will allow multiple DNA samples 54, 54B to be tagmented in the respective areas 76A, 76B, etc., which will persist through clustering.

Because the transposome complexes 38A, or 38B, etc. are introduced sequentially and are attached only in the regions 76A, 76B, 76C, etc. at which they are capable of hybridizing, the transposome complexes 38A, or 38B, etc. in a particular fluid may include an index sequence in the transferred strand 42 and/or in an adapter 50 introduced to the flow cell 10. This enables standard indexing in addition to the spatial indexing.

Once all of the fully adapted DNA sample fragments 56 are generated, amplification and cluster generation may be performed as described herein. Cleavage of either the forward or reverse fully adapted DNA sample fragments may then be performed. Denaturation will release the cleaved fragment, leaving the other (e.g., reverse or forward) single stranded fully adapted fragments (e.g., 56A, 56B, etc.) attached to the flow cell surface.

Sequencing may then be performed. In one example, sequencing by synthesis is performed by introducing a sequencing primer followed by an incorporation mix including labeled nucleotides. Optical imaging may be used to detect each instance of nucleotide incorporation.

Another example method includes i) generating partially adapted first DNA sample fragments in the first region 76A of the flow cell 10 by introducing the first fluid (including the first plurality of transposome complexes 38A, or 38B, etc.) into the flow cell 10 at a hybridization temperature, whereby the first spatial tags 102A respectively hybridize to the target primers 100A attached within the depressions 26 located at the first region 76A of the flow cell 10, introducing a first DNA sample 54 into the flow cell 10, whereby the first DNA sample 54 is tagmented by the first plurality of transposome complexes 38A, or 38B, etc., removing a transposase enzyme 46 of each of the first plurality of transposome complexes 38A, or 38B, etc.; and ii) generating partially adapted second DNA sample fragments in the second region 76B of the flow cell 10 by introducing the second fluid into the flow cell 10 at a hybridization temperature, whereby the second spatial tags 102B respectively hybridize to the target primers 100B attached within the depressions 26 located at the second region 76B of the flow cell 10, introducing a second DNA sample 54B into the flow cell 10, whereby the second DNA sample 54B is tagmented by the second plurality of transposome complexes 38A, or 38B, etc., and removing a transposase enzyme 46 of each of the second plurality of transposome complexes 38A, or 38B, etc. ; and performing gap fill ligation or an extension reaction on each of the partially adapted first and second DNA sample fragments to form fully adapted first and second DNA sample fragments.

Another example method involves solution based binding to generate a bound complex where the DNA is bound to the transposome complexes 38A, or 38B, etc. The bound complex is shown in Fig. 16A and Fig. 16B. The bound complex is introduced into the flow cell lane 36 where it will become attached in the desired region 76A, or 76B, etc. through hybrization of the spatial tags 102 and complementary target primers 100. The bound complex is then passivated before an additional bound complex (formed with a different DNA sample) is added.

Generally, this method includes exposing the first plurality of transposome complexes 38A, or 38B, etc. to a first DNA sample 54, whereby the first DNA sample 54 binds to at least some of the first plurality of transposome complexes 38A, or 38B, etc. to form a first bound complex; introducing the first bound complex to the flow cell 10, whereby the first spatial tags 102A respectively hybridize to the target primers 100A attached within the depressions 26 located at the first region 76A of the flow cell 10; passivating the first bound complex at the first region 76A of the flow cell 10; exposing the second plurality of transposome complexes 38A, or 38B, etc. to a second DNA sample 54B, whereby the second DNA sample 54B binds to at least some of the second plurality of transposome complexes 38A, or 38B, etc. to form a second bound complex; and introducing the second bound complex to the flow cell 10, whereby the second spatial tags 102B respectively hybridize to the complementary target primers 100B attached within the depressions 26 located at the second region 76B of the flow cell 10.

At the outset of this example method, the first fluid (including the first plurality of transposome complexes 38A, or 38B, etc. with the first spatial tag 102A) is mixed with the first DNA sample 54 outside of the flow cell 10. The tagmentation buffer is not included so that tagmentation does not take place prematurely. Thus, the first DNA sample 54 binds to the transposome complexes 38A, or 38B, etc., but is not tagmented. This forms a bound complex of the first DNA sample 54 bound to the first plurality of transposome complexes 38A, or 38B, etc.

The bound complex is then introduced into the flow cell lane 36 using any suitable technique.

The temperature of the flow cell lane 36 is at, or is brought to, a hybridization temperature so that the first spatial tags 102A respectively hybridize to the complementary target primers 100A located at the first region 76A. Because the target primers 100B, 100C, 100D, 100E, and 100F located in other regions 76B, 76C, 76D, 76E, and 76F are not complementary to the first spatial tags 100A, the bound complex will not bind in these regions 76B, 76C, 76D, 76E, and 76F.

The bound complex may then be passivated by introducing a passivation component (e.g., a hydrophobic polymer or an anti-fouling agent) to the flow cell lane 36. The end group(s) of the passivation component may include biotin that can attach to the avidin/streptavidin 60' of the transposome complexes 38A, or 38B, etc. containing the spatial tag 102. Alternatively, the end group(s) of the passivation component may include any functional group that is orthogonal, i.e., not responsive to, the dehybridization conditions used to detach the spatial tag 102. In still another example, the passivation component may have its own passivation tag (similar to spatial tag 102) that can attach to a passivation target primer located in a specific area of the flow cell surface. The passivation tags or target primers could have cleavage sites that allow them to be removed at a desirable time in the workflow. As an example, a hydrophobic polymer X has oligo A, while area 76A has complimentary oligo A'. After the transposome complexes 38E, 38F are hybridized via the target primers in area 76A, the hydrophobic polymer X is hybridized via AA' to passivate area 76A. When passivation needs to be removed, the oligo A or A' can be cleaved via a chemistry, such as vinyl dT or 8 oxoG, this is orthogonal to the cleavage site of the primers and/or transposome complexes.

Passivation may involve forming a hydrophobic polymer shell 104 over the first bound complex, as depicted in Fig. 16A. Passivation may alternatively involve attaching an anti-fouling agent 106 to the first bound complex, as depicted in Fig. 16B.

Examples of suitable hydrophobic polymers that can be used to form the shell 104 include polypropylene glycol, polycaprolactone, poly(lactic-co-glycolic acid) (PLGA), cellulose acetate, ethyl cellulose, poly alkyl (meth)acrylate, or polydimethylsiloxane (PDMS).

The anti-fouling agent 106 may be phosphate, phosphonate, a zwitterionic end group, an amphoteric polymer end group, carboxylic acid, poly(ethylene glycol), a perfluoro end group, hydroxyl, sulfonic acid, a positively chargeable end group, an alkoxy end group, or an anionic polymer end group. Examples of the zwitterionic end group are selected from the group consisting of phosphocholine, a sulfobetaine, and a carboxybetaine. One example of an amphoteric polymer end group is a polymer chain including both carboxylic acid and amine functional groups, such as a co-polymer of methacrylic acid and dimethylammonium ethyl methacrylate. Some examples of the anti-fouling agent 106 with the perfluorinated end group are selected from the group consisting of (heptadecafluoro-1,1,2,2-tetrahydrodecyl) trimethoxysilane, 3-(heptafluoroisopropoxy)propyltrimethoxysilane, nonafluorohexyltrimethoxysilane, [perfluoro(polypropyleneoxy)]methoxypropyltrimethoxysilane, 1,3-bis(trifluoropropyl)-1,1,3,3-tetramethyldisilazane, and (tridecafluoro-1,1,2,2-tetrahydrooctyl)dimethylchlorosilane. Examples of the positively chargeable end group include amine and ammonium. Examples of the anionic polymer end group include polyphosphates, polysulfonates, and polycarboxylates (e.g., poly(meth)acrylic acid).

The anti-fouling agent 106 may include a linker, such as ethylene or another short alkyl chain, poly(meth) acrylate, tetrazine, or an azide, that has the biotin or other end group for attachment to the bound complex (e.g., through the avidin/streptavidin 60').

The second bound complex may be prepared (off-board the flow cell 10) by mixing a second DNA sample 54B and the second fluid (including the second plurality of transposome complexes 38A, or 38B, etc. with the spatial tag 102B). In this example, the second fluid also does not include the tagmentation buffer. In the second fluid, the second DNA sample 54B binds to the transposome complexes 38A, or 38B, etc., but is not tagmented. This forms a bound complex of the second DNA sample 54B bound to the second plurality of transposome complexes 38A, or 38B, etc.

The second bound complex is then introduced into the flow cell lane 36 (which includes the passivated first bound complex) using any suitable technique. The temperature of the flow cell lane 36 is at, or is brought to, a hybridization temperature so that the second spatial tags 102B respectively hybridize to the complementary target primers 100B located at the second region 76B. Because the target primers 100A, 100C, 100D, 100E, and 100F located in other regions 76A, 76C, 76D, 76E, and 76F are not complementary to the second spatial tags 100B, the bound complex will not bind in these regions 76A, 76C, 76D, 76E, and 76F. Moreover, the bound complex at the region 76A is passivated, and thus any free DNA sample 54B (e.g., at the ends) will not bind to unused transposome complexes 38A, or 38B, etc. at the first region 76A. The passivation also prevents any free transposome complexes 38A, or 38B, etc. from the second solution from binding additionally to the first DNA sample 54A.

The second bound complex may then be passivated by introducing a passivation component (e.g., a hydrophobic polymer or an anti-fouling agent) to the flow cell lane 36.

The processes of bound complex formation, bound complex introduction and attachment within the desired flow cell region, and passivation of the newly introduced bound complex can then again be repeated for as many bound complexes and areas/regions 76C, 76D, 76E, 76F as desired and/or possible within a given flow cell lane 36. This will allow multiple bound complexes (each of which includes a different DNA) to be attached in the respective regions 76A, 76B, etc. Once all of the bound complexes are introduced and bound to the regions 76A, 76B, etc., the passivation component can be removed. When the biotin-avidin/streptavidin interaction is used, the passivation component can be released by introducing any example of the biotin streptavidin cleavage composition disclosed herein into the flow cell 10. Alternatively, if the passivation tags or target primers have cleavage sites, individual cleaving agents may be introduced to cleave and thus remove the passivation component from a particular area 76A, 76B, etc.

Tagmentation can be initiated either before or after the passivation component is removed. This may be accomplished by introducing any example of the tagmentation buffer into the flow cell lane 36 and ensuring that the temperature is at or above 30°C. Tagmentation may be performed as described herein.

In this example method, the transposase enzymes 46 of each of the transposome complexes 38A, or 38B, etc. is then removed. Transposase enzyme 46 removal may be performed using any of the methods described herein (e.g., using sodium dodecyl sulfate (SDS) or proteinase or another chaotropic agent. Heating may not be desirable so as to avoid dehybridization of the spatial tags 102 and target primers 100. The released transposase enzymes 46 may be removed from the flow cell 10 using the wash solution.

If not removed prior to tagmentation, it is to be understood that the passivation component should be removed prior to generating the fully adapted DNA fragments 56 and prior to breaking the bond between the spatial tag 102 and the transposome complexes 38E, 38F. The removal used will depend upon how the passivation component is attached (e.g., dehybridzation, cleavage of a cleavage site, etc.)

The fully adapted DNA fragments 56 may be formed in all of the regions 76A, 76B, 76C, 76D, 76E, 76F using performing gap fill ligation or an extension reaction as described herein, and the process used will depend upon the transposome complex 38A, or 38B, or 38C and 38D, or 38E and 38F that used.

Once all of the fully adapted DNA sample fragments 56 are generated, they are exposed to amplification and cluster generation using the primers 55, 57. Cleavage of either the forward or reverse fully adapted DNA sample fragments may then be performed, followed by denaturization. These processes may occur at the same time as amplification.

Sequencing may then be performed. In one example, sequencing by synthesis is performed by introducing a sequencing primer followed by an incorporation mix including labeled nucleotides. Optical imaging may be used to detect each instance of nucleotide incorporation.

Any of the examples set forth herein may be adjusted for simultaneous paired end sequencing. In these instances, the amplification domains and primers may be based on the primers described in WO 2020/005503, which is incorporated by reference herein in its entirety.

### Additional Notes

Each of Fig. 9A through Fig. 13D illustrates the flow in the direction from left to right across the flow cell. The various examples depict that the area being activated is downstream of inactive areas. It is to be understood, however, that the area being activated could be upstream of inactive areas or downstream of one or more inactive areas and upstream of one or more other inactive areas.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

Reference throughout the specification to "one example", "another example", "an example", and so forth, means that a particular element (e.g., feature, structure, and/or characteristic) described in connection with the example is included in at least one example described herein, and may or may not be present in other examples. In addition, it is to be understood that the described elements for any example may be combined in any suitable manner in the various examples unless the context clearly dictates otherwise.

While several examples have been described in detail, it is to be understood that the disclosed examples may be modified. Therefore, the foregoing description is to be considered non-limiting.
Aspects of the invention will now be described with reference to the following numbered clauses:
1. A flow cell, comprising:
   a substrate having depressions separated by interstitial regions;
   a polymeric hydrogel positioned within each of the depressions; and
   a plurality of transposome complexes immobilized within each of the depressions by a biotin-containing linker, each of the plurality of the transposome complexes being of a single type including a transposon end with a portion of a transferred strand hybridized to a portion of a non-transferred strand, wherein the transferred strand includes a first amplification domain and is free of an index sequence.
2. The flow cell as defined in clause 1, wherein the non-transferred strand consists of the portion hybridized to the portion of the transferred strand.
3. The flow cell as defined in clause 1 or clause 2, further comprising a first primer and a second primer immobilized within each of the depressions by respective second biotin-containing linkers, and wherein:
   the first primer corresponds with the first amplification domain; and
   the second primer has a sequence that is complementary to a second amplification domain of an adapter that is to be hybridized to a portion of the transferred strand.
4. The flow cell as defined in clause 1, wherein:
   the transferred strand and the non-transferred strand form a forked adapter; and
   the non-transferred strand further includes a sequencing primer sequence, an index sequence, and a second amplification domain that is complementary to a primer that is to be immobilized within each of the depressions of the flow cell.
5. The flow cell as defined in clause 1, further comprising a first primer and a second primer immobilized within each of the depressions by respective second biotin-containing linkers, wherein:
   the first primer corresponds with the first amplification domain;
   the transferred strand and the non-transferred strand form a forked adapter; and
   the non-transferred strand further includes a sequencing primer sequence, an index sequence, and a second amplification domain that is complementary to the second primer.
6. A tagmentation kit, comprising:
   a flow cell including:
      a substrate having depressions separated by interstitial regions; and
      a plurality of transposome complexes immobilized within each of the depressions by a biotin-containing linker, each of the plurality of the transposome complexes being of a single type including a transposon end with a portion of a transferred strand hybridized to a non-transferred strand, wherein the transferred strand includes a first amplification domain; and
   an adapter fluid including:
      a carrier fluid; and
      an adapter including:
         a sequence complementary to the portion of the transferred strand of each of the plurality of transposome complexes;
         a sequencing primer sequence;
         an index sequence; and
         a second amplification domain that is complementary to a primer that is to be immobilized within each of the depressions of the flow cell.
7. The tagmentation kit as defined in clause 6, further comprising a grafting fluid including:
   a second carrier fluid;
   a first primer; and
   a second primer that is complementary to the second amplification domain, each of the first and second primers having a second biotin-containing linker at its 5' end.
8. The tagmentation kit as defined in clause 6, wherein the flow cell further includes:
   a first primer; and
   a second primer that is complementary to the second amplification domain, each of the first and second primers immobilized within each of the depressions by a second biotin-containing linker at its 5' end.
9. A tagmentation kit, comprising:
   a flow cell including:
      a substrate having depressions separated by interstitial regions; and
      a plurality of transposome complexes immobilized within each of the depressions by a biotin-containing linker, each of the plurality of the transposome complexes being of a single type including a transposon end with a portion of a transferred strand hybridized to a portion of a non-transferred strand to form a forked adapter, wherein the transferred strand includes a first amplification domain, a sequencing primer sequence, an index sequence, and a second amplification domain that is complementary to a primer that is to be immobilized within each of the depressions of the flow cell; and
   a grafting fluid including:
      a carrier fluid;
      a first primer; and
      a second primer that is complementary to the second amplification domain, each of the first and second primers having a second biotin-containing linker at its 5' end.
10. A method, comprising:
   generating at least partially adapted DNA sample fragments on a flow cell using a plurality of transposome complexes attached to a surface of the flow cell within a flow channel by a biotin-containing linker;
   cleaving the at least partially adapted DNA sample fragments such that the biotin-containing linkers remain attached to the surface; and
   one of:
      using the flow cell for a subsequent cycle of generating at least partially adapted DNA sample fragments; or
      using the flow cell to amplify at least some of the previously cleaved and at least partially adapted DNA sample fragments.
11. The method as defined in clause 10, wherein:
   each of the plurality of the transposome complexes is of a single type including a transposon end with a portion of a transferred strand hybridized to a portion of a non-transferred strand to form a forked adapter, wherein the transferred strand includes a first amplification domain, and wherein the non-transferred strand further includes a sequencing primer sequence, an index sequence, and a second amplification domain that is complementary to a primer that is or is to be immobilized within each of the depressions of the flow cell;
   each of the at least partially adapted DNA sample fragments is a fully adapted DNA sample fragment; and
   generating the fully adapted DNA sample fragments includes:
      introducing a DNA sample and a tagmentation buffer to the flow cell;
      performing tagmentation of the DNA sample using the plurality of transposome complexes;
      removing a transposase enzyme of each of the plurality of transposome complexes; and
      performing gap fill ligation to attach the DNA sample fragments to respective adapters.
12. The method as defined in clause 11, wherein after cleaving the fully adapted DNA sample fragments such that the biotin-containing linker remains attached to the surface and prior to using the flow cell, the method further comprises removing streptavidin from the biotin-containing linker.
13. The method as defined in any one of clause 10 through clause 12, wherein the flow cell is used for the subsequent cycle of generating at least partially adapted DNA sample fragments, and wherein the subsequent cycle of generating at least partially adapted DNA sample fragments involves:
   introducing a new plurality of the transposome complexes into the flow channel, whereby at least some of the new plurality of the transposome complexes attach to at least some of the biotin-containing linkers within the flow channel;
   introducing a new DNA sample and a new tagmentation buffer to the flow channel; and
   repeating the tagmentation, transposase enzyme removal, and gap fill ligation with the new DNA sample.
14. The method as defined in clause 11 or clause 12, wherein the flow cell is used for the amplification of the previously cleaved and fully adapted DNA sample fragments, and wherein the amplification of the previously cleaved and fully adapted DNA sample fragments involves:
   attaching first and second primers to at least some of the biotin-containing linkers within the flow channel; and
   introducing single stranded forms of the previously cleaved and fully adapted DNA sample fragments into the flow cell.
15. The method as defined in clause 10, wherein:
   each of the plurality of the transposome complexes is of a single type including a transposon end with a portion of a transferred strand hybridized to a non-transferred strand;
   the transferred strand includes a first amplification domain;
   each of the at least partially adapted DNA sample fragments is a fully adapted DNA sample fragment; and
   generating the fully adapted DNA sample fragments includes:
      introducing a DNA sample and a tagmentation buffer to the flow channel;
      performing tagmentation of the DNA sample using the plurality of transposome complexes;
      removing a transposase enzyme of each of the plurality of transposome complexes; and
      replacing the non-transferred strand of each of the plurality of transposome complexes with an adapter including:
         a sequence complementary to the portion of the transferred strand;
         a sequencing primer sequence;
         an index sequence; and
         a second amplification domain that is complementary to a primer that is or is to be immobilized within each of the depressions of the flow cell, thereby dehybridizing the non-transferred strand and hybridizing the adapter to the portion of the transferred strand of each of the plurality of transposome complexes; and
      performing gap fill ligation to attach the DNA sample fragments to respective adapters.
16. The method as defined in clause 15, wherein after cleaving the fully adapted DNA sample fragments such that the biotin-containing linker remains attached to the surface and prior to using the flow cell, the method further comprises removing streptavidin from the biotin-containing linker.
17. The method as defined in clause 15 or clause 16, wherein the flow cell is used for the subsequent cycle of generating at least partially adapted DNA sample fragments, and wherein the subsequent cycle of generating fully adapted DNA sample fragments includes:
   introducing a new plurality of the transposome complexes into the flow channel, whereby at least some of the new plurality of the transposome complexes attach to at least some of the biotin-containing linkers within the flow channel;
   introducing a new DNA sample and a new tagmentation buffer to the flow channel; and
   repeating the tagmentation, transposase enzyme removal, replacement of the non-transferred strand with the adapter, and gap fill ligation with the new DNA sample.
18. The method as defined in clause 15 or clause 16, wherein the flow cell is used for the amplification of the previously cleaved and fully adapted DNA sample fragments, and wherein the amplification of the previously cleaved and fully adapted DNA sample fragments involves:
   attaching first and second primers to at least some of the biotin-containing linkers within the flow channel; and
   introducing single stranded forms of the previously cleaved and fully adapted DNA sample fragments into the flow cell.
19. The method as defined in clause 10, wherein:
   the plurality of the transposome complexes includes first transposome complexes including a first amplification domain and second transposome complexes including a second amplification domain;
   each of the at least partially adapted DNA sample fragments is a fully adapted DNA sample fragment; and
   generating the fully adapted DNA sample fragments includes:
      introducing a DNA sample and a tagmentation buffer to the flow cell;
      performing tagmentation of the DNA sample using the plurality of transposome complexes;
      removing a transposase enzyme of each of the plurality of transposome complexes; and
      performing an extension reaction.
20. The method as defined in clause 19, wherein some of the fully adapted DNA sample fragments include a targeted region, and wherein after cleaving the fully adapted DNA sample fragments, the method further comprises generating an enriched sample by:
   denaturing the fully adapted DNA sample fragments to generate single stranded fully adapted DNA sample fragments, wherein some of the single stranded fully adapted DNA sample fragments include the targeted region;
   transporting the single stranded fully adapted DNA sample fragments to an enrichment receptacle on the flow cell; and
   introducing biotinylated capture probes to the enrichment receptacle, whereby the some of the single stranded fully adapted DNA sample fragments with the targeted region respectively hybridize to the biotinylated capture probes to form enriched complexes, and some other of the single stranded fully adapted DNA sample fragments without the targeted regions remain unattached.
21. The method as defined in clause 20, further comprising:
   replenishing streptavidin in the flow channel;
   transporting the enriched sample to the flow channel including the replenished streptavidin, whereby the enriched complexes attach to the biotin-containing linkers and the single stranded fully adapted DNA sample fragments without the targeted regions remain unattached;
   introducing a wash solution to the flow channel to remove the single stranded fully adapted DNA sample fragments without the targeted regions;
   releasing the some of the single stranded fully adapted DNA sample fragments with the targeted region from the biotinylated capture probes; and
   transporting the some of the single stranded fully adapted DNA sample fragments with the targeted region to a holding receptacle.
22. The method defined in clause 21, wherein while the some of the single stranded fully adapted DNA sample fragments with the targeted region are in the holding receptacle, the method further comprises removing streptavidin from the biotin-containing linker in the flow channel; and
   attaching first and second primers to at least some of the biotin-containing linkers within the flow channel.
23. The method defined in clause 21 or clause 22, wherein the flow cell is used for the amplification of the previously cleaved and fully adapted DNA sample fragments, and wherein the amplification of the previously cleaved and fully adapted DNA sample fragments is initiated by transporting the some of the single stranded fully adapted DNA sample fragments with the targeted region from the holding receptacle to the flow channel after the first and second primers have been attached.
24. The method as defined in clause 10, wherein:
   each of the plurality of the transposome complexes is of a single type including a transposon end with a portion of a transferred strand hybridized to a non-transferred strand;
   the transferred strand includes a first amplification domain;
   each of the at least partially adapted DNA sample fragments is a partially adapted DNA sample fragment;
   generating the partially adapted DNA sample fragments includes:
      introducing a DNA sample and a tagmentation buffer to the flow channel;
      performing tagmentation of the DNA sample using the plurality of transposome complexes; and
      removing a transposase enzyme of each of the plurality of transposome complexes.
25. The method as defined in clause 24, wherein after the partially adapted DNA sample fragments are cleaved, the method further comprises:
   denaturing the partially adapted DNA sample fragments to generate single stranded partially adapted DNA sample fragments, wherein some of the single stranded partially adapted DNA sample fragments include a targeted region;
   transporting the single stranded partially adapted DNA sample fragments to an enrichment receptacle on the flow cell, the enrichment receptacle including surface bound adapters having a second amplification domain and a region complementary to the targeted region, whereby the some of the single stranded partially adapted DNA sample fragments with the targeted region respectively hybridize to the surface bound adapters, and some other of the single stranded partially adapted DNA sample fragments without the targeted regions remain unattached;
   introducing a wash solution to the enrichment receptacle to remove the single stranded partially adapted DNA sample fragments without the targeted regions; and
   performing an extension reaction along the surface bound adapters, thereby generating fully adapted DNA sample fragments.
26. A method, comprising:
   capturing a plurality of biotinylated dDpn1 at a plurality of biotin-containing linkers attached to a surface within a flow channel of a flow cell;
   introducing a DNA sample to the flow cell, whereby methylated bacterial DNA within the DNA sample is captured by the biotinylated dDpn1;
   introducing a wash solution to the flow channel to remove uncaptured DNA sample therefrom;
   releasing the methylated bacterial DNA from the biotinylated dDpn1 such that the biotin-containing linker remains attached to the surface;
   transporting the cleaved methylated bacterial DNA to a holding receptacle;
   while the cleaved methylated bacterial DNA is in the holding receptacle:
      replenishing streptavidin from the biotin-containing linker;
      introducing a plurality of biotinylated transposome complexes to the flow channel, whereby the plurality of biotinylated transposome complexes attach to some of the biotin-containing linkers; and
      introducing a plurality of biotinylated primers to the flow channel, whereby the plurality of biotinylated primers attach to some other of the biotin-containing linkers; and
   transporting the cleaved methylated bacterial DNA back to the flow channel containing the biotinylated transposome complexes and the biotinylated primers.
27. A method, comprising:
   introducing a tagmentation entity having a first visible light responsive member attached thereto to a flow cell having a second visible light responsive member attached thereto; and
   exposing a predetermined area of the flow cell to visible light while the tagmentation entity is present in the flow cell, thereby coupling the first and second visible light responsive members and attaching the tagmentation entity to the flow cell at the predetermined area.
28. The method as defined in clause 27, further comprising:
   introducing primers having the first visible light responsive member attached thereto to the flow cell; and
   exposing a second predetermined area of the flow cell to visible light while the primers are present in the flow cell, thereby coupling the first and second visible light responsive members and attaching the primers to the flow cell at the second predetermined area.
29. The method as defined in clause 27 or clause 28, wherein after the tagmentation entity is coupled, the method further comprises:
   introducing a DNA sample to the flow cell;
   initiating tagmentation of the DNA sample using the coupled tagmentation entity;
   removing a transposase enzyme of the tagmentation entity; and
   coupling a second tagmentation entity, having the first visible light responsive member attached thereto, at a second predetermined area of the flow cell by:
      introducing the second tagmentation entity into the flow cell; and
      exposing the second predetermined area of the flow cell to visible light while the second tagmentation entity is present in the flow cell, thereby coupling the first and second visible light responsive members and attaching the second tagmentation entity to the flow cell at the second predetermined area.
30. The method as defined in clause 29, wherein the first and second tagmentation entities have different index sequences.
31. A kit, comprising:
   a flow cell including:
      a substrate having depressions separated by interstitial regions; and
      a biotinylated polymeric hydrogel positioned within each of the depressions;
   a transposome fluid including:
      a first carrier fluid; and
      a plurality of biotinylated transposome complexes;
   a grafting fluid including:
      a second carrier fluid; and
      a plurality of biotinylated amplification primers; and
   streptavidin.
32. The kit as defined in clause 31, further comprising biotinylated enrichment probes.
33. The kit as defined in clause 31 or clause 32, further comprising a biotin streptavidin cleavage composition.
34. The kit as defined in any one of clause 31 through clause 33, further comprising a cleaving agent that is to cleave a cleavage site of each biotinylated transposome complex.
35. The kit as defined in any one of clause 31 through clause 34, wherein:
   each biotinylated transposome complex is of a single type including a transposon end with a portion of a transferred strand hybridized to a non-transferred strand, wherein the transferred strand includes a first amplification domain; and
   the kit further comprises an adapter fluid including:
      a third carrier fluid; and
      an adapter including:
         a sequence complementary to the portion of the transferred strand of each of the plurality of transposome complexes;
         a sequencing primer sequence;
         an index sequence; and
         a second amplification domain that is complementary to one type of the plurality of biotinylated amplification primers.
36. The kit as defined in any one of clause 31 through clause 34, wherein the plurality of biotinylated transposome complexes includes:
   first transposome complexes including a first amplification domain that has a same sequence as one type of the plurality of biotinylated amplification primers; and
   second transposome complexes including a second amplification domain that has a same sequence as a second type of the plurality of biotinylated amplification primers.
37. The kit as defined in any one of clause 31 through clause 34, wherein:
   the plurality of biotinylated transposome complexes includes first transposome complexes including a first amplification domain that has a same sequence as one type of the plurality of biotinylated amplification primers; and
   the flow cell further comprises an enrichment chamber including surface bound adapters having a second amplification domain that has a same sequence as a second type of the plurality of biotinylated amplification primers and a region complementary to a target sample region.
38. A method, comprising:
   selectively activating a sub-set of transposome complexes in a predetermined area of a flow cell by heating the predetermined area to an activation temperature and cooling an other area of the flow cell below the activation temperature, wherein the flow cell includes:
      a substrate having depressions separated by interstitial regions;
      a polymeric hydrogel positioned within each of the depressions; and
      the transposome complexes respectively immobilized within each of the depressions; and
   introducing a DNA sample to the flow cell, whereby a portion of the DNA sample binds to and is tagmented by the sub-set of transposome complexes in the predetermined area and an other portion of the DNA sample binds to, but remains untagmented by, the transposome complexes in the other area.
39. The method as defined in clause 38, wherein the activation temperature ranges from about 20°C to about 55°C.
40. The method as defined in clause 38 or clause 39, further comprising:
   removing a transposase enzyme of each of the transposome complexes, thereby releasing the other portion of the DNA sample and whereby the portion of the DNA sample remains attached in the predetermined area;
   washing the removed transposase enzymes and the released DNA sample from the flow cell; and
   introducing fresh transposase enzymes to the flow cell, thereby reforming transposome complexes in the other area.
41. The method as defined in any one of clause 38 through clause 40, wherein:
   a second predetermined area of the flow cell corresponds with at least a portion of the other area; and
   the method further comprises:
      selectively activating a second sub-set of transposome complexes in the second predetermined area by heating the second predetermined area to the activation temperature and cooling at least the predetermined area of the flow cell below the activation temperature; and
      introducing a second DNA sample to the flow cell, whereby a portion of the second DNA sample binds to and is tagmented by the second sub-set of transposome complexes in the second predetermined area.
42. A method, comprising:
   introducing a DNA sample to a flow cell including:
      a substrate having depressions separated by interstitial regions;
      a polymeric hydrogel positioned within each of the depressions; and
      transposome complexes immobilized within each of the depressions;
      whereby the DNA sample binds to the transposome complexes across the flow cell;
   introducing an encapsulated complex into the flow cell, the encapsulated complex including a catalytic metal surrounded by a coating material; and
   selectively releasing the catalytic metal from the encapsulated complex at a predetermined area of the flow cell by exposing the encapsulated complex to a mechanism that initiates removal of the coating material, thereby initiating tagmentation of a portion of the DNA sample in the predetermined area while leaving an other portion of the DNA sample untagmented in an other area.
43. The method as defined in clause 42, wherein the mechanism is heat and the method further comprises cooling the other area of the flow cell to prevent catalytic metal release in the other area.
44. The method as defined in clause 42, wherein the mechanism is light.
45. The method as defined in clause 42, wherein the mechanism is a change in pH.
46. The method as defined in any of clause 42 through clause 45, further comprising:
   removing a transposase enzyme of each of the transposome complexes, thereby releasing the other portion of the DNA sample from the other area and whereby the portion of the DNA sample remains attached in the predetermined area;
   washing the removed transposase enzymes and the released other portion of the DNA sample from the flow cell; and
   introducing fresh transposase enzymes to the flow cell, thereby reforming transposome complexes in the other area.
47. The method as defined in clause 46, wherein:
   a second predetermined area of the flow cell corresponds with at least a portion of the other area; and
   the method further comprises:
      introducing a second DNA sample to the flow cell, whereby the second DNA sample binds to the transposome complexes across the flow cell;
      introducing a second encapsulated complex into the flow cell, the second encapsulated complex including a second catalytic metal surrounded by a second coating material; and
      selectively releasing the second catalytic metal from the second encapsulated complex at the second predetermined area by exposing the second encapsulated complex to a second mechanism that initiates removal of the second coating material, thereby initiating tagmentation of a portion of the second DNA sample in the second predetermined area.
48. A method, comprising:
   introducing a DNA sample to a flow cell including:
      a substrate having depressions separated by interstitial regions;
      a polymeric hydrogel positioned within each of the depressions;
      transposome complexes immobilized within each of the depressions; and
      a removable coating overlying the transposome complexes;
   exposing a sub-set of the transposome complexes at a predetermined area of the flow cell by removing a portion of the removable coating from the predetermined area; and
   initiating tagmentation of at least a portion of the DNA sample at the predetermined area.
49. The method as defined in clause 48, wherein exposing the sub-set of the transposome complexes involves heating the predetermined area, and the method further comprises cooling an other area of the flow cell to prevent removal of an other portion of the removable coating from the other area.
50. The method as defined in clause 48, wherein exposing the sub-set of the transposome complexes involves exposing the predetermined area to visible light.
51. The method as defined in clause 48, wherein exposing the sub-set of the transposome complexes involves changing a pH at the predetermined area.
52. The method as defined in any one of clause 48 through clause 51, further comprising:
   removing a transposase enzyme from the transposome complexes in the sub-set; and
   washing the removed transposase enzymes from the flow cell.
53. The method as defined in clause 52, wherein:
   a second predetermined area of the flow cell corresponds with at least a portion of the other area; and
   the method further comprises:
      introducing a second DNA sample to the flow cell;
      exposing a second sub-set of the transposome complexes at the second predetermined area of the flow cell by removing a second portion of the removable coating from the second predetermined area; and
      initiating tagmentation of at least a portion of the second DNA sample at the second predetermined area.
54. A kit, comprising:
   a flow cell including:
      a substrate having depressions separated by interstitial regions;
      a polymeric hydrogel positioned within each of the depressions;
      an amplification primer set attached to the polymeric hydrogel within each of the depressions; and
      a target primer attached to the polymeric hydrogel within each of the depressions, wherein the target primer attached within the depressions located at a first region of the flow cell is orthogonal to the target primer attached within the depressions located at a second region of the flow cell;
   a first fluid including:
      a liquid carrier; and
      a first plurality of transposome complexes dispersed in the liquid carrier, each of the first plurality of transposome complexes including a first spatial tag that is complementary to the target primer attached within the depressions located at the first region of the flow cell; and
   a second fluid including:
      a liquid carrier; and
      a second plurality of transposome complexes dispersed in the liquid carrier, each of the second plurality of transposome complexes including a second spatial tag that is complementary to the target primer attached within the depressions located at the second region of the flow cell.
55. The kit as defined in clause 54, wherein:
   each first spatial tag is attached at a 5' end of a transferred strand of each of the first plurality of transposome complexes; and
   each second spatial tag is attached at a 5' end of a transferred strand of each of the second plurality of transposome complexes.
56. The kit as defined in clause 54, wherein:
   some of the first spatial tags are respectively attached at a 5' end of a transferred strand of some of the first plurality of transposome complexes;
   some other of the first spatial tags are respectively attached at a 3' end of a non-transferred strand of some other of the first plurality of transposome complexes;
   some of the second spatial tags are respectively attached at a 5' end of a transferred strand of some of the second plurality of transposome complexes; and
   some other of the second spatial tags are respectively attached at a 3' end of a non-transferred strand of some other of the second plurality of transposome complexes.
57. A method for using the kit of clause 54, the method comprising:
   operatively positioning the flow cell in a sequencing instrument;
   generating fully adapted first DNA sample fragments in the first region of the flow cell by:
      introducing the first fluid into the flow cell at a hybridization temperature, whereby the first spatial tags respectively hybridize to the target primers attached within the depressions located at the first region of the flow cell;
      introducing a first DNA sample into the flow cell, whereby the first DNA sample is tagmented by the first plurality of transposome complexes;
      removing a transposase enzyme of each of the first plurality of transposome complexes; and
      performing gap fill ligation or an extension reaction; and
   generating fully adapted second DNA sample fragments in the second region of the flow cell by:
      introducing the second fluid into the flow cell at a hybridization temperature, whereby the second spatial tags respectively hybridize to the target primers attached within the depressions located at the second region of the flow cell;
      introducing a second DNA sample into the flow cell, whereby the second DNA sample is tagmented by the second plurality of transposome complexes;
      removing a transposase enzyme of each of the second plurality of transposome complexes; and
      performing gap fill ligation or an extension reaction.
58. A method for using the kit of clause 54, the method comprising:
   operatively positioning the flow cell in a sequencing instrument;
   exposing the first plurality of transposome complexes to a first DNA sample, whereby the first DNA sample binds to at least some of the first plurality of transposome complexes to form a first bound complex;
   introducing the first bound complex to the flow cell, whereby the first spatial tags respectively hybridize to the target primers attached within the depressions located at the first region of the flow cell;
   passivating the first bound complex at the first region of the flow cell;
   exposing the second plurality of transposome complexes to a second DNA sample, whereby the second DNA sample binds to at least some of the second plurality of transposome complexes to form a second bound complex; and
   introducing the second bound complex to the flow cell, whereby the second spatial tags respectively hybridize to the target primers attached within the depressions located at the second region of the flow cell.
59. The method as defined in clause 58, further comprising passivating the second bound complex at the second region of the flow cell.
60. The method as defined in clause 58 or clause 59, wherein passivating the first bound complex involves attaching an anti-fouling agent to the first bound complex.
61. The method as defined in clause 58 or clause 59, wherein passivating the first bound complex involves forming a hydrophobic polymer shell over the first bound complex.
62. The method as defined in any one of clause 58 through clause 61, further comprising:
   de-passivating the first bound complex at the first region of the flow cell; and
   simultaneously initiating the formation of respective sets of fully adapted DNA fragments at each of the first and second regions of the flow cell.
63. A method for using the kit of clause 54, the method comprising:
   operatively positioning the flow cell in a sequencing instrument;
   generating partially adapted first DNA sample fragments in the first region of the flow cell by:
      introducing the first fluid into the flow cell at a hybridization temperature, whereby the first spatial tags respectively hybridize to the target primers attached within the depressions located at the first region of the flow cell;
      introducing a first DNA sample into the flow cell, whereby the first DNA sample is tagmented by the first plurality of transposome complexes; and
      removing a transposase enzyme of each of the first plurality of transposome complexes;
   generating partially adapted second DNA sample fragments in the second region of the flow cell by:
      introducing the second fluid into the flow cell at a hybridization temperature, whereby the second spatial tags respectively hybridize to the target primers attached within the depressions located at the second region of the flow cell;
      introducing a second DNA sample into the flow cell, whereby the second DNA sample is tagmented by the second plurality of transposome complexes; and
      removing a transposase enzyme of each of the second plurality of transposome complexes; and
   performing gap fill ligation or an extension reaction, thereby generating fully adapted first and second DNA sample fragments.
64. A method, comprising:
   attaching an amplification primer set along a flow cell lane; and
   sequentially attaching orthogonal target primers in at least two different regions of the flow cell lane.

## Claims

1. A tagmentation kit, comprising:
a flow cell including:
a substrate having depressions separated by interstitial regions; and
a plurality of transposome complexes immobilized within each of the depressions by a biotin-containing linker, each of the plurality of the transposome complexes being of a single type including a transposon end with a portion of a transferred strand hybridized to a non-transferred strand, wherein the transferred strand includes a first amplification domain and a first index sequence; and
an adapter fluid including:
a carrier fluid; and
an adapter including:
a sequence complementary to the portion of the transferred strand of each of the plurality of transposome complexes;
a sequencing primer sequence;
a second index sequence; and
a second amplification domain that is complementary to a primer that is to be immobilized within each of the depressions of the flow cell.

2. The tagmentation kit as defined in claim 1, further comprising a grafting fluid including:
a second carrier fluid;
a first primer; and
a second primer that is complementary to the second amplification domain, each of the first and second primers having a second biotin-containing linker at its 5' end.

3. The tagmentation kit as defined in claim 1, wherein the flow cell further includes:
a first primer; and
a second primer that is complementary to the second amplification domain, each of the first and second primers immobilized within each of the depressions by a second biotin-containing linker at its 5' end.

4. The tagmentation kit as defined in claim 1, wherein the flow cell further includes a biotinylated polymeric hydrogel positioned within each of the depressions.

5. The tagmentation kit as defined in claim 1, wherein the transferred strand further includes a sequencing primer sequence attached to the portion of a transferred strand in the transposon end, and wherein the index sequence is between the sequencing primer sequence and the first amplification domain.

6. The tagmentation kit as defined in claim 1, wherein:
each of the plurality of transposome complexes has a 5' end functional group;
the 5' end functional group is biotin; and
the biotin-containing linker includes a graftable group and biotin or a graftable group and biotin and streptavidin.

7. A tagmentation kit, comprising:
a flow cell including:
a substrate having depressions separated by interstitial regions; and
a plurality of transposome complexes immobilized within each of the depressions by a biotin-containing linker, each of the plurality of the transposome complexes being of a single type including a transposon end with a portion of a transferred strand hybridized to a portion of a non-transferred strand to form a forked adapter, wherein the transferred strand includes a first amplification domain, a sequencing primer sequence, and an index sequence, and wherein the non-transferred strand includes a second amplification domain that is complementary to a second primer that is to be immobilized within each of the depressions of the flow cell; and
a grafting fluid including:
a carrier fluid;
a first primer; and
the second primer that is complementary to the second amplification domain, each of the first and second primers having a second biotin-containing linker at its 5' end.

8. The tagmentation kit as defined in claim 7, wherein the non-transferred strand is an adapter that further includes a sequencing primer sequence attached to the portion of the non-transferred strand, a second index sequence, and the second amplification domain.

9. The tagmentation kit as defined in claim 7, wherein the flow cell further includes a biotinylated polymeric hydrogel positioned within each of the depressions.

10. The tagmentation kit as defined in claim 7, wherein:
each of the plurality of transposome complexes has a 5' end functional group;
the 5' end functional group is biotin; and
the biotin-containing linker includes a graftable group and biotin or a graftable group and biotin and streptavidin.
